# EUROPEAN PATENT APPLICATION

(11) **EP 4 719 021 A1**
(43) Date of publication of application: **01.04.2026**
(21) Application number: 25203707.2
(22) Date of filing: 22.09.2025
(51) Int. Cl.: H10K 85/60, H10K 50/11

(54) **LIGHT-EMITTING DEVICE INCLUDING HETEROCYCLIC COMPOUND, ELECTRONIC APPARATUS INCLUDING THE LIGHT-EMITTING DEVICE, AND THE HETEROCYCLIC COMPOUND**

(30) Priority: 25.09.2024 KR 20240130209
(71) Applicant: Samsung Display Co., Ltd., Yongin-si, Gyeonggi-do 17113 (KR)
(72) Inventor: HAN, Junghoon, 17113 Yongin-si (KR); KIM, Dojin, 17113 Yongin-si (KR); AHN, Heechoon, 17113 Yongin-si (KR); UM, Hyunah, 17113 Yongin-si (KR); LEE, Yeseul, 17113 Yongin-si (KR); LEE, Hyunwoo, 17113 Yongin-si (KR)
(74) Representative: Marks & Clerk LLP

(57) **Abstract**

Embodiments provide a heterocyclic compound, a light-emitting device including the heterocyclic compound, an electronic apparatus including the light-emitting device, and an electronic equipment including the light-emitting device. The light-emitting device includes a first electrode (110), a second electrode (150) facing the first electrode, an interlayer (130) between the first electrode and the second electrode and including an interlayer, and the heterocyclic compound. The heterocyclic compound is represented by Formula 1, which is explained in the specification.

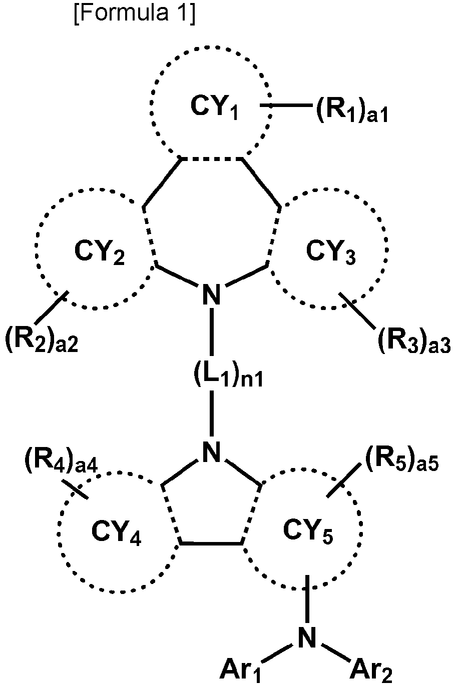

## Description

### BACKGROUND

### 1. Technical Field

Embodiments relate to a light-emitting device including a heterocyclic compound, an electronic apparatus including the light-emitting device, and the heterocyclic compound.

### 2. Description of the Related Art

Light-emitting devices are self-emissive devices that have wide viewing angles, high contrast ratios, short response times, and excellent characteristics in terms of luminance, driving voltage, and response speed.

As an example, a light-emitting device may have a structure in which a first electrode is arranged on a substrate, and a hole transport region, an emission layer, an electron transport region, and a second electrode are sequentially arranged on the first electrode. Holes provided from the first electrode move toward the emission layer through the hole transport region, and electrons provided from the second electrode move toward the emission layer through the electron transport region. Carriers, such as holes and electrons, recombine in the emission layer to produce excitons. The excitons transition from an excited state to a ground state, thereby generating light.

It is to be understood that this background of the technology section is, in part, intended to provide useful background for understanding the technology. However, this background of the technology section may also include ideas, concepts, or recognitions that were not part of what was known or appreciated by those skilled in the pertinent art prior to a corresponding effective filing date of the subject matter disclosed herein.

### SUMMARY

Embodiments include a light-emitting device including a heterocyclic compound, an electronic apparatus including the light-emitting device, and the heterocyclic compound.

Additional aspects will be set forth in part in the description which follows and, in part, will be apparent from the description, or may be learned by practice of the embodiments of the disclosure.

According to embodiments,
a light-emitting device includes
a first electrode,
a second electrode facing the first electrode,
an interlayer between the first electrode and the second electrode and including an emission layer, and
a heterocyclic compound represented by Formula 1:

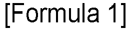

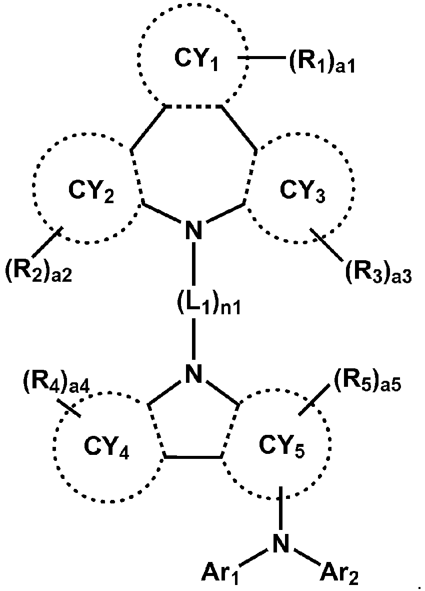

In Formula 1,
rings CY₁ to CY₅ are each independently a C₅-C₆₀ carbocyclic group or a C₂-C₆₀ heterocyclic group,
n1 is an integer from 1 to 5,
L₁ is a C₃-C₆₀ carbocyclic group that is unsubstituted or substituted with at least one R₁₀ₐ or a C₁-C₆₀ heterocyclic group that is unsubstituted or substituted with at least one R₁₀ₐ,
a1 to a5 are each independently an integer from 0 to 10,
Ar₁, Ar₂, and R₁ to R₅ are each independently hydrogen, deuterium, -F, -Cl, - Br, -I, a hydroxyl group, a cyano group, a nitro group, an amidino group, a hydrazino group, a hydrazono group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₁₀ cycloalkyl group that is unsubstituted or substituted with at least one R₁₀ₐ, a C₁-C₁₀ heterocycloalkyl group that is unsubstituted or substituted with at least one R₁₀ₐ, a C₃-C₁₀ cycloalkenyl group that is unsubstituted or substituted with at least one R₁₀ₐ, a C₁-C₁₀ heterocycloalkenyl group that is unsubstituted or substituted with at least one R₁₀ₐ, a C₆-C₆₀ aryl group that is unsubstituted or substituted with at least one R₁₀ₐ, a C₆-C₆₀ aryloxy group that is unsubstituted or substituted with at least one R₁₀ₐ, a C₆-C₆₀ arylthio group that is unsubstituted or substituted with at least one R₁₀ₐ, a C₁-C₆₀ heteroaryl group that is unsubstituted or substituted with at least one R₁₀ₐ, a C₁-C₆₀ heteroaryloxy group that is unsubstituted or substituted with at least one R₁₀ₐ, a C₁-C₆₀ heteroarylthio group that is unsubstituted or substituted with at least one R₁₀ₐ, a monovalent non-aromatic condensed polycyclic group that is unsubstituted or substituted with at least one R₁₀ₐ, a monovalent non-aromatic condensed heteropolycyclic group that is unsubstituted or substituted with at least one R₁₀ₐ, - Si(Q₁)(Q₂)(Q₃), -B(Q₁)(Q₂), -N(Q₁)(Q₂), -P(Q₁)(Q₂), -C(=O)(Q₁), -S(=O)(Q₁), - S(=O)₂(Q₁), -P(=O)(Q₁)(Q₂), or -P(=S)(Q₁)(Q₂),
two or more neighboring groups among Ar₁, Ar₂, and R₁ to R₅ are optionally bonded to each other to form a C₅-C₆₀ carbocyclic group that is unsubstituted or substituted with at least one R₁₀ₐ or a C₁-C₆₀ heterocyclic group that is unsubstituted or substituted with at least one R₁₀ₐ,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, or a C₁-C₆₀ alkoxy group; or
a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, the interlayer may further include a hole transport region between the first electrode and the emission layer, and an electron transport region between the emission layer and the second electrode; the hole transport region may include a hole injection layer, a hole transport layer, an emission auxiliary layer, an electron-blocking layer, or any combination thereof; and the electron transport region may include a buffer layer, a hole-blocking layer, an electron transport layer, an electron injection layer, or any combination thereof.

In an embodiment, the emission layer may include a host, and a dopant; and the host may include the heterocyclic compound.

In an embodiment, the light-emitting device may further include at least one of a first capping layer outside the first electrode, and a second capping layer outside the second electrode, wherein the at least one of the first capping layer and the second capping layer may include the heterocyclic compound.

In an embodiment, the emission layer may emit blue light.

According to embodiments, an electronic apparatus includes the light-emitting device.

In an embodiment, the electronic apparatus may further include: a thin-film transistor electrically connected to the light-emitting device; and a color filter, a color conversion layer, a touch screen layer, a polarizing layer, or any combination thereof. In an embodiment, the color conversion layer may include quantum dots.

According to embodiments, an electronic equipment includes the light-emitting device.

In an embodiment, the electronic equipment may be a flat panel display, a curved display, a computer monitor, a medical monitor, a television, an advertisement board, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a microdisplay, a three-dimensional (3D) display, a virtual reality display, an augmented reality display, a vehicle, a video wall with multiple displays tiled together, a theater screen, a stadium screen, a phototherapy device, or a signboard.

According to embodiments, a heterocyclic compound is represented by Formula 1, which is explained herein.

In an embodiment, ring CY₁ to ring CY₅ may each independently be a benzene group, a naphthalene group, an anthracene group, a phenanthrene group, a triphenylene group, a pyrene group, a chrysene group, an acenaphthylene group, a perylene group, a benzopyrene group, a benzochrysene group, a benzotriphenylene group, a fluoranthene group, a coronene group, a cyclopentadiene group, a 1,2,3,4-tetrahydronaphthalene group, an acridine group, a thiophene group, a furan group, an indole group, a benzoborole group, a benzophosphole group, an indene group, a benzosilole group, a benzogermole group, a benzothiophene group, a benzoselenophene group, a benzofuran group, a benzotellurophene group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzotellurophene group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, ring CY₁ to ring CY₅ may each independently be a benzene group, a naphthalene group, or a pyridine group.

In an embodiment, L₁ may be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ; and
R₁₀ₐ may be the same as defined in Formula 1.

In an embodiment, L₁ may be a group represented by one of Formulae 3-1 to 3-30, which are explained below.

In an embodiment, in Formula 1, a moiety represented by 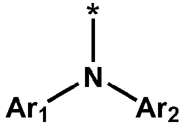 may be a moiety represented by Formula 6, which is explained below.

In an embodiment, in Formula 1, a moiety represented by 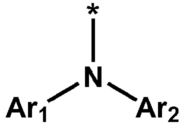 may be a moiety represented by one of Formulae 6-1 to 6-3, which are explained below.

In an embodiment, R₁ to R₅ may each independently be:
hydrogen, deuterium, -F, -Cl, -Br, -I, a hydroxyl group, a cyano group, a nitro group, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -CI, -Br, -I, -CD₃, -CD₂H, -CDH₂, -CF₃, -CF₂H, -CFH₂, a hydroxyl group, a cyano group, a nitro group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzothiazolyl group, a benzoisoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzosilolyl group, a dibenzocarbazolyl group, an imidazopyridinyl group, an imidazopyrimidinyl group, -O(Q₃₁), -S(Q₃₁), -Si(Q₃₁)(Q₃₂)(Q₃₃), -N(Q₃₁)(Q₃₂), - B(Q₃₁)(Q₃₂), -P(Q₃₁)(Q₃₂), -C(=O)(Q₃₁), -S(=O)₂(Q₃₁), -P(=O)(Q₃₁)(Q₃₂), or any combination thereof; or
-C(Q₁)(Q₂)(Q₃), -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
Q₁ to Q₃ and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, the heterocyclic compound may be represented by one of Formulae 1-1 to 1-4, which are explained below.

In an embodiment, the heterocyclic compound may be one of Compounds 1 to 100, which are explained below.

At least some of the above and other features of the invention are set out in the claims.

It is to be understood that the embodiments above are described in a generic and explanatory sense only and not for the purpose of limitation, and the disclosure is not limited to the embodiments described above.

### BRIEF DESCRIPTION OF THE DRAWINGS

The accompanying drawings are included to provide a further understanding of the embodiments, and are incorporated in and constitute a part of this specification. The drawings illustrate embodiments of the disclosure and principles thereof. The above and other aspects and features of the disclosure will become more apparent by describing in detail embodiments thereof with reference to the accompanying drawings, in which:
FIG. 1 is a schematic cross-sectional view of a light-emitting device according to an embodiment;
FIG. 2 is a schematic cross-sectional view of an electronic apparatus according to an embodiment;
FIG. 3 is a schematic cross-sectional view of an electronic apparatus according to another embodiment;
FIG. 4 is a schematic perspective view of an electronic equipment according to an embodiment;
FIG. 5 is a schematic perspective view of an exterior of a vehicle as an electronic equipment according to an embodiment; and
FIGS. 6A to 6C are each a schematic diagram of an interior of a vehicle according to embodiments.

### DETAILED DESCRIPTION OF THE EMBODIMENTS

The disclosure will now be described more fully hereinafter with reference to the accompanying drawings, in which embodiments are shown. This disclosure may, however, be embodied in different forms and should not be construed as limited to the embodiments set forth herein. Rather, these embodiments are provided so that this disclosure will be thorough and complete, and will fully convey the scope of the disclosure to those skilled in the art.

In the drawings, the sizes, thicknesses, ratios, and dimensions of the elements may be exaggerated for ease of description and for clarity. Like reference numbers and/or like reference characters refer to like elements throughout.

In the description, it will be understood that when an element (or region, layer, part, etc.) is referred to as being "on", "connected to", or "coupled to" another element, it can be directly on, connected to, or coupled to the other element, or one or more intervening elements may be present therebetween. In a similar sense, when an element (or region, layer, part, etc.) is described as "covering" another element, it can directly cover the other element, or one or more intervening elements may be present therebetween.

In the description, when an element is "directly on," "directly connected to," or "directly coupled to" another element, there are no intervening elements present. For example, "directly on" may mean that two layers or two elements are disposed without an additional element such as an adhesion element therebetween.

As used herein, the expressions used in the singular such as "a," "an," and "the," are intended to include the plural forms as well, unless the context clearly indicates otherwise.

As used herein, the term "and/or" includes any and all combinations of one or more of the associated listed items. For example, "A and/or B" may be understood to mean "A, B, or A and B." The terms "and" and "or" may be used in the conjunctive or disjunctive sense and may be understood to be equivalent to "and/or".

In the specification and the claims, the term "at least one of" is intended to include the meaning of "at least one selected from the group consisting of" for the purpose of its meaning and interpretation. For example, "at least one of A, B, and C" may be understood to mean A only, B only, C only, or any combination of two or more of A, B, and C, such as ABC, ACC, BC, or CC. When preceding a list of elements, the term, "at least one of," modifies the entire list of elements and does not modify the individual elements of the list.

It will be understood that, although the terms first, second, etc. may be used herein to describe various elements, these elements should not be limited by these terms. These terms are only used to distinguish one element from another element. Thus, a first element could be termed a second element without departing from the teachings of the disclosure. Similarly, a second element could be termed a first element, without departing from the scope of the disclosure.

The spatially relative terms "below", "beneath", "lower", "above", "upper", or the like, may be used herein for ease of description to describe the relations between one element or component and another element or component as illustrated in the drawings. It will be understood that the spatially relative terms are intended to encompass different orientations of the device in use or operation, in addition to the orientation depicted in the drawings. For example, in the case where a device illustrated in the drawing is turned over, the device positioned "below" or "beneath" another device may be placed "above" another device. Accordingly, the illustrative term "below" may include both the lower and upper positions. The device may also be oriented in other directions and thus the spatially relative terms may be interpreted differently depending on the orientations.

The terms "about" or "approximately" as used herein is inclusive of the stated value and means within an acceptable range of deviation for the recited value as determined by one of ordinary skill in the art, considering the measurement in question and the error associated with measurement of the recited quantity (for example, the limitations of the measurement system). For example, "about" may mean within one or more standard deviations, or within ±20%, ±10%, or ±5% of the stated value.

It should be understood that the terms "comprises," "comprising," "includes," "including," "have," "having," "contains," "containing," and the like are intended to specify the presence of stated features, integers, steps, operations, elements, components, or combinations thereof in the disclosure, but do not preclude the presence or addition of one or more other features, integers, steps, operations, elements, components, or combinations thereof.

Unless otherwise defined or implied herein, all terms (including technical and scientific terms) used have the same meaning as commonly understood by those skilled in the art to which this disclosure pertains. It will be further understood that terms, such as those defined in commonly used dictionaries, should be interpreted as having a meaning that is consistent with their meaning in the context of the relevant art and should not be interpreted in an ideal or excessively formal sense unless clearly defined in the specification.

According to an embodiment, a light-emitting device (for example, an organic light-emitting device) includes: a first electrode; a second electrode facing the first electrode; an interlayer between the first electrode and the second electrode and including an emission layer; and a heterocyclic compound represented by Formula 1.

Hereinafter, the heterocyclic compound represented by Formula 1 is described.

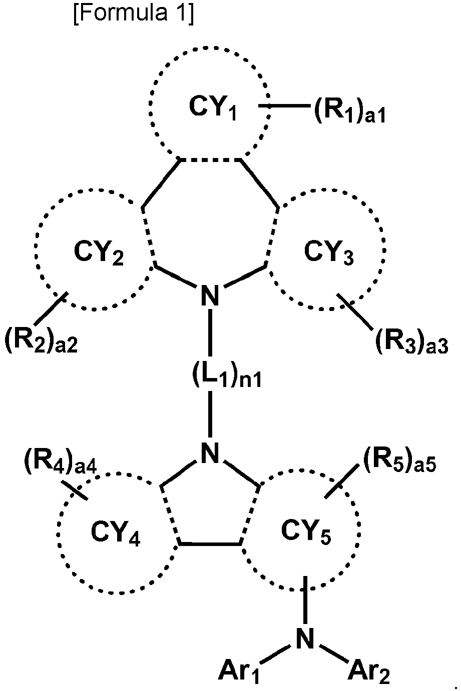

In Formula 1,
ring CY₁ to ring CY₅ are each independently a C₅-C₆₀ (e.g. C₅-C₃₀) carbocyclic group or a C₂-C₆₀ (e.g. C₂-C₂₀) heterocyclic group,
n1 is an integer from 1 to 5,
L₁ is a C₃-C₆₀ (e.g. C₃-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ,
a1 to a5 are each independently an integer from 0 to 10,
Ar₁, Ar₂, and R₁ to R₅ are each independently hydrogen, deuterium, -F, -Cl, - Br, -I, a hydroxyl group, a cyano group, a nitro group, an amidino group, a hydrazino group, a hydrazono group, a C₁-C₆₀ (e.g. C₁-C₂₀) alkyl group that is unsubstituted or substituted with at least one R₁₀ₐ, a C₂-C₆₀ (e.g. C₂-C₂₀) alkenyl group that is unsubstituted or substituted with at least one R₁₀ₐ, a C₂-C₆₀ (e.g. C₂-C₂₀) alkynyl group that is unsubstituted or substituted with at least one R₁₀ₐ, a C₁-C₆₀ (e.g. C₁-C₂₀) alkoxy group that is unsubstituted or substituted with at least one R₁₀ₐ, a C₃-C₁₀ cycloalkyl group that is unsubstituted or substituted with at least one R₁₀ₐ, a C₁-C₁₀ heterocycloalkyl group that is unsubstituted or substituted with at least one R₁₀ₐ, a C₃-C₁₀ cycloalkenyl group that is unsubstituted or substituted with at least one R₁₀ₐ, a C₁-C₁₀ heterocycloalkenyl group that is unsubstituted or substituted with at least one R₁₀ₐ, a C₆-C₆₀ (e.g. C₆-C₃₀) aryl group that is unsubstituted or substituted with at least one R₁₀ₐ, a C₆-C₆₀ (e.g. C₆-C₃₀) aryloxy group that is unsubstituted or substituted with at least one R₁₀ₐ, a C₆-C₆₀ (e.g. C₆-C₃₀) arylthio group that is unsubstituted or substituted with at least one R₁₀ₐ, a C₁-C₆₀ (e.g. C₁-C₂₀) heteroaryl group that is unsubstituted or substituted with at least one R₁₀ₐ, a C₁-C₆₀ (e.g. C₁-C₂₀) heteroaryloxy group that is unsubstituted or substituted with at least one R₁₀ₐ, a C₁-C₆₀ (e.g. C₁-C₂₀) heteroarylthio group that is unsubstituted or substituted with at least one R₁₀ₐ, a monovalent non-aromatic condensed polycyclic group that is unsubstituted or substituted with at least one R₁₀ₐ, a monovalent non-aromatic condensed heteropolycyclic group that is unsubstituted or substituted with at least one R₁₀ₐ, -Si(Q₁)(Q₂)(Q₃), -B(Q₁)(Q₂), - N(Q₁)(Q₂), -P(Q₁)(Q₂), -C(=O)(Q₁), -S(=O)(Q₁), -S(=O)₂(Q₁), -P(=O)(Q₁)(Q₂), or - P(=S)(Q₁)(Q₂),
two or more neighboring groups among Ar₁, Ar₂, and R₁ to R₅ are optionally bonded to each other to form a C₅-C₆₀ (e.g. C₅-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ,
R₁₀ₐ is:
deuterium, -F, -Cl, -Br, -I, a hydroxyl group, a cyano group, or a nitro group;
a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, or a C₁-C₆₀ (e.g. C₁-C₂₀) alkoxy group, each unsubstituted or substituted with deuterium, -F, -Cl, -Br, -I, a hydroxyl group, a cyano group, a nitro group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, a C₂-C₆₀ (e.g. C₂-C₃₀) heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), or any combination thereof;
a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, or a C₂-C₆₀ (e.g. C₂-C₃₀) heteroarylalkyl group, each unsubstituted or substituted with deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, a C₂-C₆₀ (e.g. C₂-C₃₀) heteroarylalkyl group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, or a C₁-C₆₀ (e.g. C₁-C₂₀) alkoxy group; or
a C₃-C₆₀ (e.g. C₃-C₃₀) carbocyclic group, a C₁-C₆₀ (e.g. C₁-C₂₀) heterocyclic group, a C₇-C₆₀ (e.g. C₇-C₃₀) arylalkyl group, or a C₂-C₆₀ (e.g. C₂-C₃₀) heteroarylalkyl group, each unsubstituted or substituted with deuterium, -F, a cyano group, a C₁-C₆₀ (e.g. C₁-C₂₀) alkyl group, a C₁-C₆₀ (e.g. C₁-C₂₀) alkoxy group, a phenyl group, a biphenyl group, or any combination thereof.

In an embodiment, ring CY₁ to ring CY₅ may each independently be a benzene group, a naphthalene group, an anthracene group, a phenanthrene group, a triphenylene group, a pyrene group, a chrysene group, an acenaphthylene group, a perylene group, a benzopyrene group, a benzochrysene group, a benzotriphenylene group, a fluoranthene group, a coronene group, a cyclopentadiene group, a 1,2,3,4-tetrahydronaphthalene group, an acridine group, a thiophene group, a furan group, an indole group, a benzoborole group, a benzophosphole group, an indene group, a benzosilole group, a benzogermole group, a benzothiophene group, a benzoselenophene group, a benzofuran group, a benzotellurophene group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzotellurophene group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, ring CY₁ to ring CY₅ may each independently be a benzene group, a naphthalene group, or a pyridine group.

In an embodiment, n1 may be 1 or 2.

In an embodiment, L₁ may be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ.

In an embodiment, L₁ may be a group represented by one of Formulae 3-1 to 3-30:

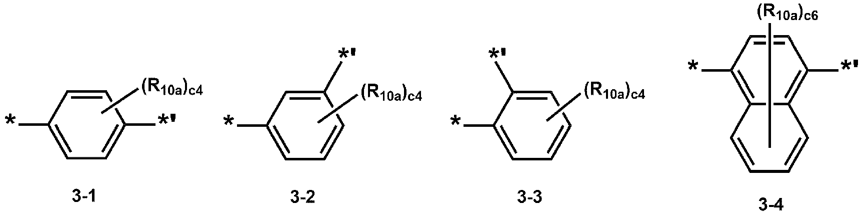

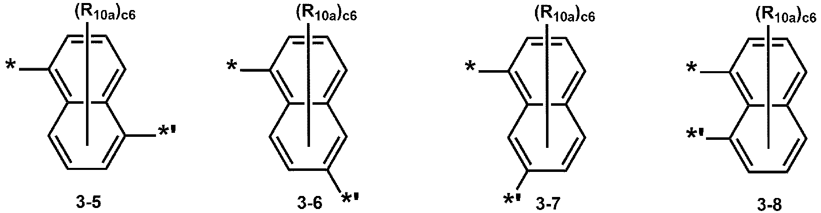

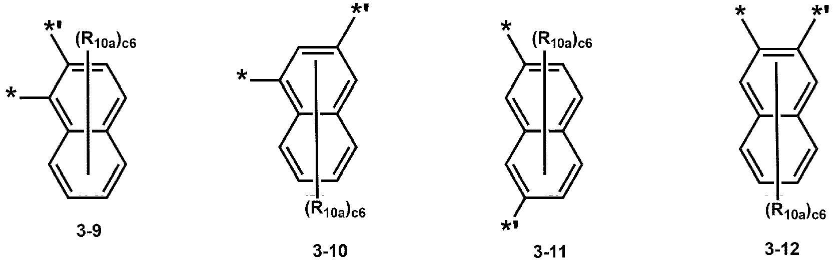

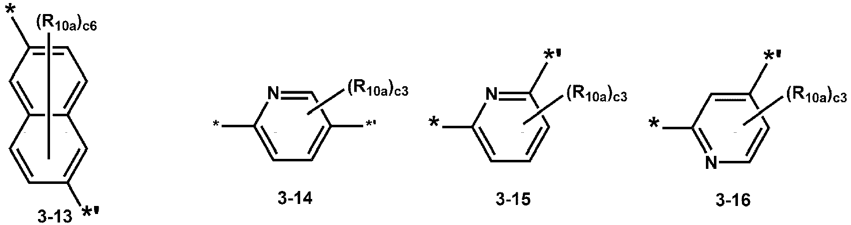

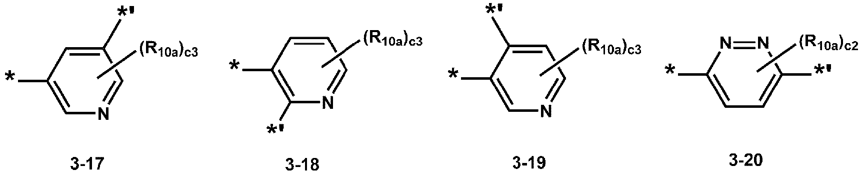

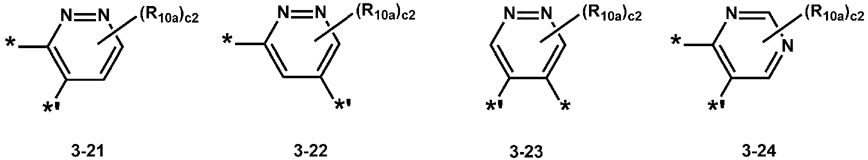

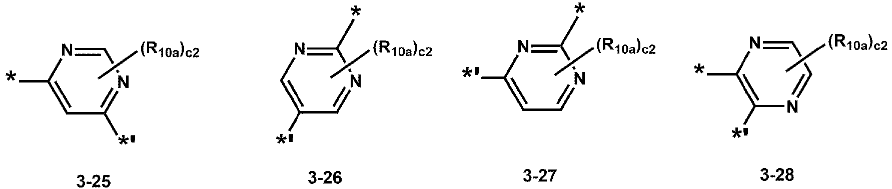

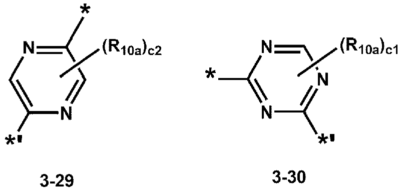

In Formulae 3-1 to 3-30,
R₁₀ₐ is the same as described herein,
c1 is 0 or 1,
c2 is an integer from 0 to 2,
c3 is an integer from 0 to 3,
c4 is an integer from 0 to 4,
c6 is an integer from 0 to 6, and
* and *' each indicate a binding site to a neighboring atom.

In an embodiment, L₁ may be a group represented by one of Formulae 3-1 to 3-3 and 3-14 to 3-19.

In an embodiment, in Formula 1, a moiety represented by 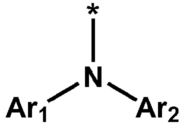 may be a moiety represented by Formula 6:

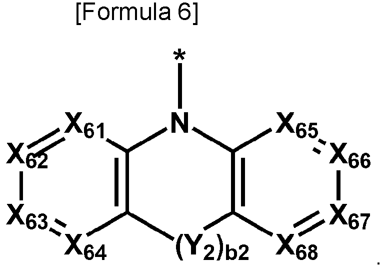

In Formula 6,
Y₂ is *'-N(Z₆ₐ)-*", *'-B(Z₆ₐ)-*", *'-P(Z₆ₐ)-*", *'-C(Z₆ₐ)(Z_{6b})-*", *'-Si(Z₆ₐ)(Z_{6b})-*", *'-Ge(Z₆ₐ)(Z_{6b})-*", *'-S-*", *'-Se-*", *'-O-*", *'-C(=O)-*", *'-S(=O)-*", *'-S(=O)₂-*", *'-C(=S)-*", *'-N=*", *'=N-*", *'-C(Z₆ₐ)=*", *'=C(Z₆ₐ)-*", *'-Si(Z₆ₐ)=*", *'=Si(Z₆ₐ)-*", *'-Ge(Z₆ₐ)=*", or *'=Ge(Z₆ₐ)-*", and *' and *" each indicate a binding site to a neighboring atom,
b2 is an integer from 0 to 2,
when b2 is 0, *-(Y₂)_{b2}-*' is a single bond,
when b2 is 2, two Y₂ are identical to or different from each other,
X₆₁ is N or C(Z₆₁), X₆₂ is N or C(Z₆₂), X₆₃ is N or C(Z₆₃), X₆₄ is N or C(Z₆₄), X₆₅ is N or C(Z₆₅), X₆₆ is N or C(Z₆₆), X₆₇ is N or C(Z₆₇), and X₆₈ is N or C(Z₆₈),
Z₆ₐ, Z_{6b}, and Z₆₁ to Z₆₈ are each independently the same as described in connection with R₁₀ₐ,
two or more neighboring groups among Z₆ₐ, Z_{6b}, and Z₆₁ to Z₆₈ are optionally bonded to each other to form a C₅-C₆₀ (e.g. C₅-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ, and
* indicates a binding site to a neighboring atom.

In an embodiment, in Formula 1, a moiety represented by 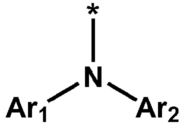 may be a moiety represented by one of Formulae 6-1 to 6-3:

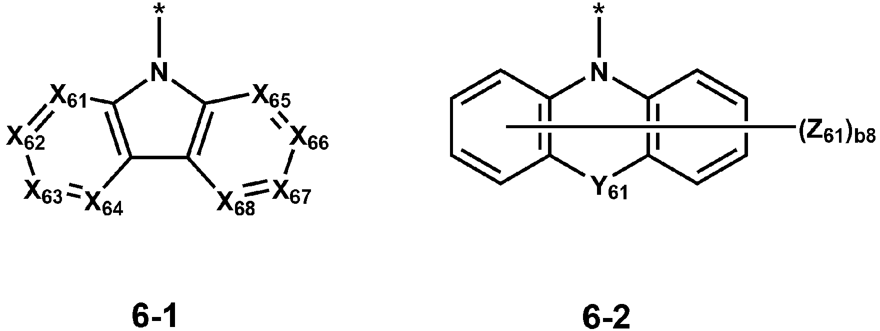

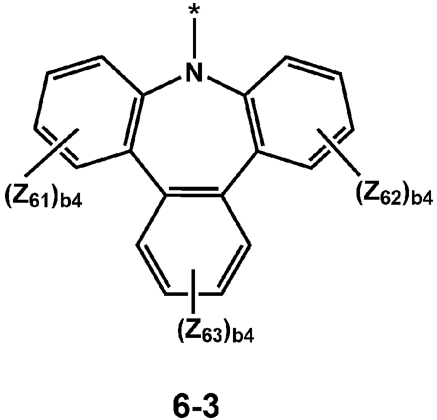

In Formulae 6-1 to 6-3,
X₆₁ is N or C(Z₆₁), X₆₂ is N or C(Z₆₂), X₆₃ is N or C(Z₆₃), X₆₄ is N or C(Z₆₄), X₆₅ is N or C(Z₆₅), X₆₆ is N or C(Z₆₆), X₆₇ is N or C(Z₆₇), and X₆₈ is N or C(Z₆₈),
Y₆₁ is O, S, Se, C(Z₆ₐ)(Z_{6b}), Si(Z₆ₐ)(Z_{6b}), or N(Z₆ₐ),
Z₆ₐ, Z_{6b}, and Z₆₁ to Z₆₈ are each independently the same as described in connection with R₁₀ₐ,
b4 is an integer from 0 to 4,
b8 is an integer from 0 to 8,
two or more neighboring groups among Z₆ₐ, Z_{6b}, and Z₆₁ to Z₆₈ are optionally bonded to each other to form a C₅-C₆₀ (e.g. C₅-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ, and
* indicates a binding site to a neighboring atom.

In an embodiment, R₁ to R₅ may each independently be:
hydrogen, deuterium, -F, -Cl, -Br, -I, a hydroxyl group, a cyano group, a nitro group, a C₁-C₂₀ (e.g. C₁-C₁₀) alkyl group, or a C₁-C₂₀ (e.g. C₁-C₁₀) alkoxy group;
a C₁-C₂₀ (e.g. C₁-C₁₀) alkyl group or a C₁-C₂₀ (e.g. C₁-C₁₀) alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzothiazolyl group, a benzoisoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzosilolyl group, a dibenzocarbazolyl group, an imidazopyridinyl group, an imidazopyrimidinyl group, -O(Q₃₁), -S(Q₃₁), -Si(Q₃₁)(Q₃₂)(Q₃₃), -N(Q₃₁)(Q₃₂), - B(Q₃₁)(Q₃₂), -P(Q₃₁)(Q₃₂), -C(=O)(Q₃₁), -S(=O)₂(Q₃₁), -P(=O)(Q₃₁)(Q₃₂), or any combination thereof; or
-C(Q₁)(Q₂)(Q₃), -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
Q₁ to Q₃ and Q₃₁ to Q₃₃ may each independently be: hydrogen; deuterium; -F; -CI; -Br; -I; a hydroxyl group; a cyano group; a nitro group; a C₁-C₆₀ (e.g. C₁-C₂₀) alkyl group; a C₂-C₆₀ (e.g. C₂-C₂₀) alkenyl group; a C₂-C₆₀ (e.g. C₂-C₂₀) alkynyl group; a C₁-C₆₀ (e.g. C₁-C₂₀) alkoxy group; or a C₃-C₆₀ (e.g. C₃-C₃₀) carbocyclic group or a C₁-C₆₀ (e.g. C₁-C₂₀) heterocyclic group, each unsubstituted or substituted with deuterium, -F, a cyano group, a C₁-C₆₀ (e.g. C₁-C₂₀) alkyl group, a C₁-C₆₀ (e.g. C₁-C₂₀) alkoxy group, a phenyl group, a biphenyl group, or any combination thereof.

In an embodiment, the heterocyclic compound may be represented by one of Formulae 1-1 to 1-4:

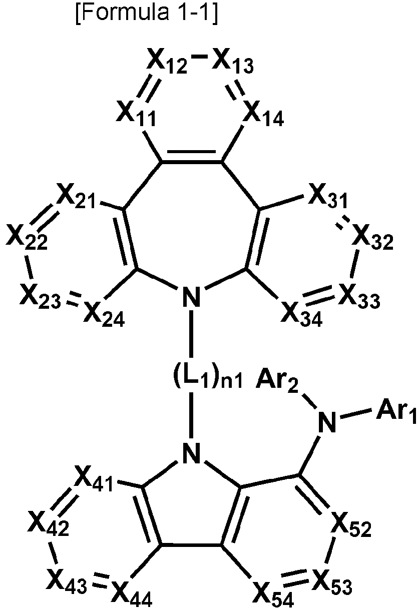

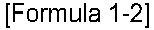

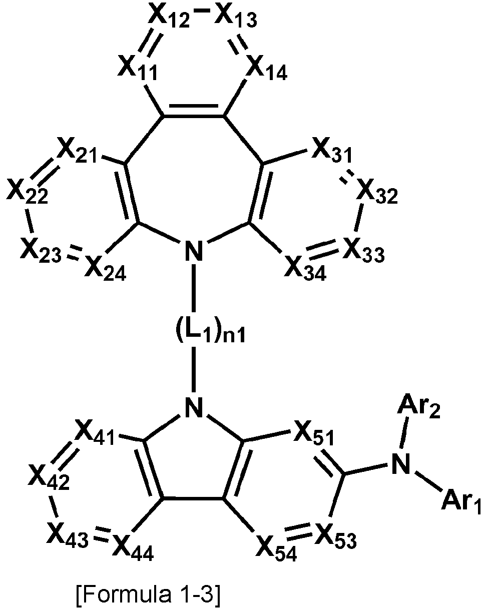

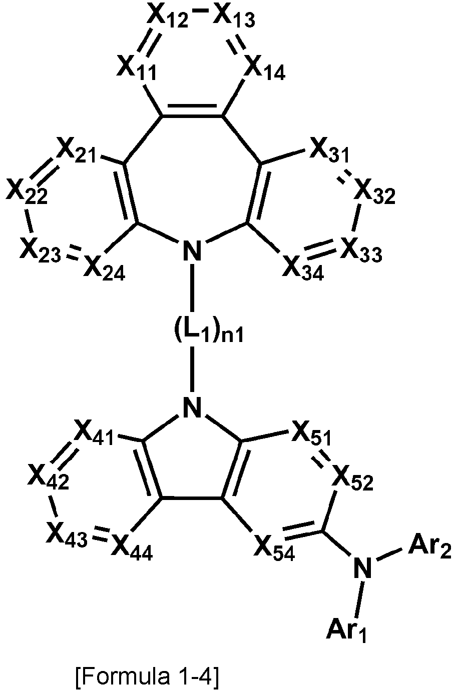

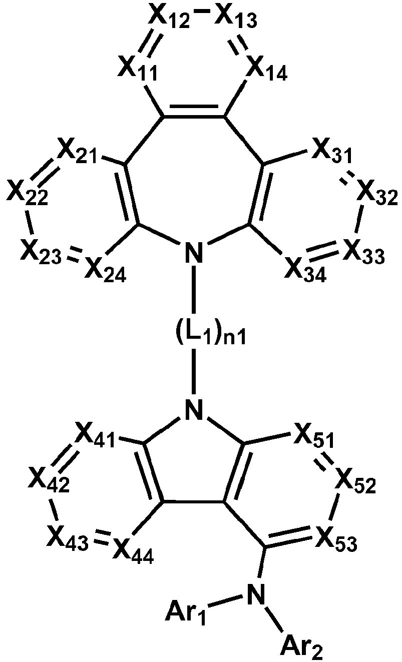

In Formulae 1-1 to 1-4,
n1, L₁, Ar₁, and Ar₂ are each the same as described herein,
X₁₁ is N or C(R₁₁), X₁₂ is N or C(R₁₂), X₁₃ is N or C(R₁₃), and X₁₄ is N or C(R₁₄),
X₂₁ is N or C(R₂₁), X₂₂ is N or C(R₂₂), X₂₃ is N or C(R₂₃), and X₂₄ is N or C(R₂₄),
X₃₁ is N or C(R₃₁), X₃₂ is N or C(R₃₂), X₃₃ is N or C(R₃₃), and X₃₄ is N or C(R₃₄),
X₄₁ is N or C(R₄₁), X₄₂ is N or C(R₄₂), X₄₃ is N or C(R₄₃), and X₄₄ is N or C(R₄₄),
X₅₁ is N or C(R₅₁), X₅₂ is N or C(R₅₂), X₅₃ is N or C(R₅₃), and X₅₄ is N or C(R₅₄),
R₁₁ to R₁₄ are each independently the same as described in connection with R1,
R₂₁ to R₂₄ are each independently the same as described in connection with R2,
R₃₁ to R₃₄ are each independently the same as described in connection with R3,
R₄₁ to R₄₄ are each independently the same as described in connection with R₄, and
R₅₁ to R₅₄ are each independently the same as described in connection with R₅.

In an embodiment, the heterocyclic compound represented by Formula 1 may include at least one deuterium.

In an embodiment, the heterocyclic compound represented by Formula 1 may be one of Compounds 1 to 28 and 30 to 100:

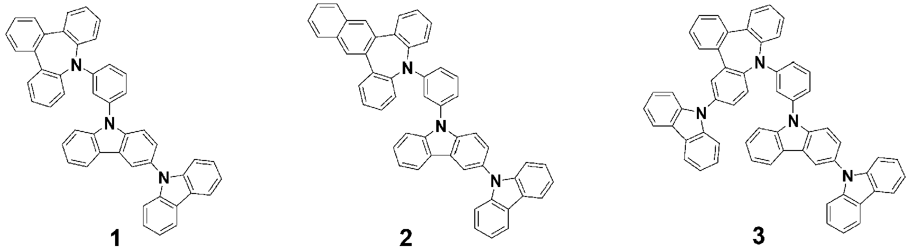

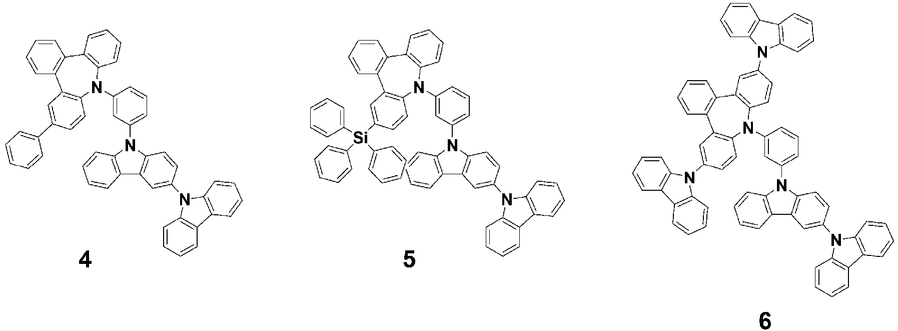

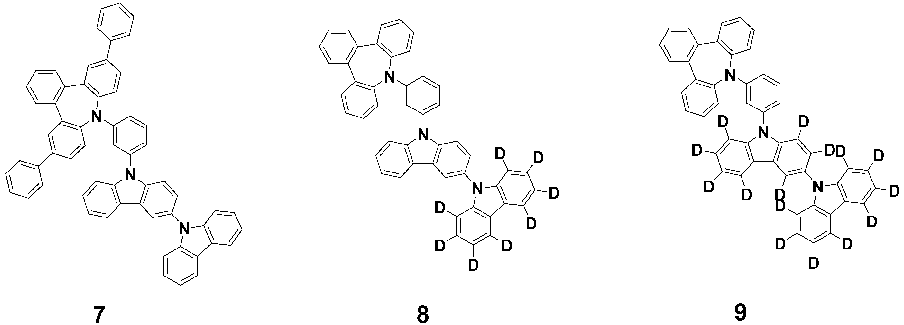

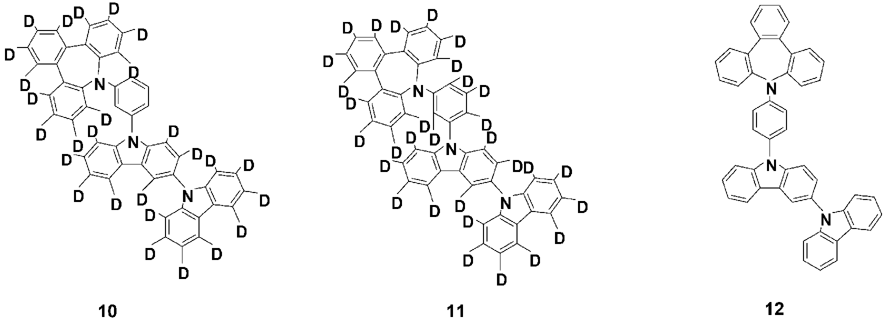

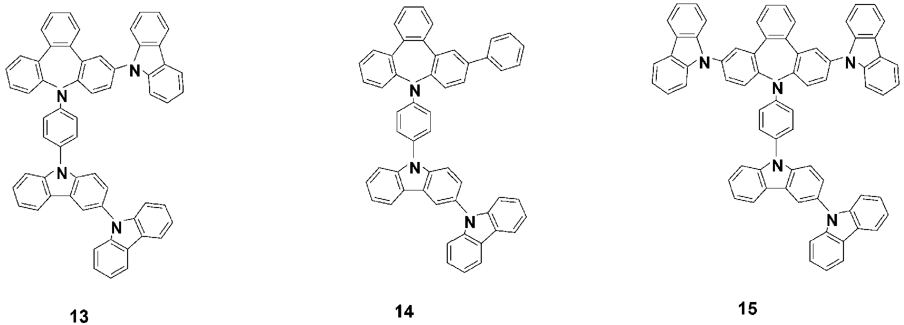

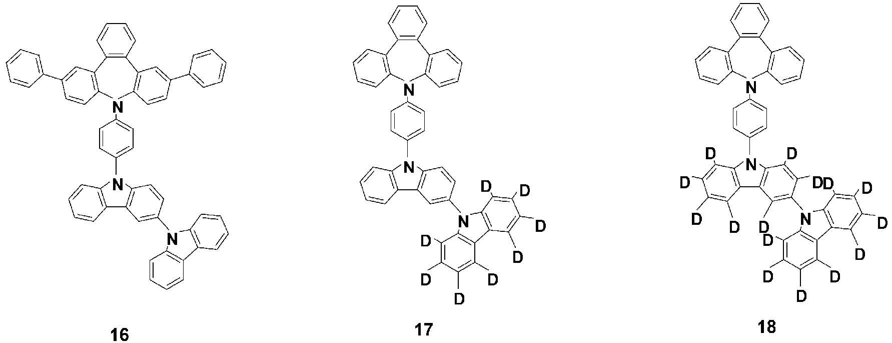

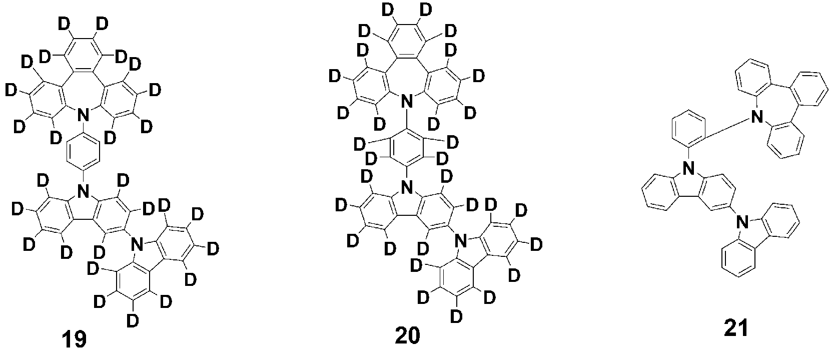

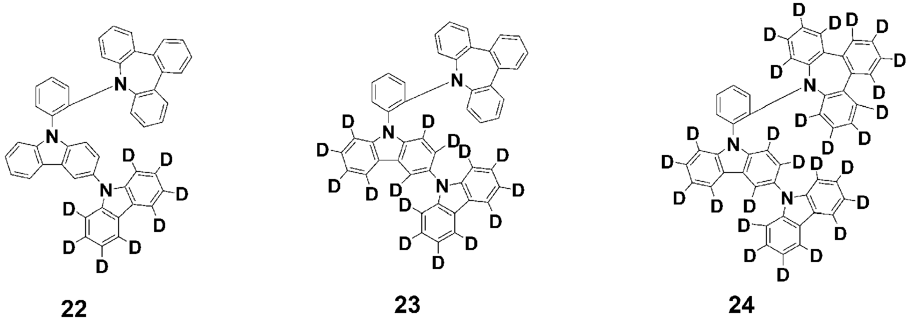

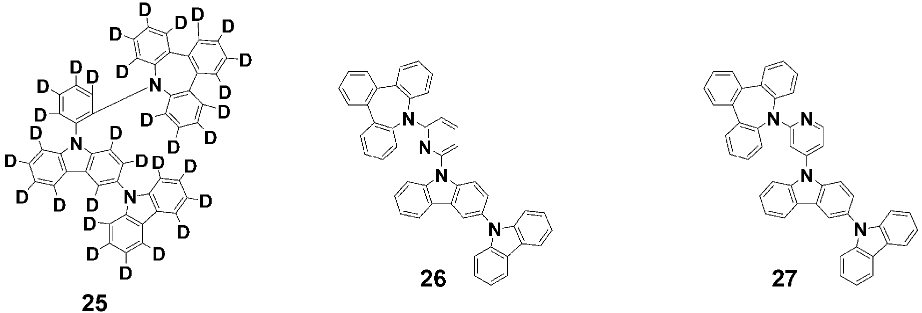

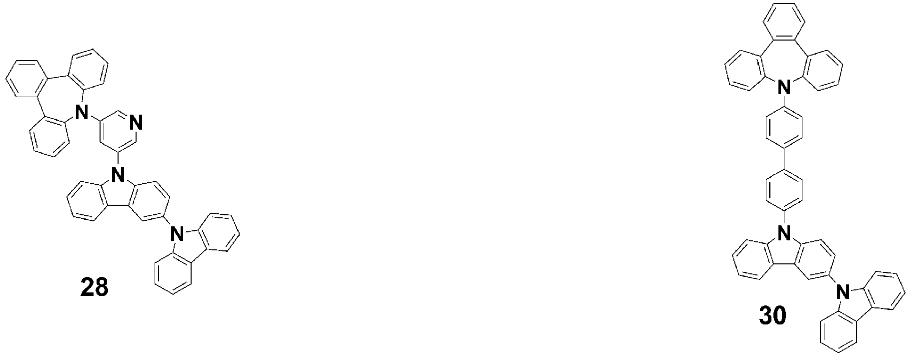

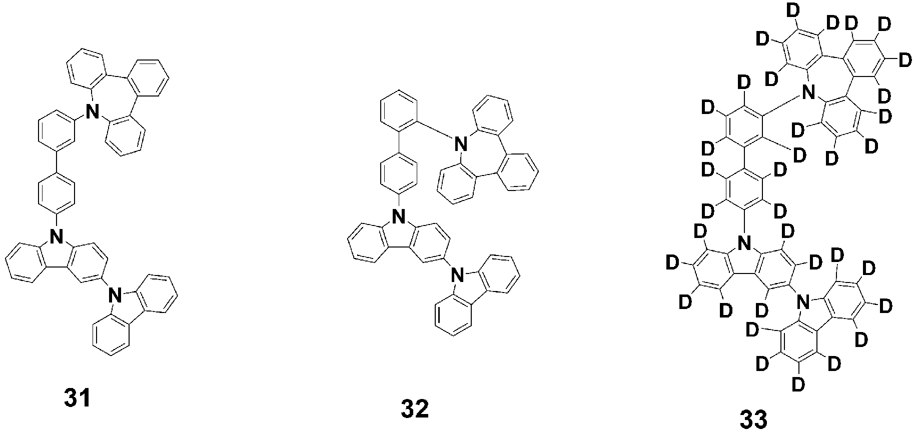

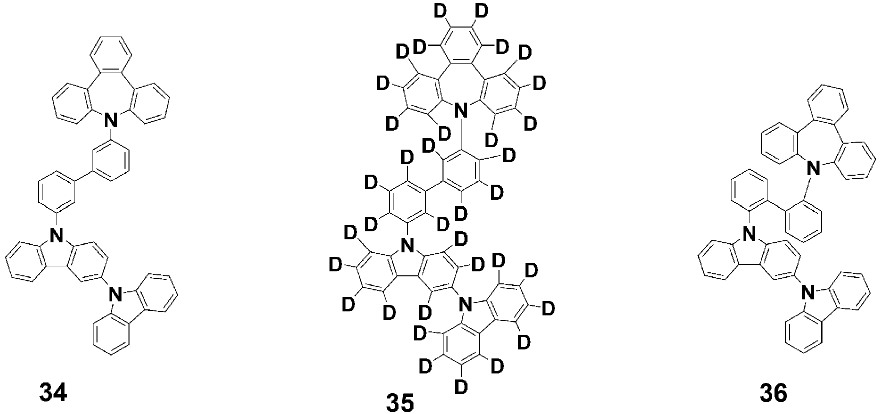

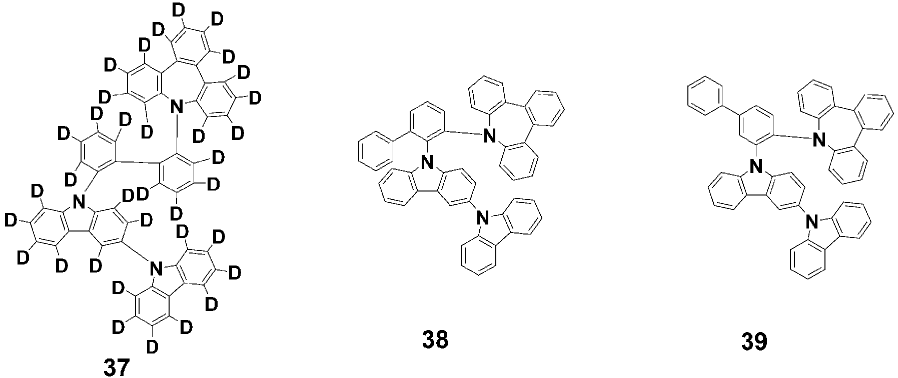

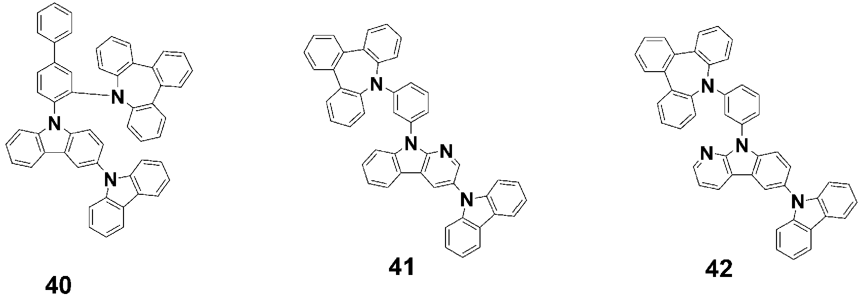

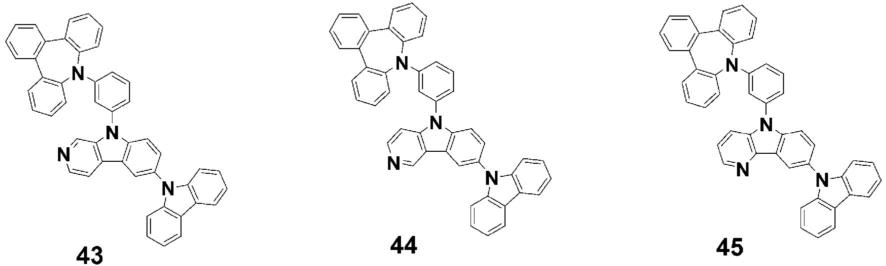

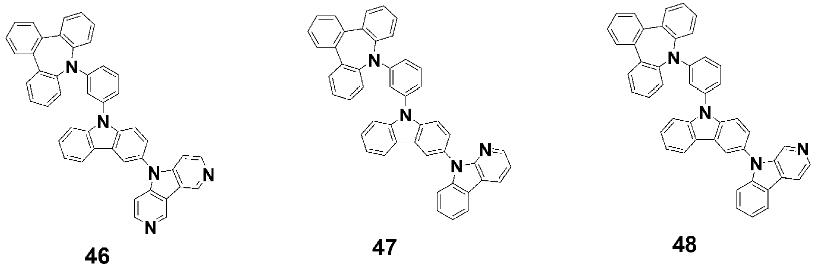

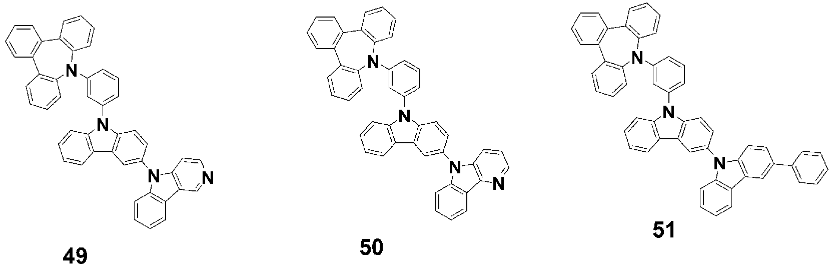

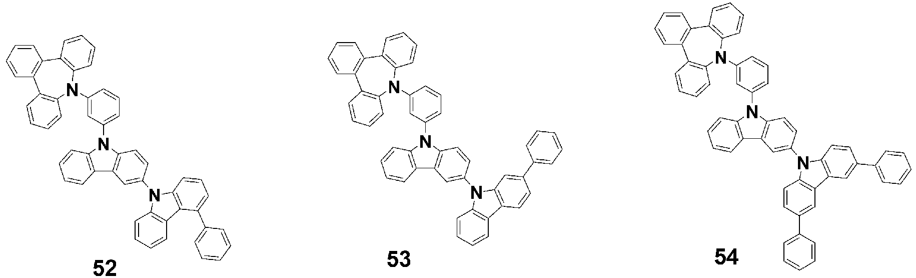

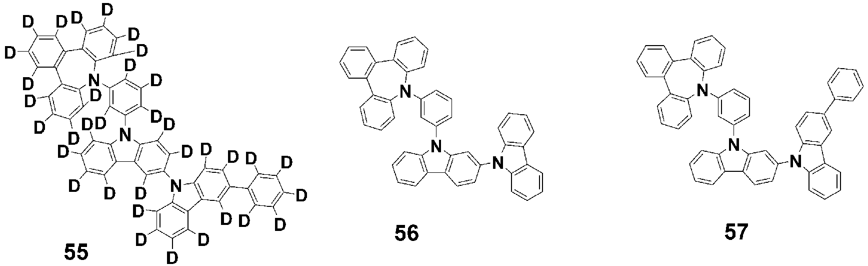

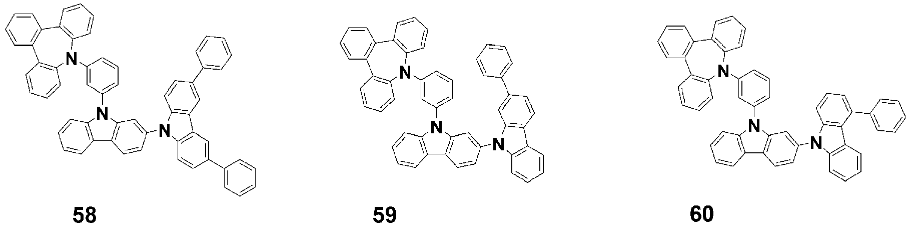

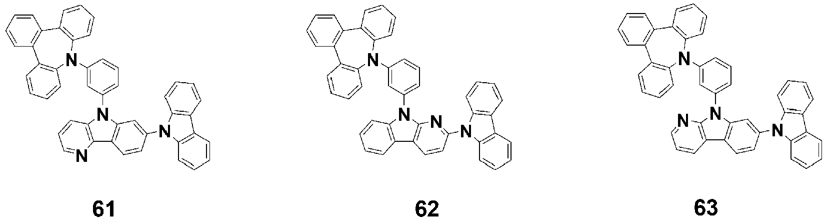

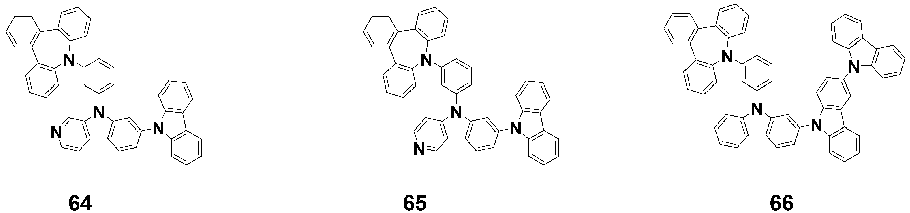

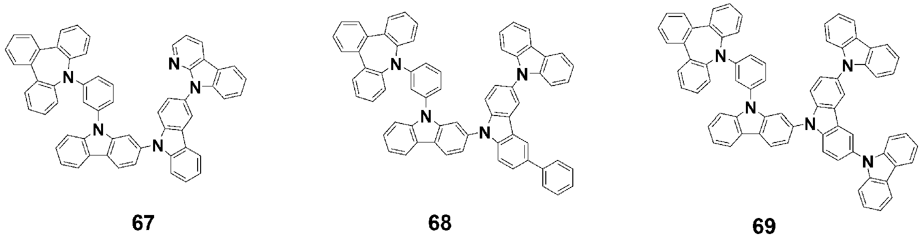

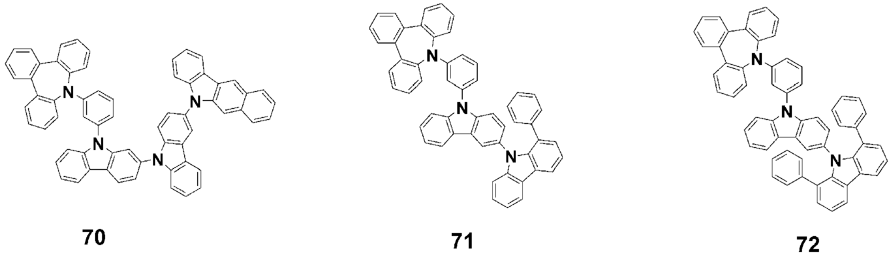

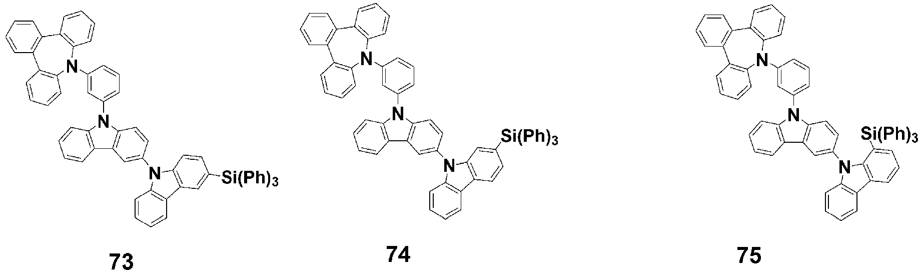

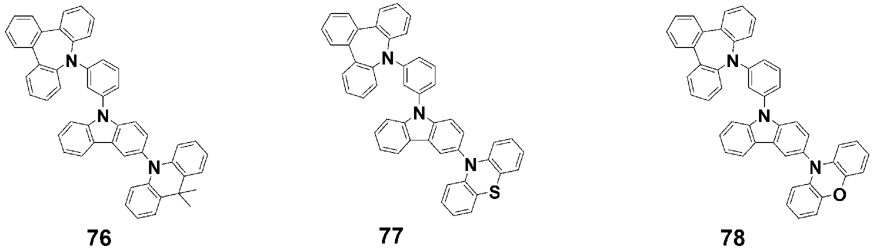

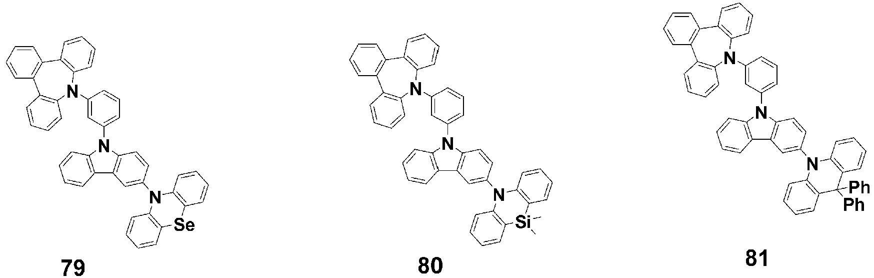

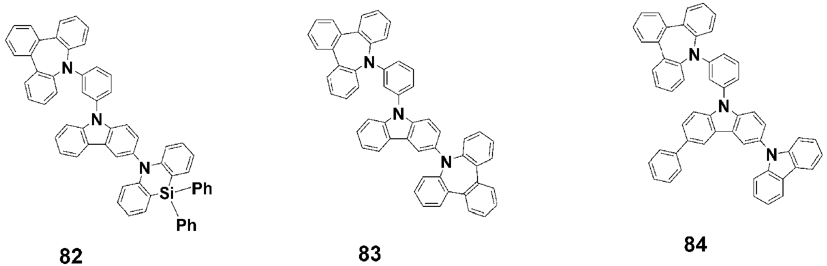

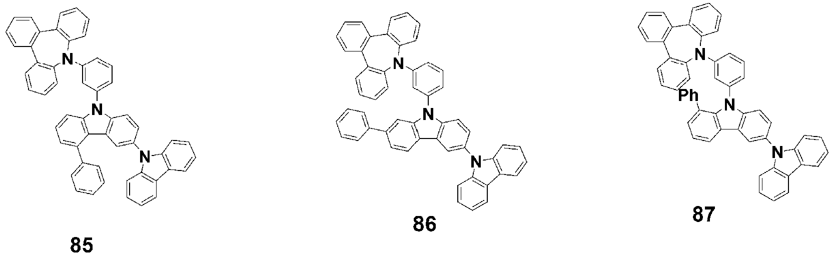

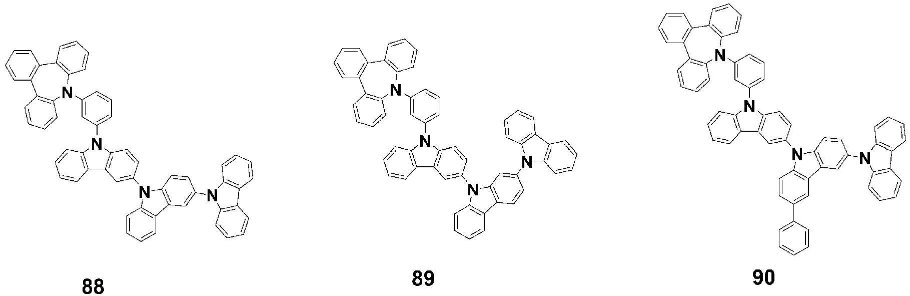

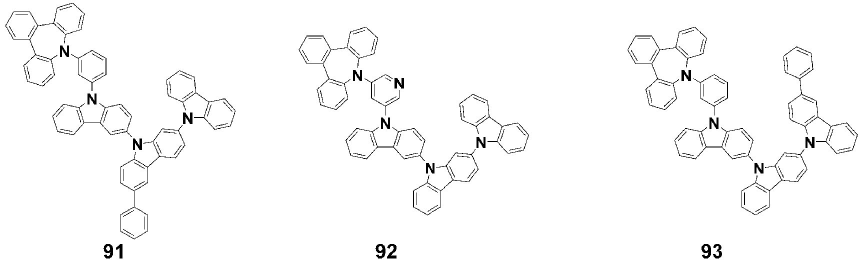

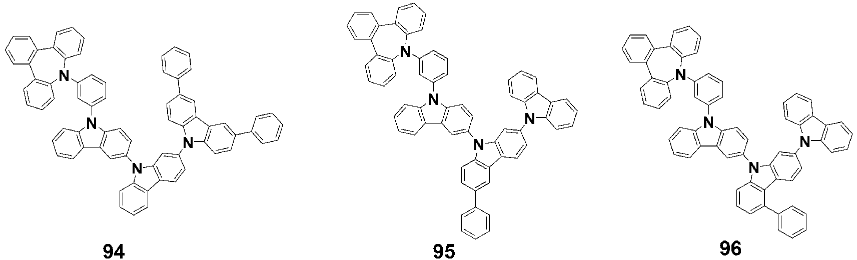

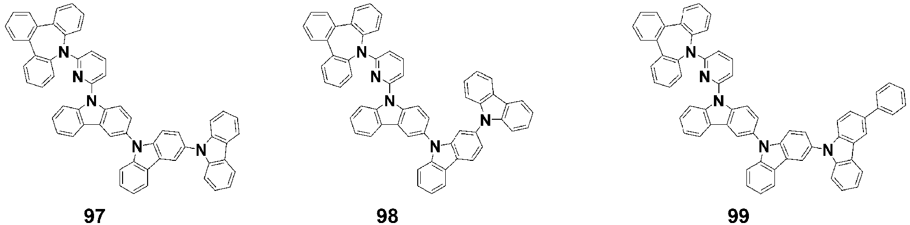

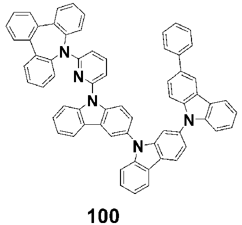

The heterocyclic compound represented by Formula 1 may inhibit intermolecular interactions due to a non-planar structure of an N-containing ring with at least seven members. Therefore, when the heterocyclic compound is used in the emission layer, color purity and lifespan may be improved.

In an embodiment, the heterocyclic compound includes at least one N-containing heterocyclic ring, which may facilitate control of hole transporting characteristics. As a result, a recombination zone in the emission layer is optimized, such that when the heterocyclic compound is used in the emission layer, luminescence efficiency may be improved.

Therefore, a light-emitting device including the heterocyclic compound may have excellent characteristics in terms of driving voltage, efficiency, and lifespan.

In an embodiment, the heterocyclic compound represented by Formula 1 may have a highest occupied molecular orbital (HOMO) energy level greater than or equal to about -5.8 eV.

In an embodiment, the heterocyclic compound represented by Formula 1 may have an energy level of a triplet excited state (T₁) that is greater than or equal to about 2.6 eV.

Methods of synthesizing the heterocyclic compound represented by Formula 1 may be readily understood by those of ordinary skill in the art by referring to the Synthesis Examples and/or the Examples described herein.

At least one of the heterocyclic compounds represented by Formula 1 is used in a light-emitting device (for example, an organic light-emitting device). Therefore, embodiments provide a light-emitting device which includes: a first electrode; a second electrode facing the first electrode; an interlayer between the first electrode and the second electrode and including an emission layer; and the heterocyclic compound represented by Formula 1.

In an embodiment,
the first electrode of the light-emitting device may be an anode,
the second electrode of the light-emitting device may be a cathode,
the interlayer may further include a hole transport region between the first electrode and the emission layer, and an electron transport region between the emission layer and the second electrode,
the hole transport region may include a hole injection layer, a hole transport layer, an emission auxiliary layer, an electron-blocking layer, or any combination thereof, and
the electron transport region may include a buffer layer, a hole-blocking layer, an electron control layer, an electron transport layer, an electron injection layer, or any combination thereof.

The hole transport layer may include a single layer or two or more layers, and the electron transport layer may include a single layer or two or more layers.

In an embodiment, the heterocyclic compound may be included between the first electrode and the second electrode of the light-emitting device. Therefore, the heterocyclic compound may be included in the interlayer of the light-emitting device, for example, the emission layer in the interlayer.

In an embodiment, the emission layer in the interlayer of the light-emitting device may include a dopant and a host, wherein the host may include the heterocyclic compound. For example, the heterocyclic compound may serve as a host. The emission layer may emit red light, green light, blue light, and/or white light. In an embodiment, the emission layer may emit blue light. The blue light may have a maximum emission wavelength in a range of, for example, about 400 nm to about 490 nm. The heterocyclic compound may emit blue light having a wavelength in a range of, for example, about 430 nm to about 480 nm.

In an embodiment, the emission layer in the interlayer of the light-emitting device may include a dopant and a host, wherein the host may include the heterocyclic compound, and the dopant may emit blue light. For example, the dopant may include a transition metal and ligand(s) in the number of m, and m may be an integer from 1 to 6. The ligand(s) in the number of m may be identical to or different from each other, at least one of the ligand(s) in the number of m may be linked to the transition metal via a carbon-transition metal bond, and the carbon-transition metal bond may be a coordinate bond. For example, at least one ligand(s) in the number of m may be a carbene ligand (for example, Ir(pmp)₃). The transition metal may be, for example, iridium, platinum, osmium, palladium, rhodium, gold, etc. The emission layer and the dopant may be the same as described herein.

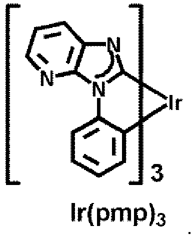

In an embodiment, the light-emitting device may further include a capping layer outside the first electrode or outside the second electrode.

In an embodiment, the light-emitting device may further include at least one of a first capping layer outside the first electrode and a second capping layer outside the second electrode, wherein at least one of the first capping layer and the second capping layer may each independently include the heterocyclic compound represented by Formula 1. The first capping layer and/or the second capping layer may be the same as described herein.

In an embodiment, the light-emitting device may further include a first capping layer outside the first electrode. For example, the first capping layer may include the heterocyclic compound represented by Formula 1.

In an embodiment, the light-emitting device may further include a second capping layer outside the second electrode. For example, the second capping layer may include the heterocyclic compound represented by Formula 1.

In an embodiment, the light-emitting device may further include a first capping layer outside the first electrode and a second capping layer outside the second electrode. For example, at least one of the first capping layer and the second capping layer may each independently include the heterocyclic compound represented by Formula 1.

In the specification, the wording "(interlayer and/or capping layer) includes a heterocyclic compound" may be understood as "(interlayer and/or capping layer) may include a heterocyclic compound represented by Formula 1 or two or more different heterocyclic compounds, each independently represented by Formula 1".

In an embodiment, the interlayer and/or the capping layer may include Compound 1 only as the heterocyclic compound. For example, Compound 1 may be included in the emission layer of the light-emitting device. In an embodiment, the interlayer may include, as the heterocyclic compound, Compound 1 and Compound 2. For example Compound 1 and Compound 2 may be included in a same layer (for example, both Compound 1 and Compound 2 may be included in the emission layer), or may be included in different layers (for example, Compound 1 may be included in the emission layer and Compound 2 may be included in the electron transport region).

In the specification, the term "interlayer" may refer to a single layer and/or multiple layers between a first electrode and a second electrode of a light-emitting device.

According to embodiments, an electronic apparatus includes the light-emitting device. The electronic apparatus may further include a thin-film transistor. For example, the electronic apparatus may further include a thin-film transistor including a source electrode and a drain electrode, wherein the first electrode of the light-emitting device may be electrically connected to the source electrode or the drain electrode. In an embodiment, the electronic apparatus may further include a color filter, a color conversion layer, a touch screen layer, a polarizing layer, or any combination thereof. The electronic apparatus may be the same as described herein.

According to embodiments, an electronic equipment includes the light-emitting device as described above. The electronic equipment may be a flat panel display, a curved display, a computer monitor, a medical monitor, a television, an advertisement board, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a microdisplay, a three-dimensional (3D) display, a virtual reality display, an augmented reality display, a vehicle, a video wall with multiple displays tiled together, a theater screen, a stadium screen, a phototherapy device, or a signboard. The electronic equipment may be the same as described herein.

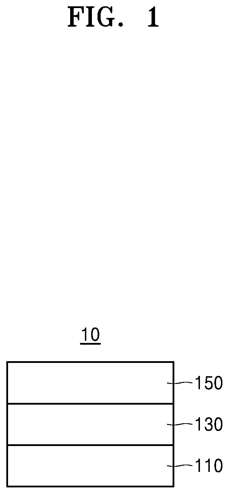

### [Description of FIG. 1]

FIG. 1 is a schematic cross-sectional view of a light-emitting device 10 according to an embodiment. The light-emitting device 10 includes a first electrode 110, an interlayer 130, and a second electrode 150.

Hereinafter, a structure of the light-emitting device 10 according to an embodiment and a method of manufacturing the light-emitting device 10 are described with reference to FIG. 1.

### [First electrode 110]

In FIG. 1, a substrate may be further included under the first electrode 110 or on the second electrode 150. In an embodiment, the substrate may be a glass substrate or a plastic substrate. In an embodiment, the substrate may be a flexible substrate and may include plastics with excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a transflective electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In an embodiment, when the first electrode 110 is a transflective electrode or a reflective electrode, a material for forming the first electrode 110 may include magnesium (Mg), silver (Ag), aluminium (Al), aluminium-lithium (Al-Li), calcium (Ca), magnesium-indium (Mg-In), magnesium-silver (Mg-Ag), or any combination thereof.

The first electrode 110 may have a structure consisting of a single layer or a structure including multiple layers. In an embodiment, the first electrode 110 may have a three-layered structure of ITO/Ag/ITO.

### [Interlayer 130]

The interlayer 130 may be arranged on the first electrode 110. The interlayer 130 may include an emission layer.

The interlayer 130 may further include a hole transport region between the first electrode 110 and the emission layer, and an electron transport region between the emission layer and the second electrode 150.

The interlayer 130 may further include, in addition to various organic materials, a metal-containing compound such as an organometallic compound, an inorganic material such as quantum dots, or the like.

In an embodiment, the interlayer 130 may include two or more emitting units stacked between the first electrode 110 and the second electrode 150, and at least one charge generation layer, each between adjacent emitting units among the two or more emitting units. When the interlayer 130 includes the two or more emitting units and the at least one charge generation layer as described above, the light-emitting device 10 may be a tandem light-emitting device.

### [Hole transport region in interlayer 130]

The hole transport region may have a structure consisting of a layer consisting of a single material, a structure consisting of a layer including different materials, or a structure including multiple layers including different materials.

The hole transport region may include a hole injection layer, a hole transport layer, an emission auxiliary layer, an electron-blocking layer, or any combination thereof.

In an embodiment, the hole transport region may have a multilayered structure, for example, a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein the layers of each structure may be stacked from the first electrode 110 in its respective stated order, but the structure of the hole transport region is not thereto.

In embodiments, the hole transport region may include a compound represented by Formula 201, a compound represented by Formula 202, or any combination thereof:

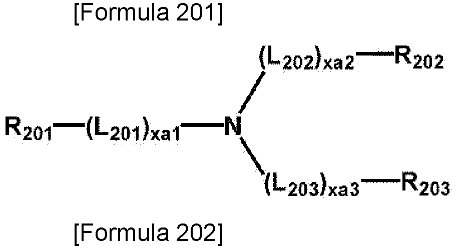

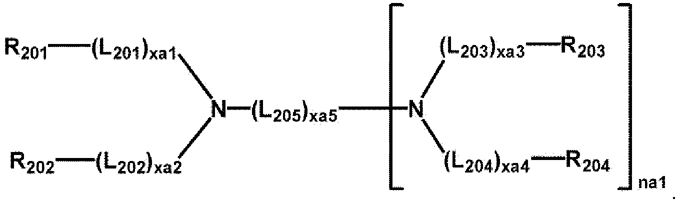

In Formulae 201 and 202,
L₂₀₁ to L₂₀₄ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
L₂₀₅ may be *-O-*', *-S-*', *-N(Q₂₀₁)-*', a C₁-C₂₀ alkylene group that is unsubstituted or substituted with at least one R₁₀ₐ, a C₂-C₂₀ alkenylene group that is unsubstituted or substituted with at least one R₁₀ₐ, a C₃-C₆₀ carbocyclic group that is unsubstituted or substituted with at least one R₁₀ₐ, or a C₁-C₆₀ heterocyclic group that is unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
R₂₀₁ and R₂₀₂ may optionally be linked to each other via a single bond, a C₁-C₅ alkylene group that is unsubstituted or substituted with at least one R₁₀ₐ, or a C₂-C₅ alkenylene group that is unsubstituted or substituted with at least one R₁₀ₐ to form a C₈-C₆₀ polycyclic group (for example, a carbazole group) that is unsubstituted or substituted with at least one R₁₀ₐ (for example, Compound HT16),
R₂₀₃ and R₂₀₄ may optionally be linked to each other via a single bond, a C₁-C₅ alkylene group that is unsubstituted or substituted with at least one R₁₀ₐ, or a C₂-C₅ alkenylene group that is unsubstituted or substituted with at least one R₁₀ₐ to form a C₈-C₆₀ polycyclic group that is unsubstituted or substituted with at least one R₁₀ₐ, and
na1 may be an integer from 1 to 4.

In an embodiment, the compound represented by Formula 201 and the compound represented by Formula 202 may each independently include at least one of groups represented by Formulae CY201 to CY217:

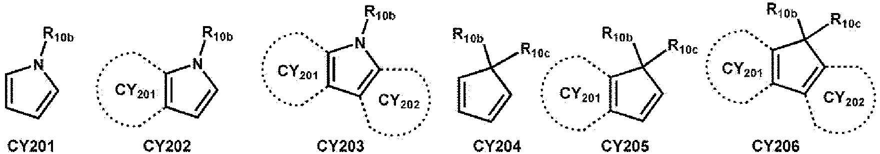

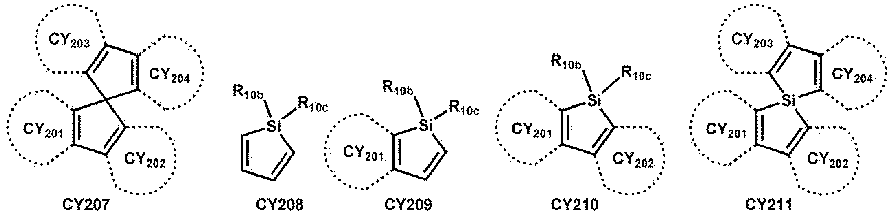

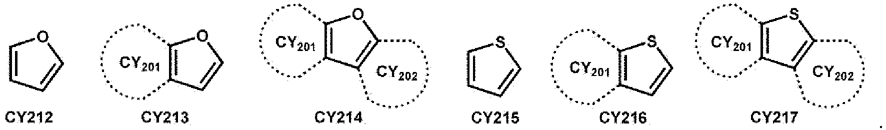

In Formulae CY201 to CY217, R_{10b} and R_{10c} may each independently be the same as described in connection with R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ as described herein.

In an embodiment, in Formulae CY201 to CY217, ring CY₂₀₁ to ring CY₂₀₄ may each independently be a benzene group, a naphthalene group, a phenanthrene group, or an anthracene group.

In an embodiment, the compound represented by Formula 201 and the compound represented by Formula 202 may include at least one of groups represented by Formulae CY201 to CY203.

In an embodiment, the compound represented by Formula 201 may include at least one of groups represented by Formulae CY201 to CY203 and at least one of groups represented by Formulae CY204 to CY217.

In an embodiment, in Formula 201, xa1 may be 1, R₂₀₁ may be a group represented by one of Formulae CY201 to CY203, xa2 may be 0, and R₂₀₂ may be a group represented by one of Formulae CY204 to CY207.

In an embodiment, the compound represented by Formula 201 and the compound represented by Formula 202 may each not include groups represented by Formulae CY201 to CY203.

In an embodiment, the compound represented by Formula 201 and the compound represented by Formula 202 may each not include groups represented by Formulae CY201 to CY203 and may each independently include at least one of groups represented by Formulae CY204 to CY217.

In an embodiment, the compound represented by Formula 201 and the compound represented by Formula 202 may each not include groups represented by Formulae CY201 to CY217.

For example, the hole transport region may include one of Compounds HT1 to HT46, m-MTDATA, TDATA, 2-TNATA, NPB (NPD), β-NPB, TPD, spiro-TPD, spiro-NPB, methylated NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), polyaniline/poly(4-styrenesulfonate) (PANI/PSS), or any combination thereof:

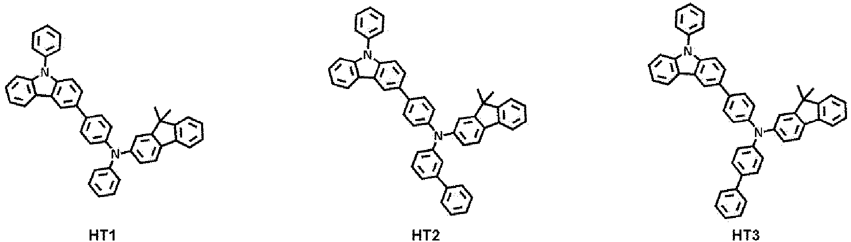

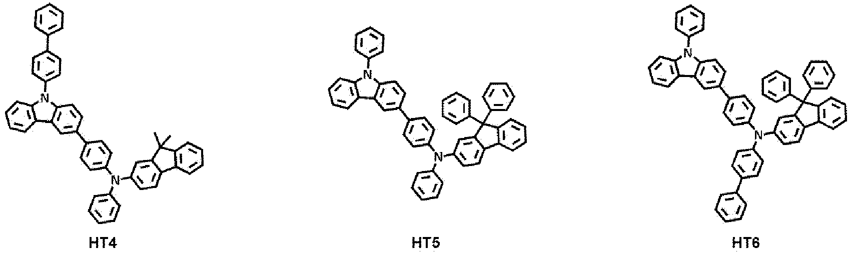

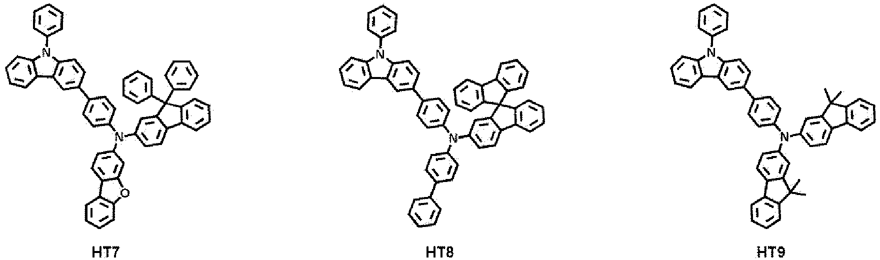

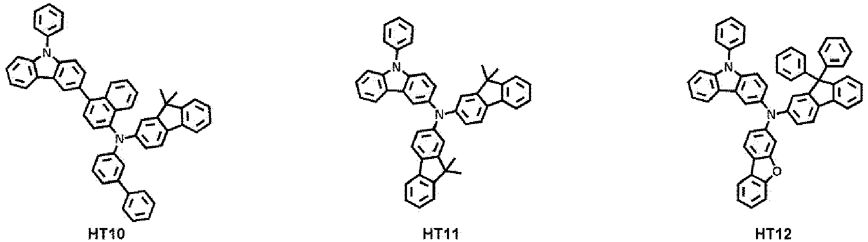

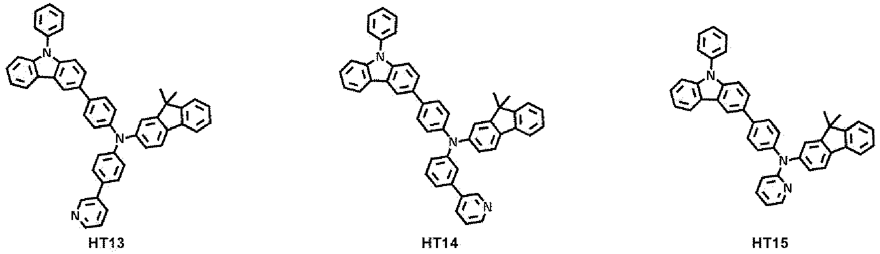

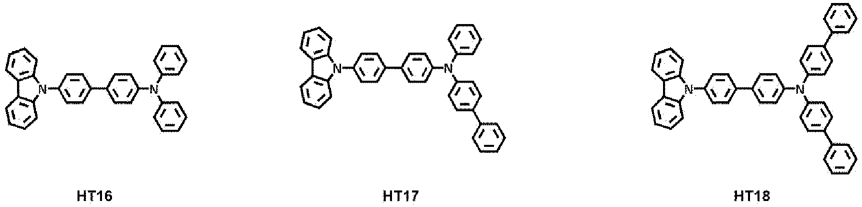

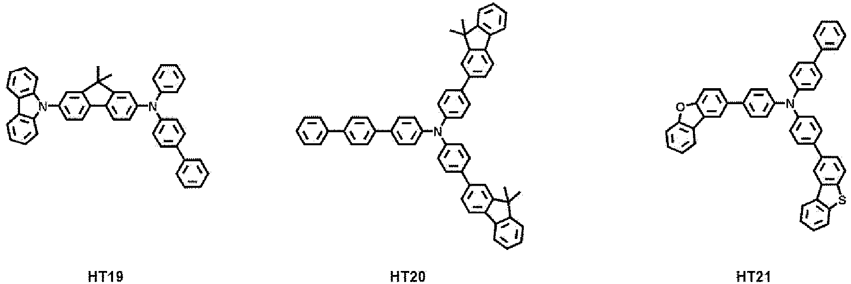

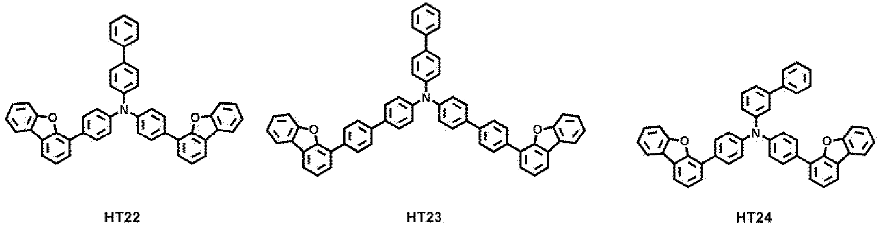

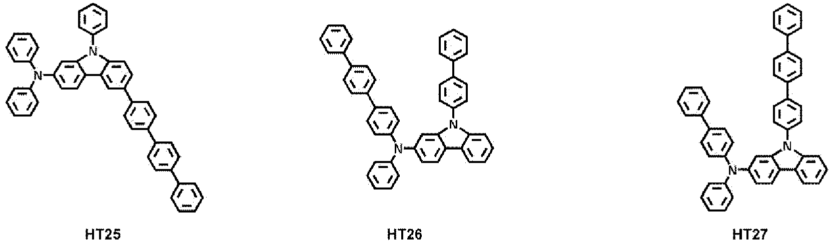

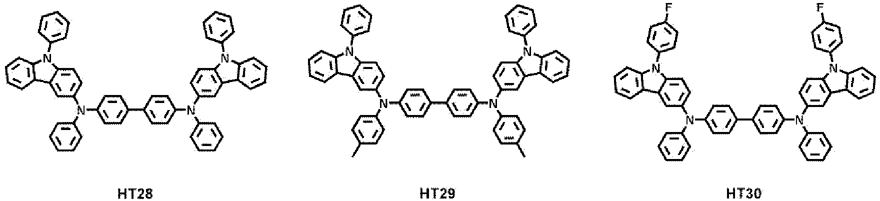

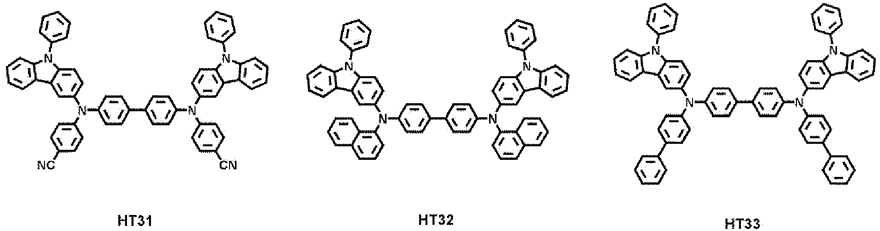

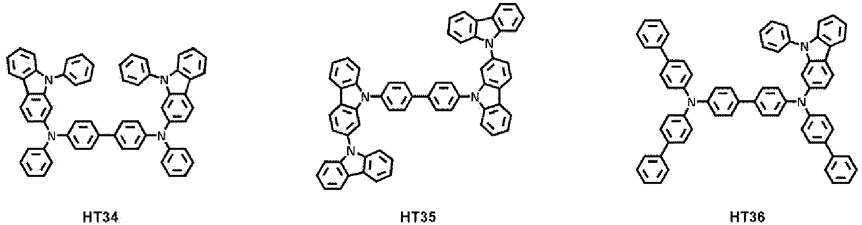

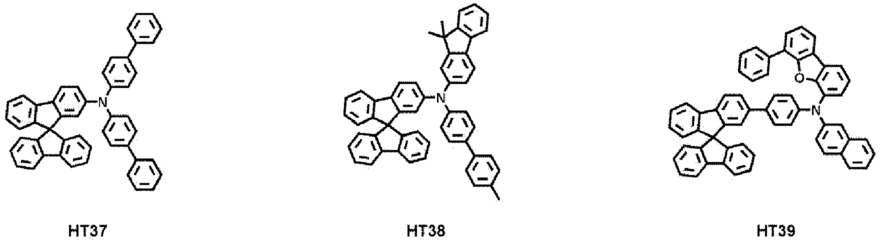

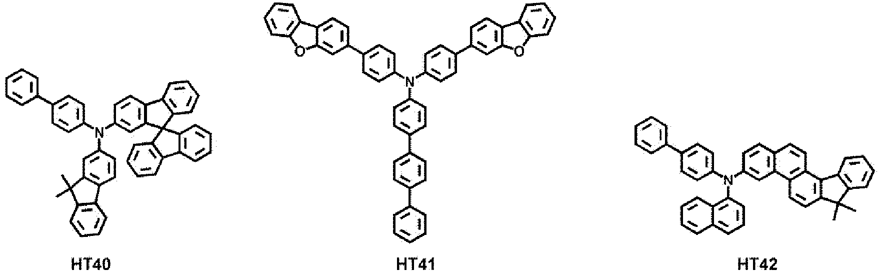

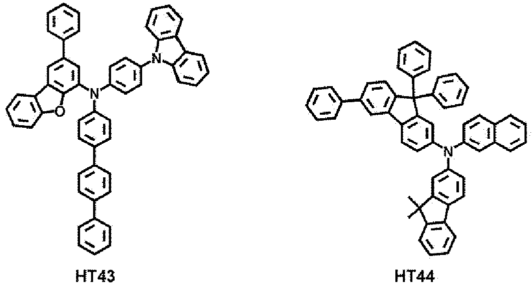

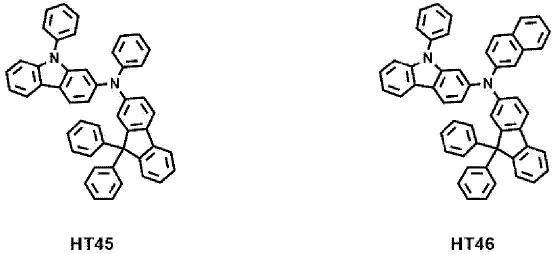

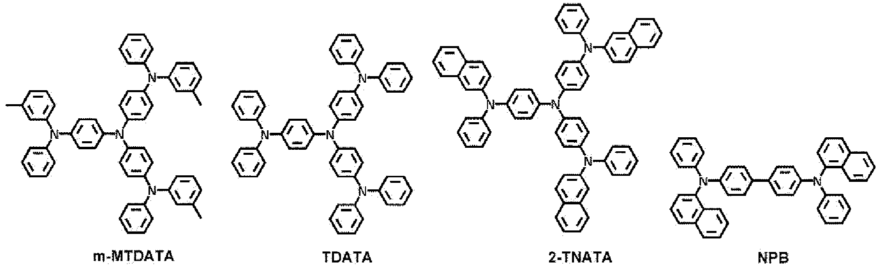

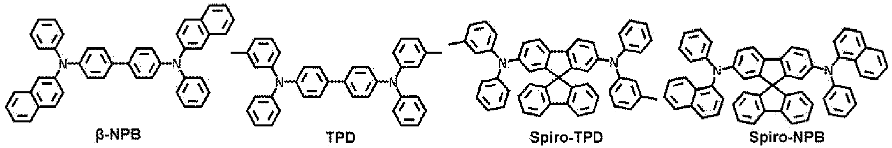

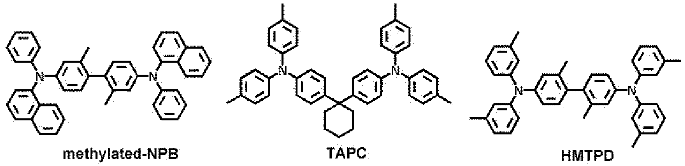

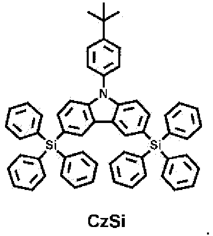

A thickness of the hole transport region may be in a range of about 50 Å to about 10,000 Å. For example, the thickness of the hole transport region may be in a range of about 100 Å to about 4,000 Å. When the hole transport region includes a hole injection layer, a hole transport layer, or any combination thereof, a thickness of the hole injection layer may be in a range of about 100 Å to about 9,000 Å, and a thickness of the hole transport layer may be about 50 Å to about 2,000 Å. For example, the thickness of the hole injection layer may be in a range of about 100 Å to about 1,000 Å. For example, the thickness of the hole transport layer may be in a range of about 100 Å to about 1,500 Å. When the thicknesses of the hole transport region, the hole injection layer, and the hole transport layer are within the ranges described above, satisfactory hole transporting characteristics may be obtained without a substantial increase in driving voltage.

The emission auxiliary layer may increase light-emission efficiency by compensating for an optical resonance distance according to a wavelength of light emitted by the emission layer, and the electron-blocking layer may block the leakage of electrons from the emission layer to the hole transport region. Materials that may be included in the hole transport region may be included in the emission auxiliary layer and the electron-blocking layer.

### [p-dopant]

The hole transport region may further include, in addition to the materials described above, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, a lowest unoccupied molecular orbital (LUMO) energy of the p-dopant may be less than or equal to about -3.5 eV.

In an embodiment, the p-dopant may include a quinone derivative, a cyano group-containing compound, a compound including element EL1 and element EL2, or any combination thereof.

Examples of a quinone derivative may include TCNQ and F4-TCNQ.

Examples of a cyano group-containing compound may include HAT-CN and a compound represented by Formula 221:

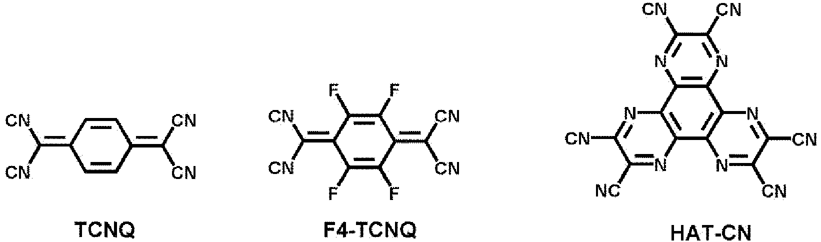

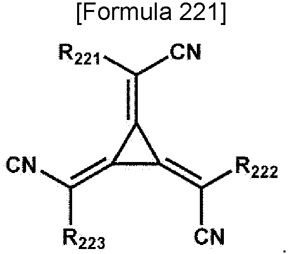

In Formula 221,
R₂₂₁ to R₂₂₃ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with: a cyano group; -F; -Cl; - Br; -I; a C₁-C₂₀ alkyl group substituted with a cyano group, -F, -Cl, -Br, -I, or any combination thereof; or any combination thereof.

In the compound including element EL1 and element EL2, element EL1 may be a metal, a metalloid, or any combination thereof, and element EL2 may be a non-metal, a metalloid, or any combination thereof.

Examples of a metal may include: an alkali metal (for example, lithium (Li), sodium (Na), potassium (K), rubidium (Rb), cesium (Cs), etc.); an alkaline earth metal (for example, beryllium (Be), magnesium (Mg), calcium (Ca), strontium (Sr), barium (Ba), etc.); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); a post-transition metal (for example, zinc (Zn), indium (In), tin (Sn), etc.); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of a metalloid may include silicon (Si), antimony (Sb), and tellurium (Te).

Examples of a non-metal may include oxygen (O) and a halogen (for example, F, Cl, Br, I, etc.).

Examples of a compound including element EL1 and element EL2 may include a metal oxide, a metal halide (for example, a metal fluoride, a metal chloride, a metal bromide, a metal iodide, etc.), a metalloid halide (for example, a metalloid fluoride, a metalloid chloride, a metalloid bromide, a metalloid iodide, etc.), a metal telluride, or any combination thereof.

Examples of a metal oxide may include a tungsten oxide (for example, WO, W₂O₃, WO₂, WO₃, W₂O₅, etc.), a vanadium oxide (for example, VO, V₂O₃, VO₂, V₂O₅, etc.), a molybdenum oxide (MoO, Mo₂O₃, MoO₂, MoO₃, Mo₂O₅, etc.), and a rhenium oxide (for example, ReO₃, etc.).

Examples of a metal halide may include an alkali metal halide, an alkaline earth metal halide, a transition metal halide, a post-transition metal halide, and a lanthanide metal halide.

Examples of an alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and Csl.

Examples of an alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, and BaI₂.

Examples of a transition metal halide may include a titanium halide (for example, TiF₄, TiCl₄, TiBr₄, TiI₄, etc.), a zirconium halide (for example, ZrF₄, ZrCl₄, ZrBr₄, ZrI₄, etc.), a hafnium halide (for example, HfF₄, HfCl₄, HfBr₄, HfI₄, etc.), a vanadium halide (for example, VF₃, VCl₃, VBr₃, VI₃, etc.), a niobium halide (for example, NbF₃, NbCl₃, NbBr₃, NbI₃, etc.), a tantalum halide (for example, TaF₃, TaCl₃, TaBr₃, TaI₃, etc.), a chromium halide (for example, CrF₃, CrCl₃, CrBr₃, CrI₃, etc.), a molybdenum halide (for example, MoF₃, MoCl₃, MoBr₃, MoI₃, etc.), a tungsten halide (for example, WF₃, WCl₃, WBr₃, WI₃, etc.), a manganese halide (for example, MnF₂, MnCl₂, MnBr₂, MnI₂, etc.), a technetium halide (for example, TcF₂, TcCl₂, TcBr₂, TcI₂, etc.), a rhenium halide (for example, ReF₂, ReCl₂, ReBr₂, ReI₂, etc.), an iron halide (for example, FeF₂, FeCl₂, FeBr₂, FeI₂, etc.), a ruthenium halide (for example, RuF₂, RuCl₂, RuBr₂, RuI₂, etc.), an osmium halide (for example, OsF₂, OsCl₂, OsBr₂, OsI₂, etc.), a cobalt halide (for example, CoF₂, CoCl₂, CoBr₂, CoI₂, etc.), a rhodium halide (for example, RhF₂, RhCl₂, RhBr₂, RhI₂, etc.), an iridium halide (for example, IrF₂, IrCl₂, IrBr₂, IrI₂, etc.), a nickel halide (for example, NiF₂, NiCl₂, NiBr₂, NiI₂, etc.), a palladium halide (for example, PdF₂, PdCl₂, PdBr₂, PdI₂, etc.), a platinum halide (for example, PtF₂, PtCl₂, PtBr₂, PtI₂, etc.), a copper halide (for example, CuF, CuCl, CuBr, Cul, etc.), a silver halide (for example, AgF, AgCl, AgBr, Agl, etc.), and a gold halide (for example, AuF, AuCl, AuBr, Aul, etc.).

Examples of a post-transition metal halide may include a zinc halide (for example, ZnF₂, ZnCl₂, ZnBr₂, ZnI₂, etc.), an indium halide (for example, InI₃, etc.), and a tin halide (for example, SnI₂, etc.).

Examples of a lanthanide metal halide may include YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, SmI₃, etc.

Examples of a metalloid halide may include an antimony halide (for example, SbCls, etc.).

Examples of a metal telluride may include an alkali metal telluride (for example, Li₂Te, Na₂Te, K₂Te, Rb₂Te, Cs₂Te, etc.), an alkaline earth metal telluride (for example, BeTe, MgTe, CaTe, SrTe, BaTe, etc.), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), a post-transition metal telluride (for example, ZnTe, etc.), and a lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### [Emission layer in interlayer 130]

When the light-emitting device 10 is a full-color light-emitting device, the emission layer may be patterned into a red emission layer, a green emission layer, and/or a blue emission layer, according to a subpixel. In an embodiment, the emission layer may have a stacked structure having two or more layers selected from a red emission layer, a green emission layer, and a blue emission layer, in which the two or more layers may contact each other or may be separated from each other, to emit white light. In embodiments, the emission layer may include two or more materials selected from a red light-emitting material, a green light-emitting material, and a blue light-emitting material, in which the two or more materials may be mixed with each other in a single layer, to emit white light. In an embodiment, the emission layer may emit blue light.

In an embodiment, the emission layer may include the heterocyclic compound represented by Formula 1 as described herein.

The emission layer may include a host and a dopant.

In an embodiment, the dopant may include the heterocyclic compound represented by Formula 1 as described herein. For example, the dopant may further include a phosphorescent dopant, a fluorescent dopant, or any combination thereof, in addition to the heterocyclic compound represented by Formula 1. In addition to the heterocyclic compound represented by Formula 1, the phosphorescent dopant, the fluorescent dopant, and the like that may be further included in the emission layer may each be the same as described below.

An amount of the dopant in the emission layer may be in a range of about 0.01 parts by weight to about 15 parts by weight, based on 100 parts by weight of the host.

In an embodiment, the emission layer may include quantum dots.

In an embodiment, the emission layer may include a delayed fluorescence material. The delayed fluorescence material may serve as a host or as a dopant in the emission layer.

A thickness of the emission layer may be in a range of about 100 Å to about 1,000 Å. For example, the thickness of the emission layer may be in a range of about 200 Å to about 600 Å. When the thickness of the emission layer is within any of the ranges described above, excellent luminescence characteristics may be obtained without a substantial increase in driving voltage.

### [Host]

In an embodiment, the host may be the heterocyclic compound represented by Formula 1.

In an embodiment, the host may include, for example, a carbazole-containing compound, an anthracene-containing compound, or any combination thereof.

In an embodiment, the host may include a compound represented by Formula 301:

[Formula 301] [Ar₃₀₁]_{xb11}-[(L₃₀₁)_{xb1}-R₃₀₁]_{xb21}

In Formula 301,
Ar₃₀₁ and L₃₀₁ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₆₀ carbocyclic group that is unsubstituted or substituted with at least one R₁₀ₐ, a C₁-C₆₀ heterocyclic group that is unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or - P(=O)(Q₃₀₁)(Q₃₀₂),
xb21 may be an integer from 1 to 5, and
Q₃₀₁ to Q₃₀₃ may each independently be the same as described in connection with Q₁.

In an embodiment, in Formula 301, when xb11 is 2 or more, two or more of Ar₃₀₁ may be linked to each other via a single bond.

In an embodiment, the host may include a compound represented by Formula 301-1, a compound represented by Formula 301-2, or any combination thereof:

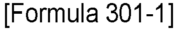

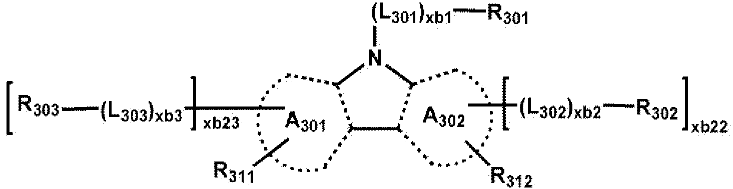

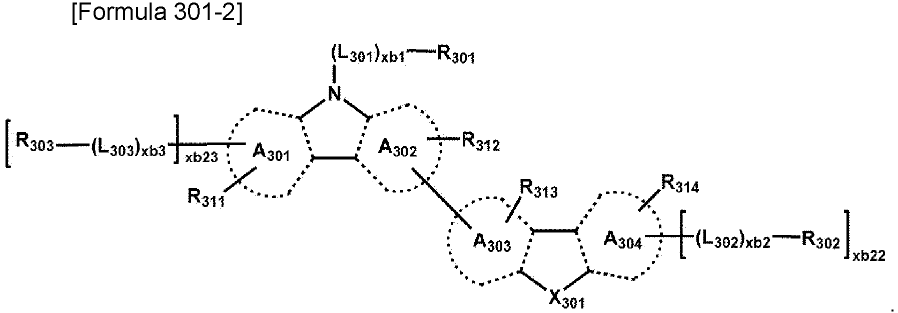

In Formula 301-1 and 301-2,
ring A₃₀₁ to ring A₃₀₄ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be the same as described in the specification,
L₃₀₂ to L₃₀₄ may each independently be the same as described in connection with L₃₀₁,
xb2 to xb4 may each independently be the same as described in connection with xb1, and
R₃₀₂ to R₃₀₅ and R₃₁₁ to R₃₁₄ may each independently be the same as described in connection with R₃₀₁.

In an embodiment, the host may include an alkali earth metal complex, a post-transition metal complex, or any combination thereof. In an embodiment, the host may include a Be complex (for example, Compound H55), an Mg complex, a Zn complex, or any combination thereof.

In an embodiment, the host may include one of Compounds H1 to H128, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-(carbazolyl)benzene (mCP), 1,3,5-tri(carbazol-9-yl)benzene (TCP), or any combination thereof:

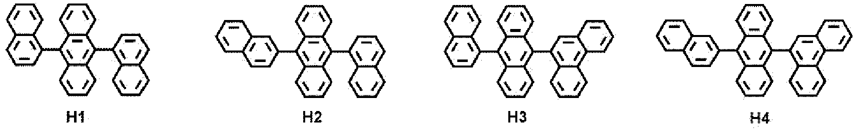

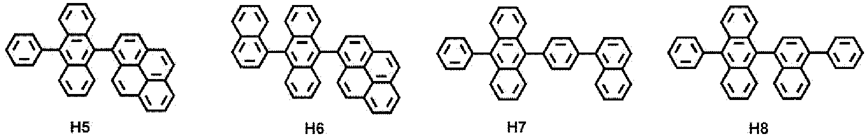

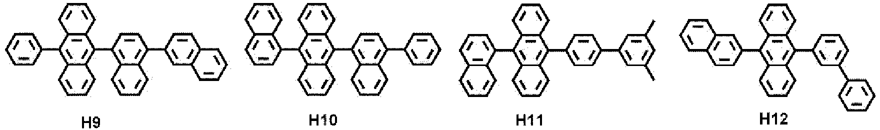

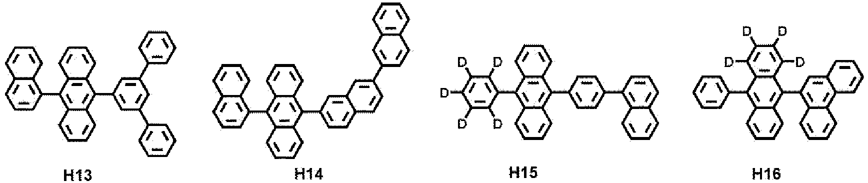

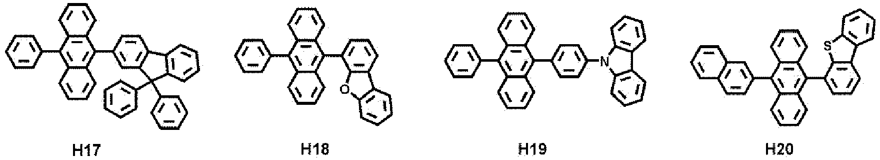

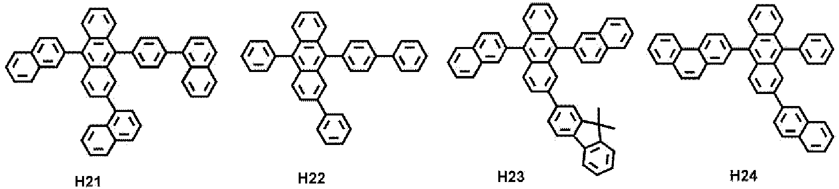

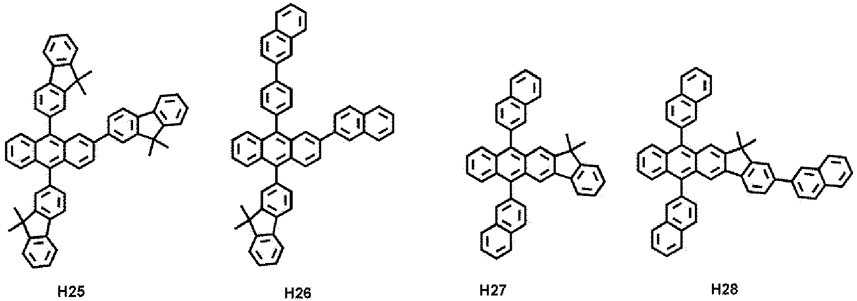

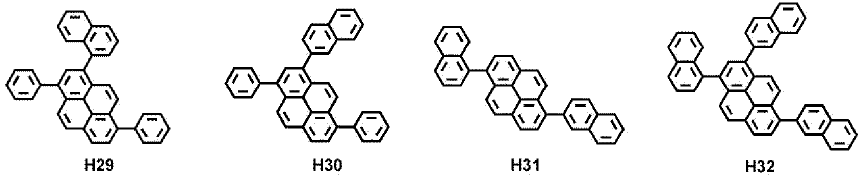

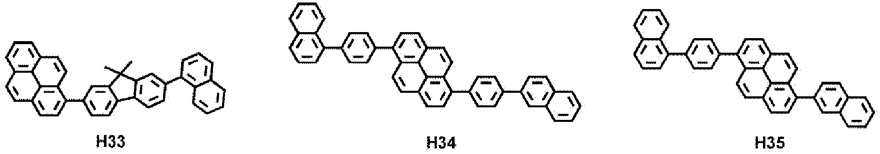

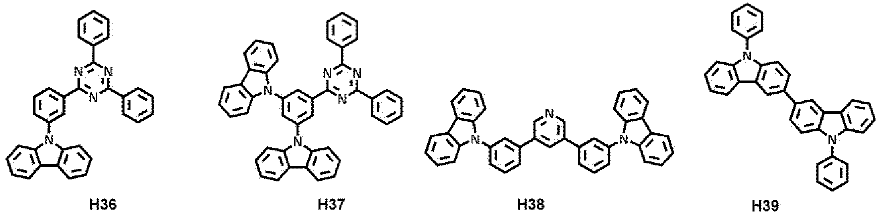

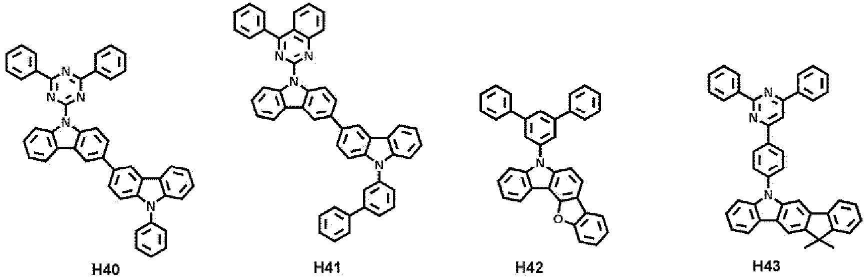

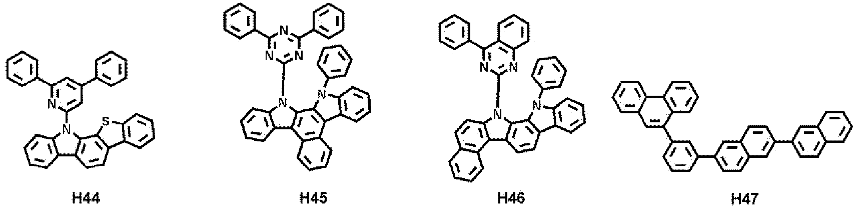

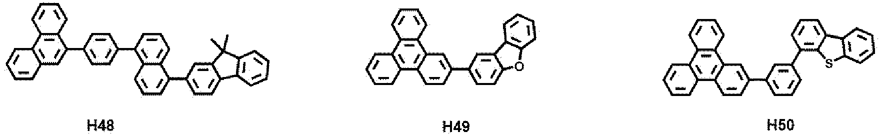

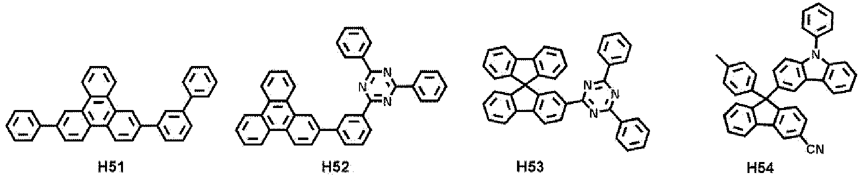

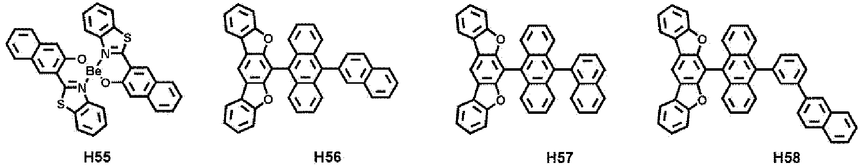

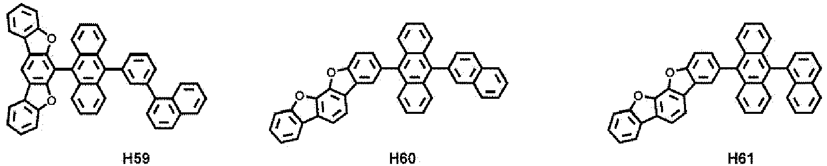

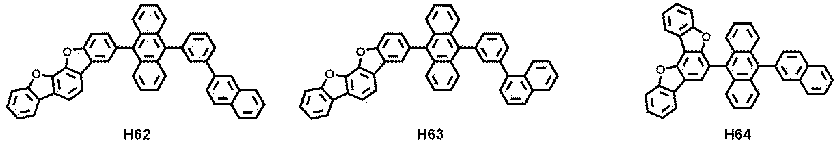

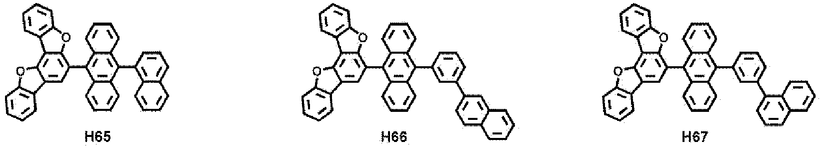

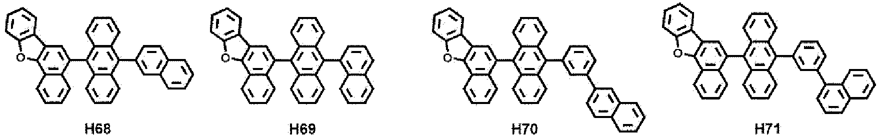

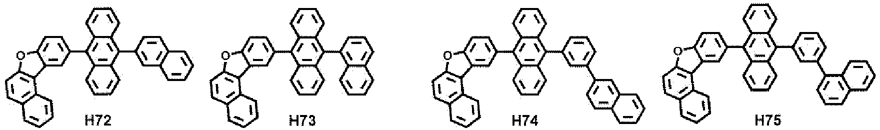

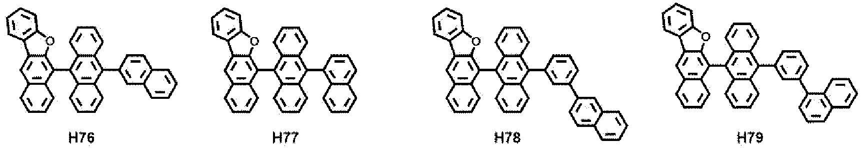

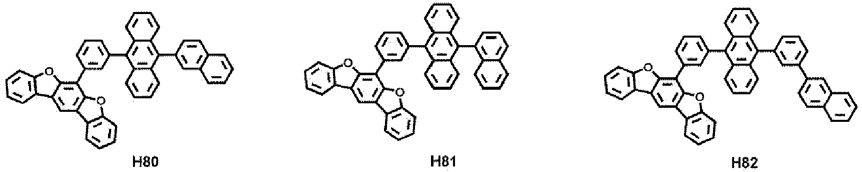

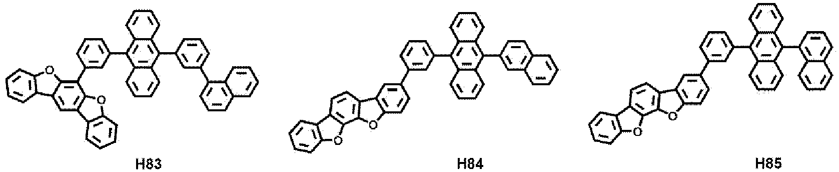

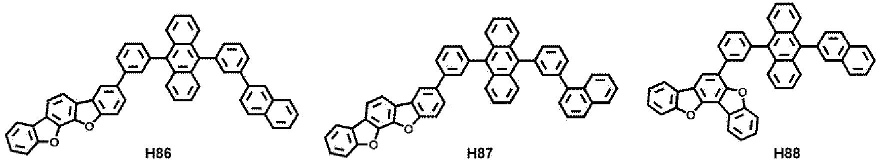

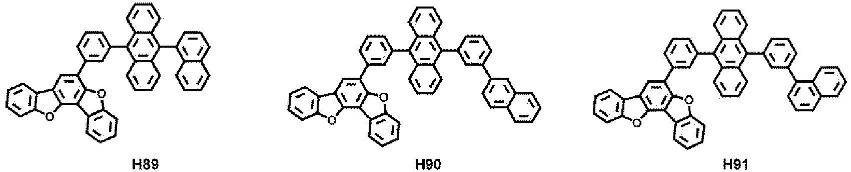

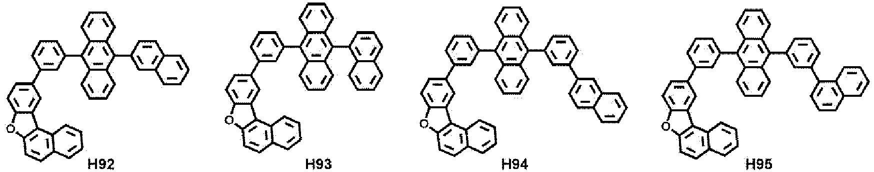

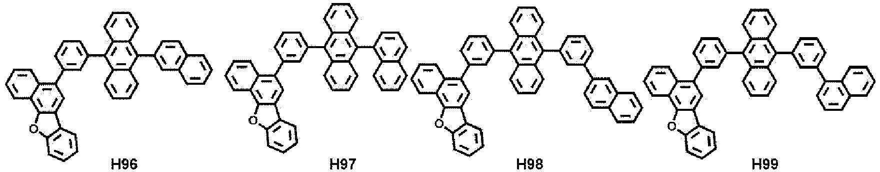

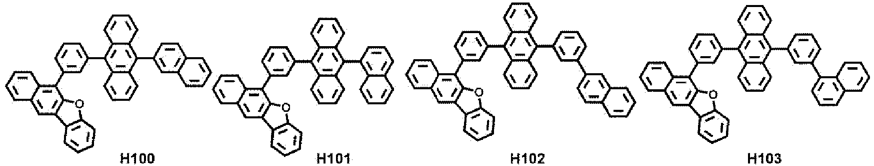

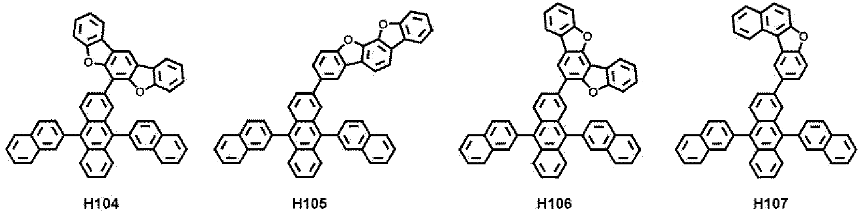

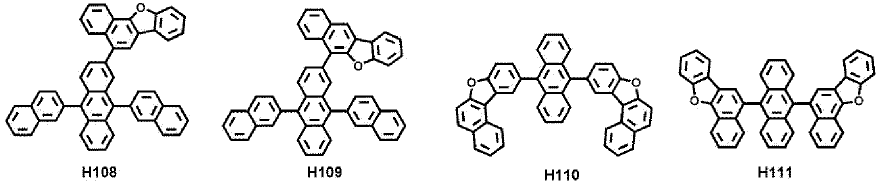

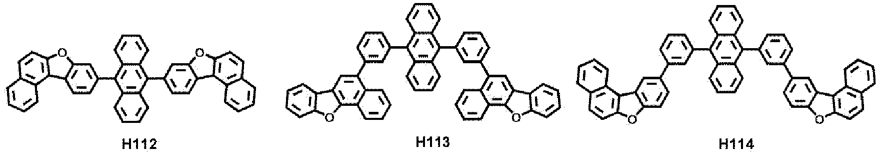

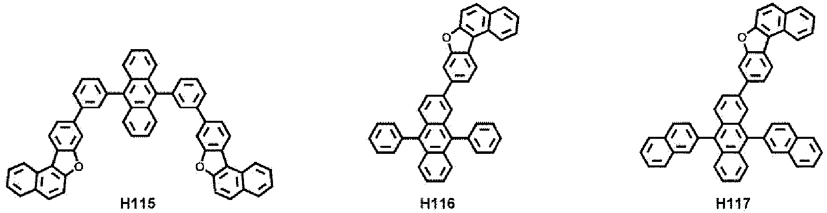

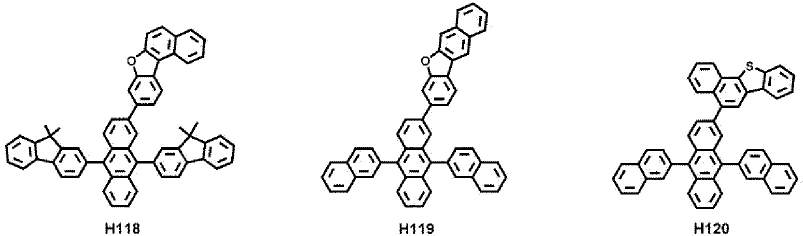

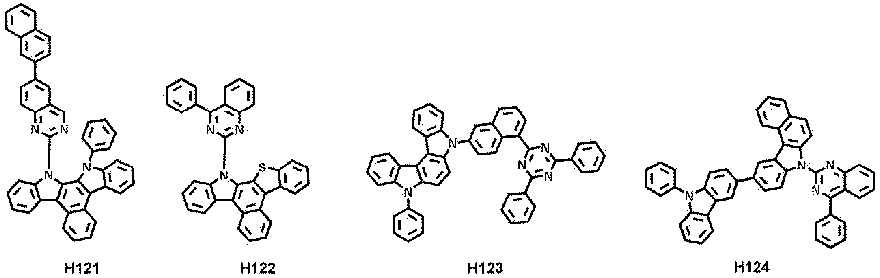

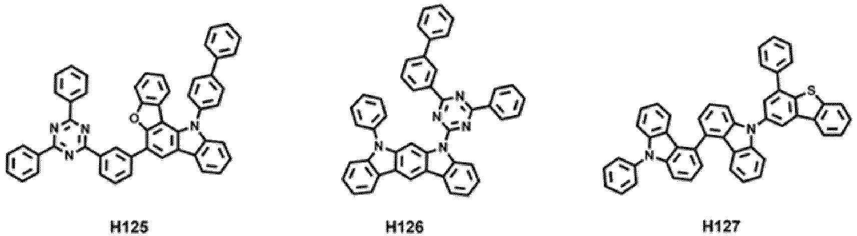

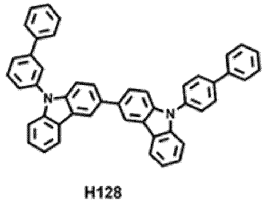

In an embodiment, the host may include a first host compound and a second host compound.

In an embodiment, the first host compound may be a hole-transporting host.

In an embodiment, the second host compound may be an electron-transporting host.

In an embodiment, the term "hole-transporting host" may refer to a compound that includes a hole-transporting moiety.

In an embodiment, the term "electron-transporting host" may refer to a compound that includes an electron-transporting moiety, or may refer to a compound having bipolar properties.

In the specification, the terms "hole-transporting host" and "electron-transporting host" may each be understood according to a relative difference between hole mobility and electron mobility in the hole-transporting host and in the electron-transporting host. For example, even when an electron-transporting host does not include an electron-transporting moiety, a bipolar compound exhibiting relatively higher electron mobility than a hole-transporting host may serve as an electron-transporting host.

In an embodiment, the hole-transporting host may be represented by any one of Formulae 311-1 to 311-6, and the electron-transporting host may be represented by any one of Formulae 312-1 to 312-4 and 313.

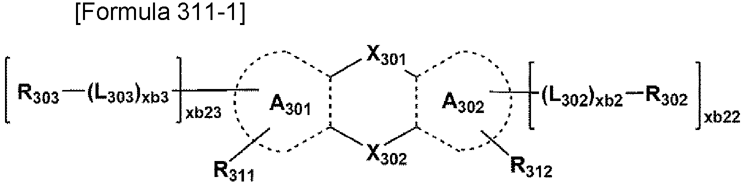

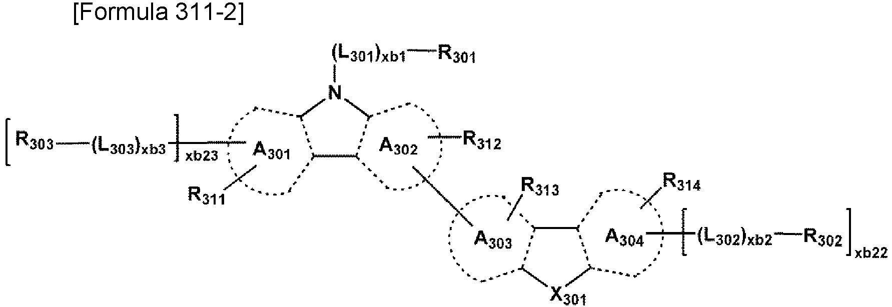

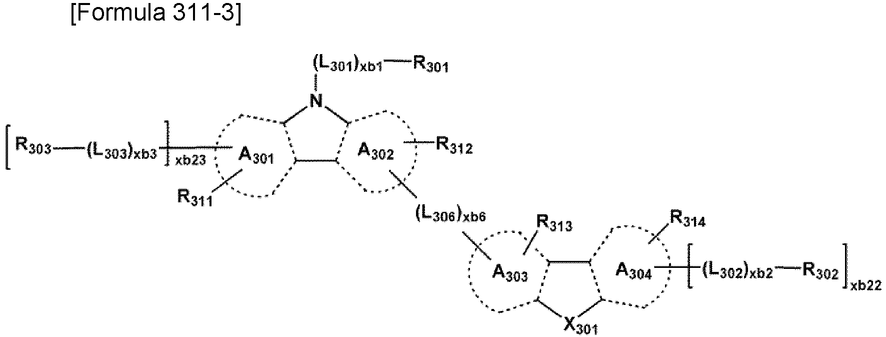

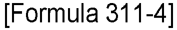

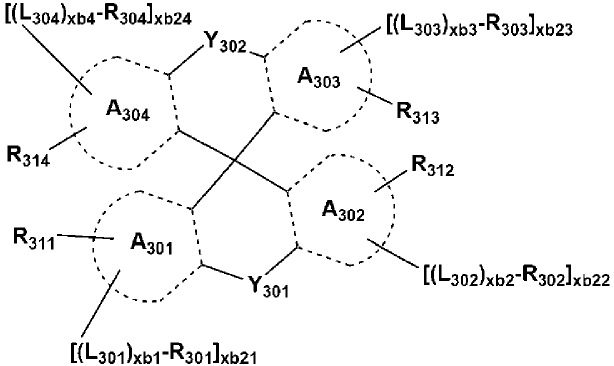

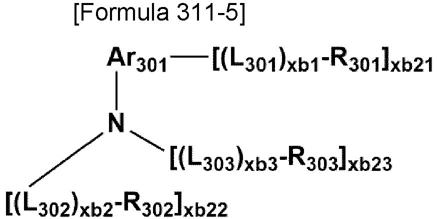

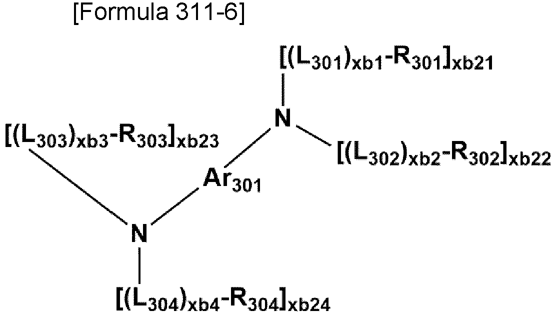

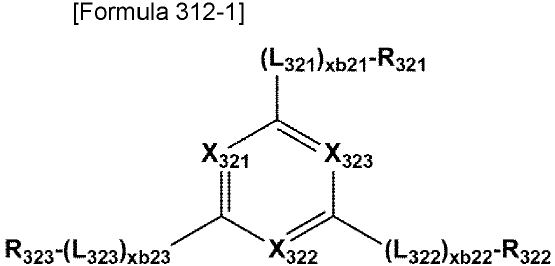

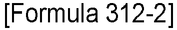

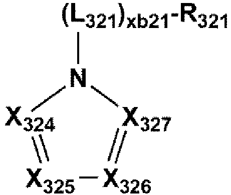

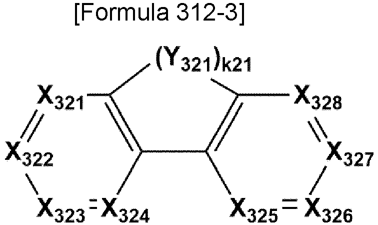

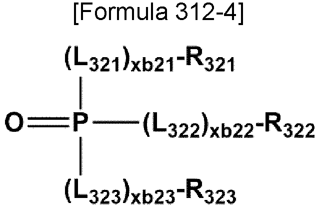

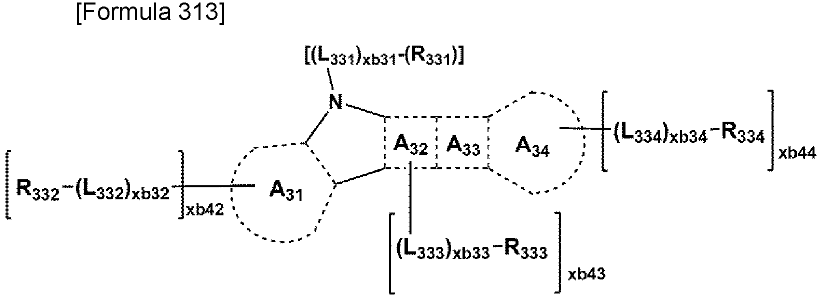

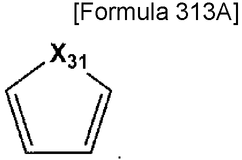

In Formulae 311-1 to 311-6, 312-1 to 312-4, 313, and 313A,
Ar₃₀₁ may be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
A₃₀₁ to A₃₀₄ may each independently be a C₃-C₆₀ carbocyclic group or a C₁-C₆₀ heterocyclic group,
X₃₀₁ may be O, S, N[(L₃₀₄)_{xb4}-R₃₀₄], C[(L₃₀₄)_{xb4}-R₃₀₄][(L₃₀₅)_{xb5}-R₃₀₅], or Si[(L₃₀₄)_{xb4}-R₃₀₄][(L₃₀₅)_{xb5}-R₃₀₅],
X₃₀₂, Y₃₀₁, and Y₃₀₂ may each independently be a single bond, O, S, N[(L₃₀₅)_{xb5}-R₃₀₅], C[(L₃₀₄)_{xb4}-R₃₀₄][(L₃₀₅)_{xb5}-R₃₀₅], Si[(L₃₀₄)_{xb4}-R₃₀₄][(L₃₀₅)_{xb5}-R₃₀₅], or S(=O)₂,
xb1 to xb5 may each independently be 0, 1, 2, 3, 4, or 5,
xb6 may be 1, 2, 3, 4, or 5,
X₃₂₁ to X₃₂₈ may each independently be N or C[(L₃₂₄)_{xb24}-R₃₂₄],
Y₃₂₁ may be *-O-*', *-S-*', *-N[(L₃₂₅)_{xb25}-R₃₂₅]-*', *-C[(L₃₂₅)_{xb25}-R₃₂₅][(L₃₂₆)_{xb26}-R₃₂₆]-*', *-C[(L₃₂₅)_{xb25}-R₃₂₅]=C[(L₃₂₆)_{xb26}-R₃₂₆]-*', *-C[(L₃₂₅)_{xb25}-R₃₂₅]=N-*', or *-N=C[(L₃₂₆)_{xb26}-R₃₂₆]-*',
k21 may be 0, 1, or 2,
when k21 is 0, Y₃₂₁ is not present,
xb21 to xb26 may each independently be 0, 1, 2, 3, 4, or 5,
A₃₁, A₃₂, and A₃₄ may each independently be a C₃-C₆₀ carbocyclic group or a C₁-C₃₀ heterocyclic group,
A₃₃ may be a group represented by Formula 313A,
X₃₁ may be N[(L₃₃₅)_{xb35}-(R₃₃₅)], O, S, Se, C[(L₃₃₅)_{xb35}-(R₃₃₅)][(L₃₃₆)_{xb36}-(R₃₃₆)], or Si[(L₃₃₅)_{xb35}-(R₃₃₅)][(L₃₃₆)_{xb36}-(R₃₃₆)],
xb31 to xb36 may each independently be 0, 1, 2, 3, 4, or 5,
xb42 to xb44 may each independently be 0, 1, 2, 3, 4, 5, 6, 7, 8, 9, or 10,
L₃₀₁ to L₃₀₆, L₃₂₁ to L₃₂₆, and L₃₃₁ to L₃₃₆ may each independently be a single bond, a C₁-C₂₀ alkylene group that is unsubstituted or substituted with at least one R₁₀ₐ, a C₁-C₂₀ alkenylene group that is unsubstituted or substituted with at least one R₁₀ₐ, a C₁-C₂₀ alkynylene group that is unsubstituted or substituted with at least one R₁₀ₐ, a C₃-C₁₀ cycloalkylene group that is unsubstituted or substituted with at least one R₁₀ₐ, a C₁-C₁₀ heterocycloalkylene group that is unsubstituted or substituted with at least one R₁₀ₐ, a C₃-C₁₀ cycloalkenylene group that is unsubstituted or substituted with at least one R₁₀ₐ, a C₁-C₁₀ heterocycloalkenylene group that is unsubstituted or substituted with at least one R₁₀ₐ, a C₆-C₆₀ arylene group that is unsubstituted or substituted with at least one R₁₀ₐ, a C₁-C₆₀ heteroarylene group that is unsubstituted or substituted with at least one R₁₀ₐ, a divalent non-aromatic condensed polycyclic group that is unsubstituted or substituted with at least one R₁₀ₐ, or a divalent non-aromatic condensed heteropolycyclic group that is unsubstituted or substituted with at least one R₁₀ₐ,
R₃₀₁ to R₃₀₅, R₃₁₁ to R₃₁₄, R₃₂₁ to R₃₂₆, and R₃₃₁ to R₃₃₆ may each independently be hydrogen, deuterium, -F, -Cl, -Br, -I, a hydroxyl group, a cyano group, a nitro group, an amidino group, a hydrazino group, a hydrazono group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₁₀ cycloalkyl group that is unsubstituted or substituted with at least one R₁₀ₐ, a C₁-C₁₀ heterocycloalkyl group that is unsubstituted or substituted with at least one R₁₀ₐ, a C₃-C₁₀ cycloalkenyl group that is unsubstituted or substituted with at least one R₁₀ₐ, a C₁-C₁₀ heterocycloalkenyl group that is unsubstituted or substituted with at least one R₁₀ₐ, a C₆-C₆₀ aryl group that is unsubstituted or substituted with at least one R₁₀ₐ, a C₆-C₆₀ aryloxy group that is unsubstituted or substituted with at least one R₁₀ₐ, a C₆-C₆₀ arylthio group that is unsubstituted or substituted with at least one R₁₀ₐ, a C₁-C₆₀ heteroaryl group that is unsubstituted or substituted with at least one R₁₀ₐ, a C₁-C₆₀ heteroaryloxy group that is unsubstituted or substituted with at least one R₁₀ₐ, a C₁-C₆₀ heteroarylthio group that is unsubstituted or substituted with at least one R₁₀ₐ, a monovalent non-aromatic condensed polycyclic group that is unsubstituted or substituted with at least one R₁₀ₐ, a monovalent non-aromatic condensed heteropolycyclic group that is unsubstituted or substituted with at least one R₁₀ₐ, -Si(Q₁)(Q₂)(Q₃), -B(Q₁)(Q₂), -N(Q₁)(Q₂), -P(Q₁)(Q₂), -C(=O)(Q₁), -S(=O)(Q₁), -S(=O)₂(Q₁), -P(=O)(Q₁)(Q₂), or -P(=S)(Q₁)(Q₂),
two or more neighboring groups among of R₃₂₁ to R₃₂₆ may optionally be bonded to each other to form a C₃-C₆₀ carbocyclic group that is unsubstituted or substituted with at least one R₁₀ₐ or a C₁-C₆₀ heterocyclic group that is unsubstituted or substituted with at least one R₁₀ₐ,
R₁₀ₐ may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, a C₁-C₆₀ heteroarylthio group, -Si(Q₁₁)(Q₁₂)(Q₁₃), -N(Q₁₁)(Q₁₂), - B(Q₁₁)(Q₁₂), -C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, or a C₁-C₆₀ heteroarylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, a C₁-C₆₀ heteroarylthio group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, the first host compound and the second host compound may form an exciplex.

### [Phosphorescent dopant]

The phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In an embodiment, the phosphorescent dopant may include an organometallic compound represented by Formula 401:

[Formula 401] M(L₄₀₁)_{xc1}(L₄₀₂)_{xc2}

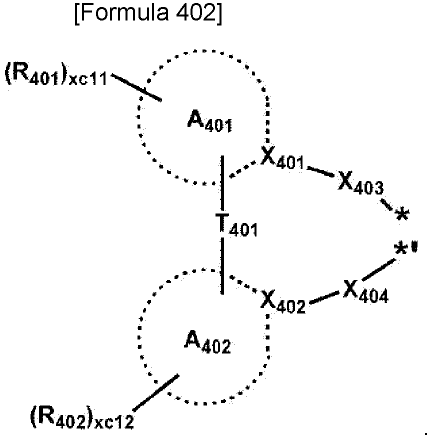

In Formulae 401 and 402,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 may be 1, 2, or 3, wherein when xc1 is 2 or more, two or more of L₄₀₁ may be identical to or different from each other,
L₄₀₂ may be an organic ligand, and xc2 may be 0, 1, 2, 3, or 4, wherein when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordinate bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each independently be the same as described in connection with Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group that is unsubstituted or substituted with at least one R₁₀ₐ, a C₁-C₂₀ alkoxy group that is unsubstituted or substituted with at least one R₁₀ₐ, a C₃-C₆₀ carbocyclic group that is unsubstituted or substituted with at least one R₁₀ₐ, a C₁-C₆₀ heterocyclic group that is unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), or -P(=O)(Q₄₀₁)(Q₄₀₂),
Q₄₀₁ to Q₄₀₃ may each independently be the same as described in connection with Q₁,
xc11 and xc12 may each independently be an integer from 0 to 10, and
* and *' in Formula 402 each indicate a binding site to M in Formula 401.

For example, in Formula 402, X₄₀₁ may be nitrogen and X₄₀₂ may be carbon, or X₄₀₁ and X₄₀₂ may each be nitrogen.

In an embodiment, in Formula 401, when xc1 is 2 or more, two ring A₄₀₁ among two or more of L₄₀₁ may be optionally linked together via T₄₀₂, which is a linking group, and two ring A₄₀₂ may be optionally linked together via T₄₀₃, which is a linking group (see Compounds PD1 to PD4 and PD7). T₄₀₂ and T₄₀₃ may each independently be the same as described in connection with T₄₀₁.

In Formula 401, L₄₀₂ may be an organic ligand. For example, L₄₀₂ may include a halogen group, a diketone group (for example, an acetylacetonate group), a carboxylic acid group (for example, a picolinate group), -C(=O), an isonitrile group, a - CN group, a phosphorus group (for example, a phosphine group, a phosphite group, etc.), or any combination thereof.

In an embodiment, the phosphorescent dopant may include, for example, one of Compounds PD1 to PD39, or any combination thereof:

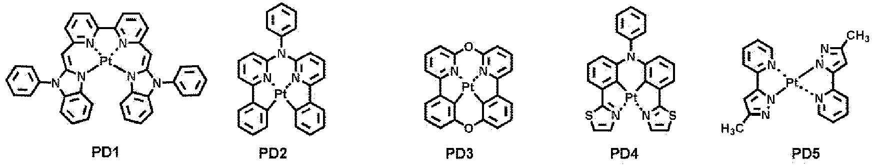

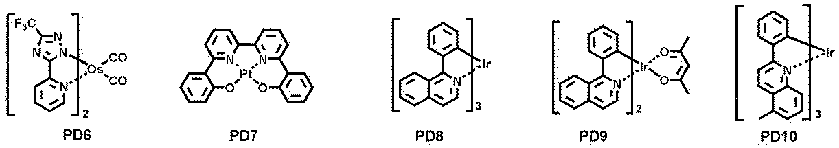

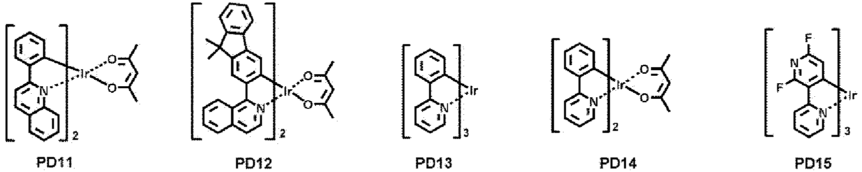

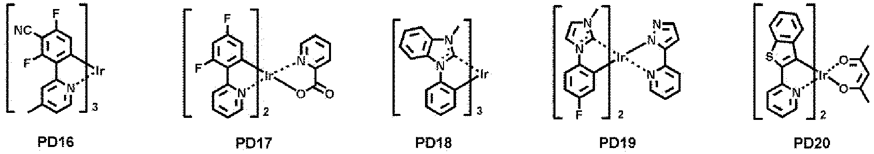

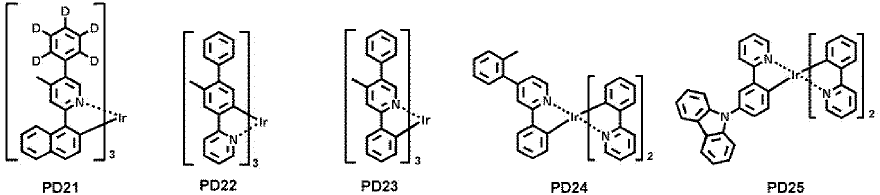

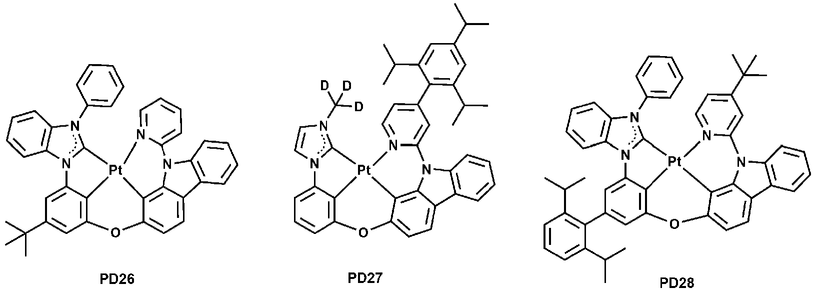

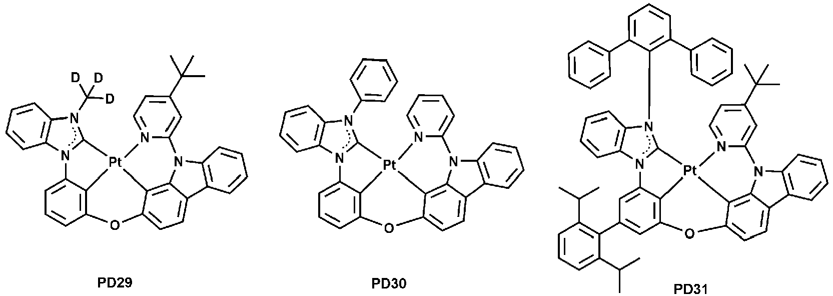

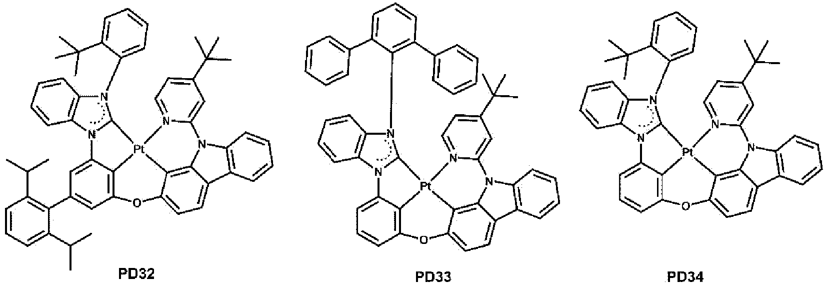

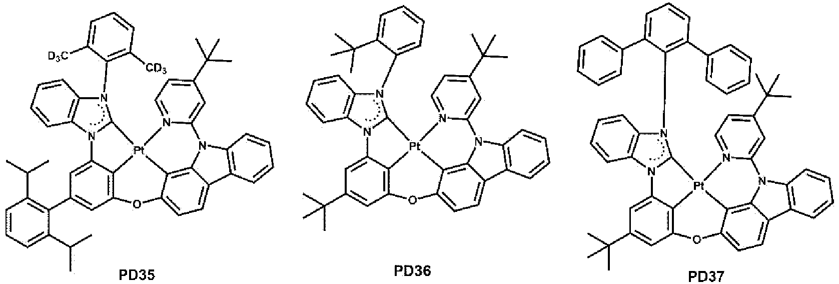

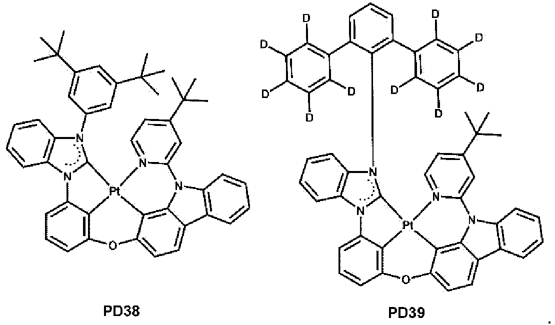

### [Fluorescent dopant]

The fluorescent dopant may include an amine group-containing compound, a styryl group-containing compound, or any combination thereof.

In an embodiment, the fluorescent dopant may include a compound represented by Formula 501:

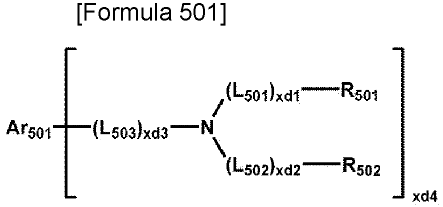

In Formula 501,
Ar₅₀₁, L₅₀₁ to L₅₀₃, R₅₀₁, and R₅₀₂ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
xd1 to xd3 may each independently be 0, 1, 2, or 3, and
xd4 may be 1, 2, 3, 4, 5, or 6.

In an embodiment, in Formula 501, Ar₅₀₁ may be a condensed cyclic group (for example, an anthracene group, a chrysene group, a pyrene group, etc.) in which three or more monocyclic groups are condensed together.

In an embodiment, in Formula 501, xd4 may be 2.

In an embodiment, the fluorescent dopant may include one of Compounds FD1 to FD36, DPVBi, DPAVBi, or any combination thereof:

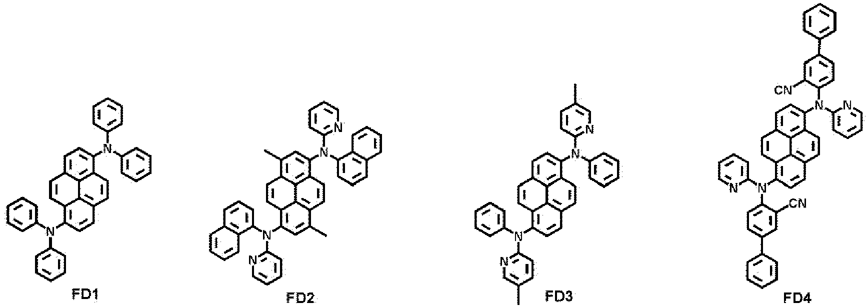

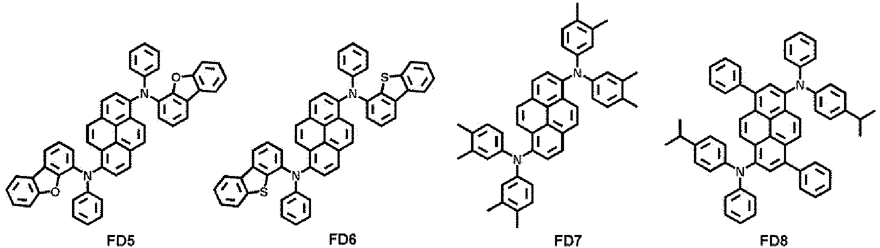

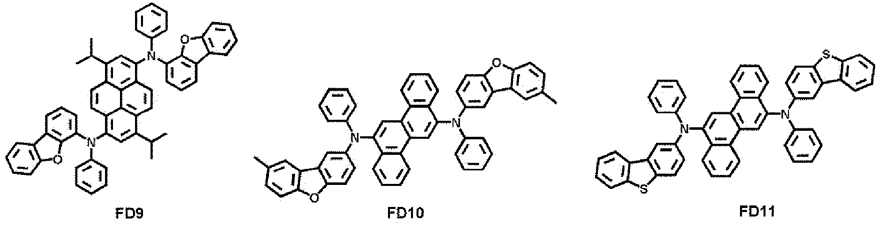

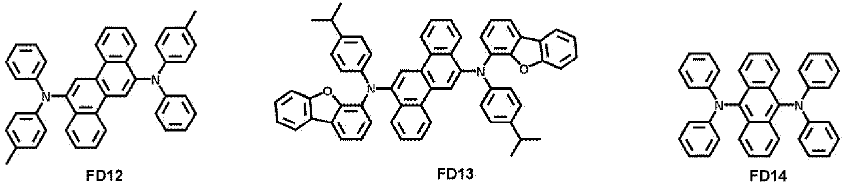

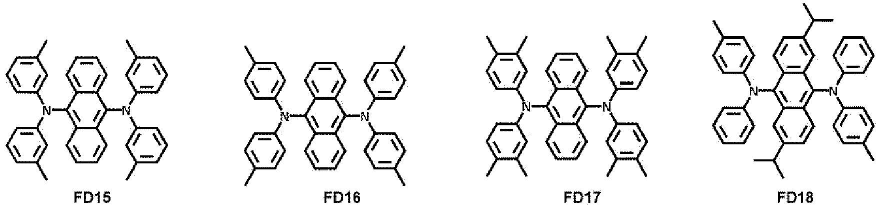

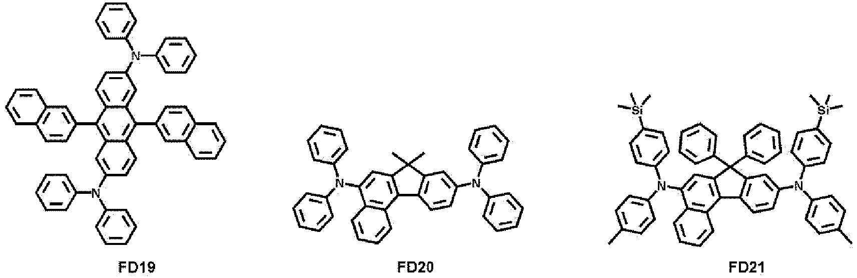

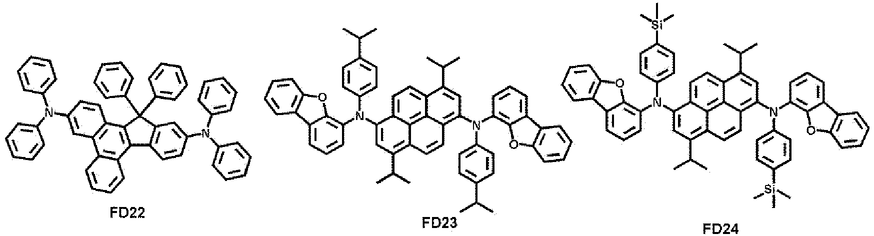

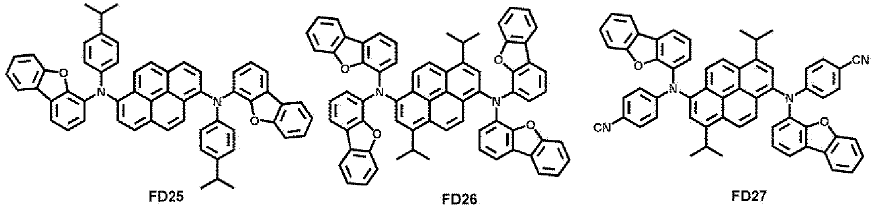

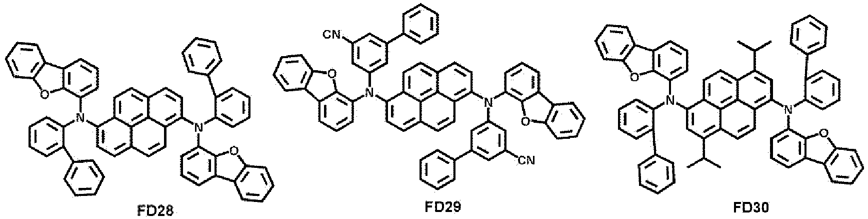

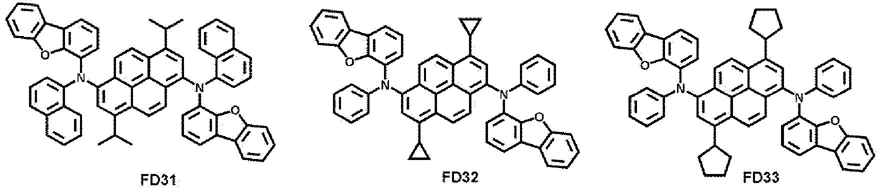

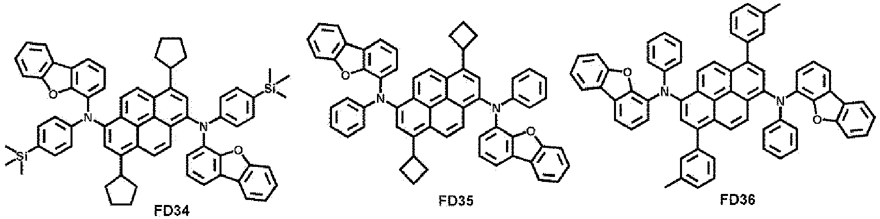

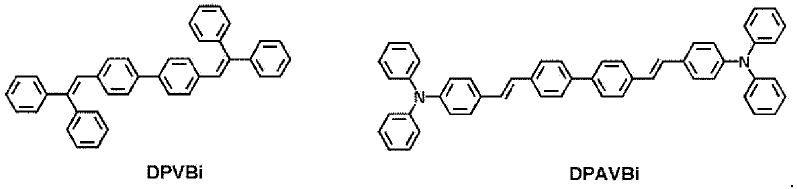

### [Delayed fluorescence material]

In an embodiment, the emission layer may further include a delayed fluorescence material.

In the specification, a delayed fluorescence material may be selected from compounds capable of emitting delayed fluorescence, based on a delayed fluorescence emission mechanism.

The delayed fluorescence material included in the emission layer may serve as a host or as a dopant, depending on the types of other materials included in the emission layer.

In an embodiment, a difference between a triplet energy level (eV) of the delayed fluorescence material and a singlet energy level (eV) of the delayed fluorescence material may be in a range of about 0 eV to about 0.5 eV. When a difference between a triplet energy level (eV) of the delayed fluorescence material and a singlet energy level (eV) of the delayed fluorescence material satisfies the range above, up-conversion from a triplet state to a singlet state of the delayed fluorescence materials may effectively occur, and thus, the light-emitting device 10 may have improved luminescence efficiency.

In an embodiment, the delayed fluorescence material may include: a material including at least one electron donor (for example, a π electron-rich C₃-C₆₀ cyclic group such as a carbazole group) and at least one electron acceptor (for example, a sulfoxide group, a cyano group, a π electron-deficient nitrogen-containing C₁-C₆₀ cyclic group, and the like); or a material including a C₈-C₆₀ polycyclic group including at least two cyclic groups that are condensed with each other while sharing boron (B).

In an embodiment, the delayed fluorescence material may include, for example, at least one of Compounds DF1 to DF9:

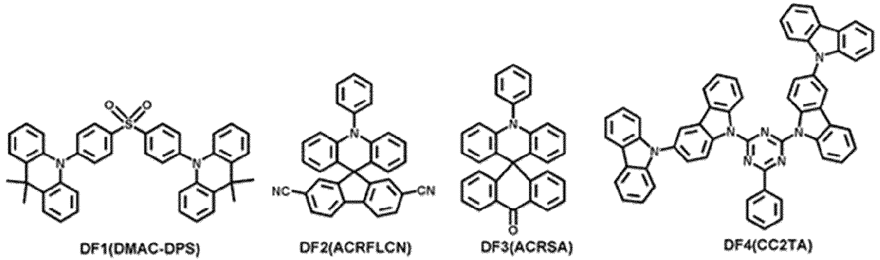

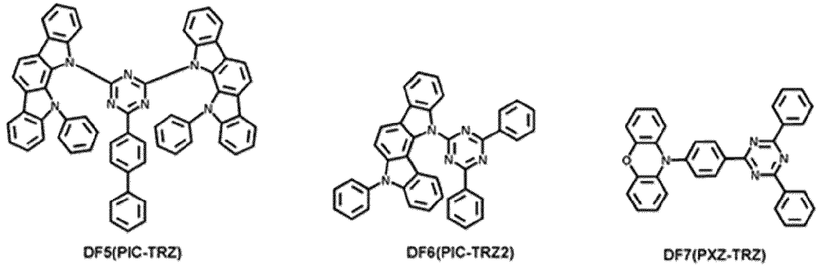

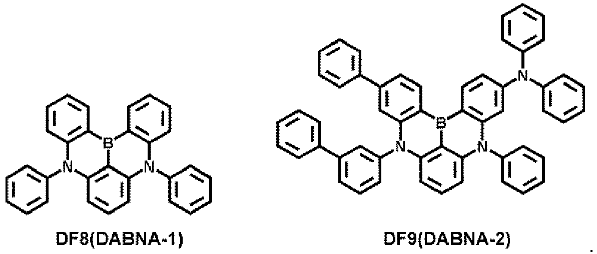

### [Quantum dot]

The emission layer may include quantum dots.

In the specification, a quantum dot may be a crystal of a semiconductor compound. Quantum dots may emit light of various emission wavelengths according to a size of the crystal. Quantum dots may emit light of various emission wavelengths by adjusting a ratio of elements in a quantum dot compound.

A diameter of the quantum dot may be, for example, in a range of about 1 nm to about 10 nm.

The quantum dot may be synthesized by a wet chemical process, a metal organic chemical vapor deposition process, a molecular beam epitaxy process, or any process similar thereto.

The wet chemical process is a method that includes mixing a precursor material with an organic solvent and growing quantum dot particle crystals. When the crystal grows, the organic solvent naturally serves as a dispersant coordinated on the surface of the quantum dot crystal and may control the growth of the crystal. Therefore, the wet chemical process may be more readily performed than vapor deposition methods, such as metal organic chemical vapor deposition (MOCVD) or molecular beam epitaxy (MBE), and the growth of quantum dot particles may be controlled through a process which costs less.

The quantum dot may include a Group III-VI semiconductor compound, a Group II-VI semiconductor compound, a Group III-V semiconductor compound, a Group I-III-VI semiconductor compound, a Group IV-VI semiconductor compound, a Group IV element or compound, or any combination thereof.

Examples of a Group II-VI semiconductor compound may include: a binary compound, such as CdS, CdSe, CdTe, ZnS, ZnSe, ZnTe, ZnO, HgS, HgSe, HgTe, MgSe, or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, or MgZnS; a quaternary compound, such as CdZnSeS, CdZnSeTe, CdZnSTe, CdHgSeS, CdHgSeTe, CdHgSTe, HgZnSeS, HgZnSeTe, or HgZnSTe; and any combination thereof.

Examples of a Group III-V semiconductor compound may include: a binary compound, such as GaN, GaP, GaAs, GaSb, AIN, AlP, AlAs, AlSb, InN, InP, InAs, or InSb; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, or InPSb; a quaternary compound, such as GaAlNP, GaAINAs, GaAINSb, GaAlPAs, GaAlPSb, GalnNP, GalnNAs, GaInNSb, GalnPAs, GalnPSb, InAlNP, InAlNAs, InAlNSb, InAlPAs, or InAlPSb; and any combination thereof. In an embodiment, the Group III-V semiconductor compound may further include a Group II element. Examples of a Group III-V semiconductor compound further including a Group II element may include InZnP, InGaZnP, and InAlZnP.

Examples of a Group III-VI semiconductor compound may include: a binary compound, such as GaS, Ga₂S₃, GaSe, Ga₂Se₃, GaTe, InS, InSe, In₂Se₃, or InTe; a ternary compound, such as InGaS₃ or InGaSe₃; and any combination thereof.

Examples of a Group I-III-VI semiconductor compound may include: a ternary compound, such as AgInS, AgInS₂, AgInSe₂, AgGaS, AgGaS₂, AgGaSe₂, CulnS, CuInS₂, CuInSe₂, CuGaS₂, CuGaSe₂, CuGaO₂, AgGaO₂, AgAlO₂, etc.; a quaternary compound, such as AgInGaS₂, AgInGaSe₂, etc.; and any combination thereof.

Examples of a Group IV-VI semiconductor compound may include: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, or SnPbTe; a quaternary compound, such as SnPbSSe, SnPbSeTe, or SnPbSTe; and any combination thereof.

Examples of a Group IV element or compound may include: a single element material, such as Si or Ge; a binary compound, such as SiC or SiGe; and any combination thereof.

Each element included in a compound, such as a binary compound, a ternary compound, or a quaternary compound, may be present in a particle at a uniform concentration or at non-uniform concentration. The formulae for quantum dot compounds as described above may each refer to the types of elements that are included in a compound, wherein an elemental ratio of a compound may vary. For example, AgInGaS₂ may indicate AgInₓGa₁₋ₓS₂ (wherein x is a real number between 0 and 1).

In an embodiment, a quantum dot may have a single structure in which the concentration of each element in the quantum dot is uniform, or the quantum dot may have a core-shell structure. In an embodiment, in the case that the quantum dot has a core-shell structure, a material included in the core and a material included in the shell may be different from each other.

The shell of the quantum dot may serve as a protective layer which prevents chemical denaturation of the core to maintain semiconductor characteristics, and/or may serve as a charging layer which imparts electrophoretic characteristics to the quantum dot. The shell may be a single layer or a multilayer. The interface between the core and the shell may have a concentration gradient in which the concentration of a material that is present in the shell decreases toward the core.

Examples of a shell of the quantum dot may include a metal oxide, a non-metal oxide, a semiconductor compound, and any combination thereof. Examples of a metal oxide or a non-metal oxide may include: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO; a ternary compound, such as MgAl₂O₄, CoFe₂O₄, NiFe₂O₄, or CoMn₂O₄; and any combination thereof.

Examples of a semiconductor compound may include, as described herein, a Group III-VI semiconductor compound, a Group II-VI semiconductor compound, a Group III-V semiconductor compound, a Group I-III-VI semiconductor compound, a Group IV-VI semiconductor compound, and any combination thereof. For example, the semiconductor compound may include CdS, CdSe, CdTe, ZnS, ZnSe, ZnTe, ZnSeS, ZnTeS, GaAs, GaP, GaS, GaSe, AgGaS, AgGaS₂, GaSb, HgS, HgSe, HgTe, InAs, InP, InGaP, InSb, AlAs, AlP, AlSb, and any combination thereof.

The quantum dot may have a full width at half maximum (FWHM) of an emission wavelength spectrum less than or equal to about 45 nm. For example, the quantum dot may have an FWHM of an emission wavelength spectrum less than or equal to about 40 nm. For example, the quantum dot may have an FWHM of an emission wavelength spectrum less than or equal to about 30 nm. When the FWHM of the quantum dot is within any of these ranges, the quantum dot may have improved color purity or improved color reproducibility. Light emitted through the quantum dot may be emitted in all directions, so that a wide viewing angle may be improved.

In an embodiment, the quantum dot may be in a spherical form, a pyramidal form, a multi-arm form, or a cubic form, or the quantum dot may be in the form of nanoparticles, nanotubes, nanowires, nanofibers, or nanoplate particles.

Since an energy band gap may be controlled by adjusting the size of the quantum dots or the ratio of elements in the quantum dot compound, light of various wavelengths may be obtained from a quantum dot-containing emission layer. Therefore, by using the aforementioned quantum dots (using quantum dots of different sizes or having different element ratios in the quantum dot compound), a light-emitting device emitting light of various wavelengths may be implemented. In an embodiment, control of the size of the quantum dots or the ratio of elements in the quantum dot compound may be selected to emit red light, green light, and/or blue light. In an embodiment, the quantum dots may be configured to emit white light by combining light of various colors.

### [Electron transport region in interlayer 130]

The electron transport region may have a structure consisting of a layer consisting of a single material, a structure consisting of a layer including different materials, or a structure including multiple layers including different materials.

The electron transport region may include a buffer layer, a hole-blocking layer, an electron control layer, an electron transport layer, an electron injection layer, or any combination thereof.

In an embodiment, the electron transport region may have an electron transport layer/electron injection layer structure, a hole-blocking layer/electron transport layer/electron injection layer structure, an electron control layer/electron transport layer/electron injection layer structure, or a buffer layer/electron transport layer/electron injection layer structure, wherein the layers of each structure may be stacked from the emission layer in its respective stated order, but the structure of the electron transport region is not limited thereto.

The electron transport region (for example, a buffer layer, a hole-blocking layer, an electron control layer, or an electron transport layer in the electron transport region) may include a metal-free compound including at least one π electron-deficient nitrogen-containing C₁-C₆₀ cyclic group.

In an embodiment, the electron transport region may include a compound represented by Formula 601.

[Formula 601] [Ar₆₀₁]ₓₑ₁₁-[(L₆₀₁)ₓₑ₁-R₆₀₁]ₓₑ₂₁

In Formula 601,
Ar₆₀₁ and L₆₀₁ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group that is unsubstituted or substituted with at least one R₁₀ₐ, a C₁-C₆₀ heterocyclic group that is unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or - P(=O)(Q₆₀₁)(Q₆₀₂),
Q₆₀₁ to Q₆₀₃ may each independently be the same as described in connection with Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group that is unsubstituted or substituted with at least one R₁₀ₐ.
In an embodiment, in Formula 601, when xe11 is 2 or more, two or more of Ar₆₀₁ may be linked together via a single bond.
In an embodiment, in Formula 601, Ar₆₀₁ may be a substituted or unsubstituted anthracene group.

In an embodiment, the electron transport region may include a compound represented by Formula 601-1:

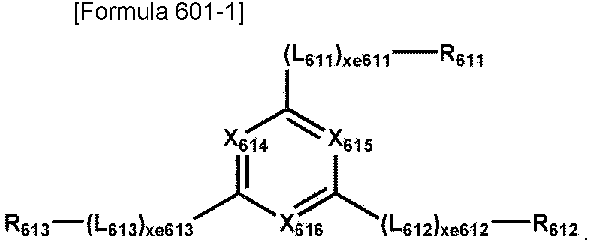

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may each be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group that is unsubstituted or substituted with at least one R₁₀ₐ, or a C₁-C₆₀ heterocyclic group that is unsubstituted or substituted with at least one R₁₀ₐ.

In an embodiment, in Formulae 601 and 601-1, xe1 and xe611 to xe613 may each independently be 0, 1, or 2.

In an embodiment, the electron transport region may include one of Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, TSPO1, TPBI, or any combination thereof:

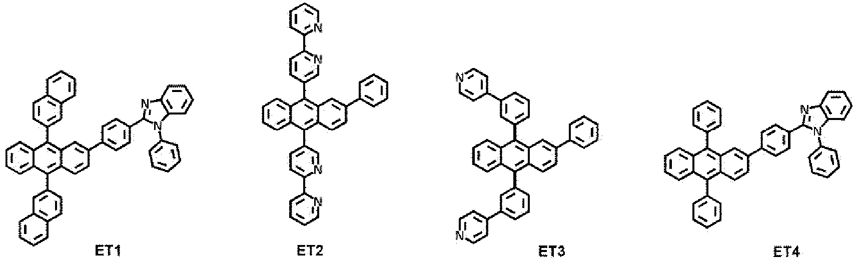

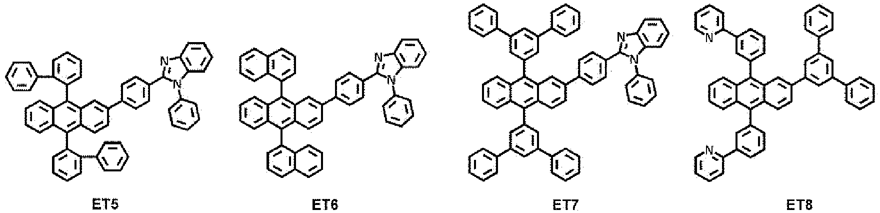

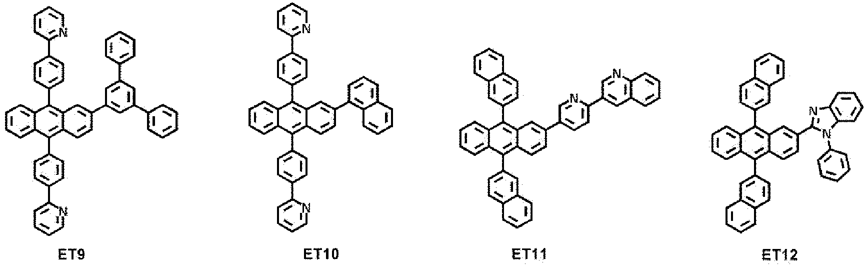

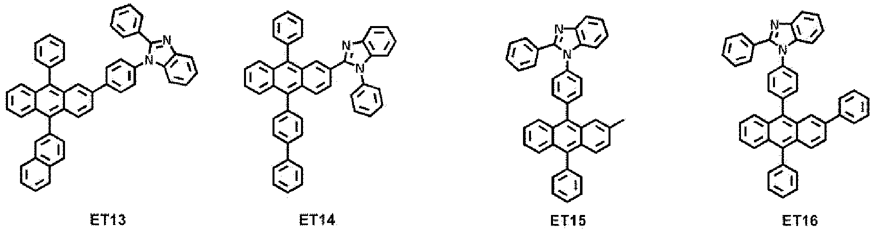

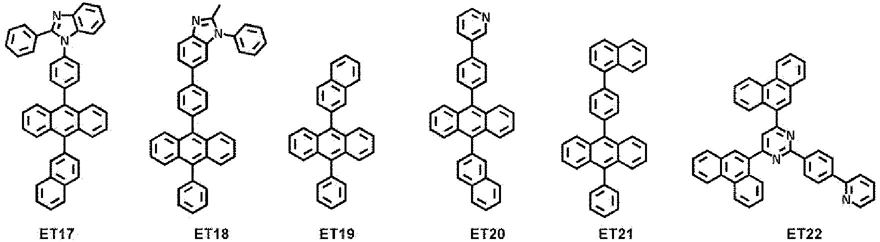

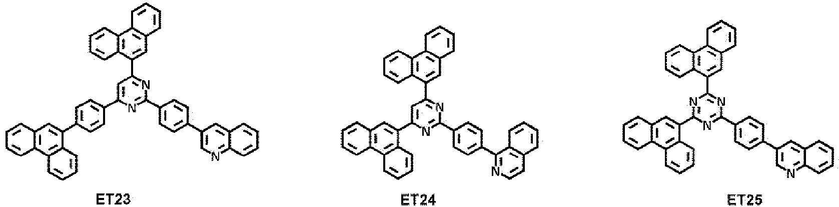

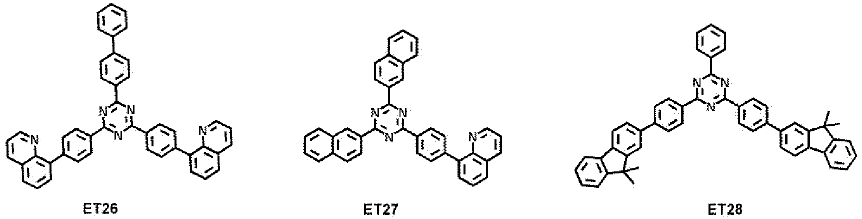

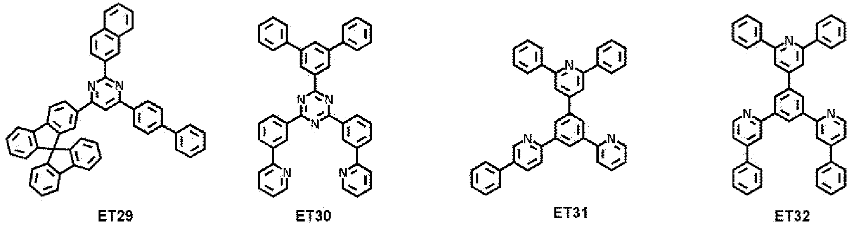

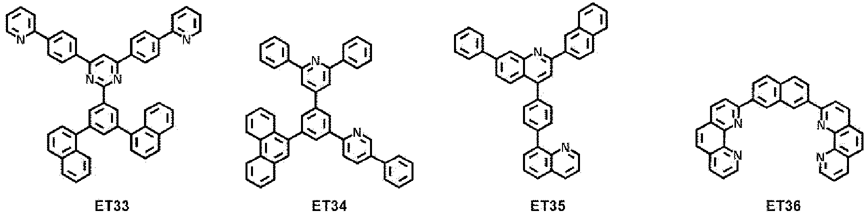

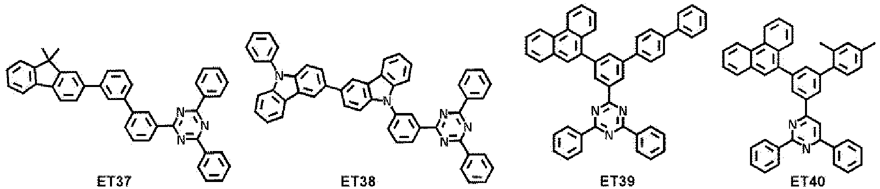

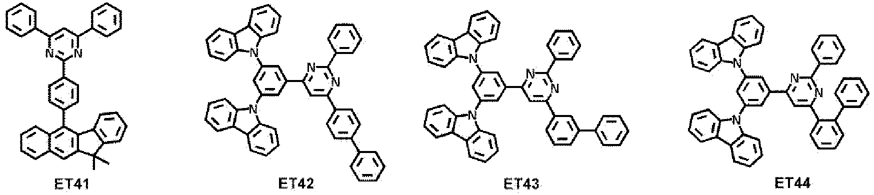

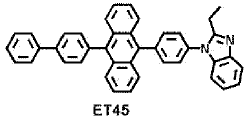

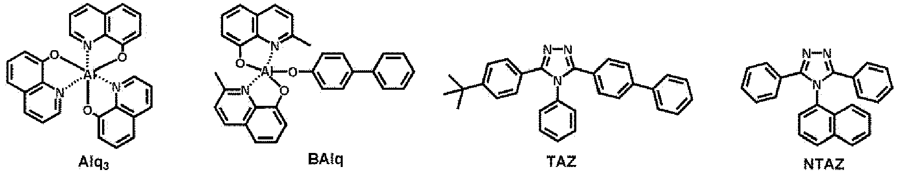

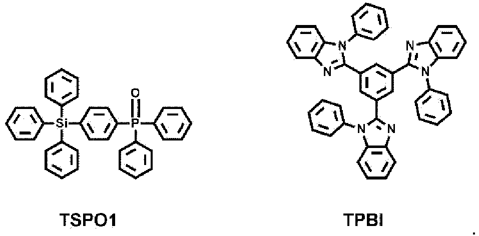

A thickness of the electron transport region may be in a range of about 100 Å to about 5,000 Å. For example, the thickness of the electron transport region may be in a range of about 160 Å to about 4,000 Å. When the electron transport region includes a buffer layer, a hole-blocking layer, an electron control layer, an electron transport layer, or any combination thereof, a thickness of the buffer layer, the hole-blocking layer, or the electron control layer may each independently be in a range of about 20 Å to about 1,000 Å, and a thickness of the electron transport layer may be about 100 Å to about 1,000 Å. For example, the thickness of the buffer layer, the hole-blocking layer, or the electron control layer may each independently be in a range of about 30 Å to about 300 Å. For example, the thickness of the electron transport layer may be in a range of about 150 Å to about 500 Å. When the thickness of the buffer layer, the hole-blocking layer, the electron control layer, the electron transport layer and/or the electron transport region are within these ranges, satisfactory electron transporting characteristics may be obtained without a substantial increase in driving voltage.

The electron transport region (for example, an electron transport layer in the electron transport region) may further include, in addition to the materials described above, a metal-containing material.

The metal-containing material may include an alkali metal complex, an alkaline earth metal complex, or any combination thereof. A metal ion of the alkali metal complex may be a Li ion, a Na ion, a K ion, a Rb ion, or a Cs ion, and a metal ion of the alkaline earth metal complex may be a Be ion, a Mg ion, a Ca ion, a Sr ion, or a Ba ion. A ligand coordinated with the metal ion of an alkali metal complex or an alkaline earth-metal complex may each independently include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

In an embodiment, the metal-containing material may include a Li complex. The Li complex may include, for example, Compound ET-D1 (LiQ) or Compound ET-D2:

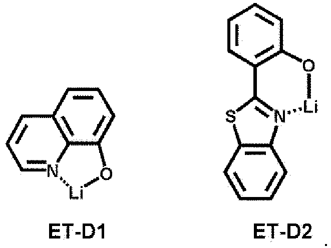

The electron transport region may include an electron injection layer that facilitates the injection of electrons from the second electrode 150. The electron injection layer may contact (e.g., directly contact) the second electrode 150.

The electron injection layer may have a structure consisting of a layer consisting of a single material, a structure consisting of a layer including different materials, or a structure including multiple layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may include oxides, halides (for example, fluorides, chlorides, bromides, iodides, etc.), or tellurides of the alkali metal, the alkaline earth metal, and the rare earth metal, or any combination thereof.

The alkali metal-containing compound may include: an alkali metal oxide, such as Li₂O, Cs₂O, or K₂O; an alkali metal halide, such as LiF, NaF, CsF, KF, Lil, Nal, Csl, or Kl; or any combination thereof. The alkaline earth metal-containing compound may include an alkaline earth metal oxide, such as BaO, SrO, CaO, BaₓSr₁₋ₓO (wherein x is a real number satisfying 0<x<1), or BaₓCa₁₋ₓO (wherein x is a real number satisfying 0<x<1). The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In an embodiment, the rare earth metal-containing compound may include a lanthanide metal telluride. Examples of a lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include: an alkali metal ion, an alkaline earth metal ion, or a rare earth metal ion; and a ligand bonded to the metal ion (for example, a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenyl benzimidazole, a hydroxyphenylbenzothiazole, a bipyridine, a phenanthroline, a cyclopentadiene, or any combination thereof.

In an embodiment, the electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In an embodiment, the electron injection layer may further include an organic material (for example, a compound represented by Formula 601).

In an embodiment, the electron injection layer may consist of an alkali metal-containing compound (for example, an alkali metal halide); or the electron injection layer may consist of an alkali metal-containing compound (for example, alkali metal halide), and an alkali metal, an alkaline earth metal, a rare earth metal, or any combination thereof. In an embodiment, the electron injection layer may be a KI:Yb co-deposited layer, an Rbl:Yb co-deposited layer, or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å. For example, the thickness of the electron injection layer may be in a range of about 3 Å to about 90 Å. When the thickness of the electron injection layer is within any of these ranges as described above, satisfactory electron injection characteristics may be obtained without a substantial increase in driving voltage.

### [Second electrode 150]

The second electrode 150 may be arranged on the interlayer 130. The second electrode 150 may be a cathode, which is an electron injection electrode. When the second electrode 150 is a cathode, a material for forming the second electrode 150 may include a material having a low-work function, such as a metal, an alloy, an electrically conductive compound, or any combination thereof.

The second electrode 150 may include lithium (Li), silver (Ag), magnesium (Mg), aluminium (Al), aluminium-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a transflective electrode, or a reflective electrode.

The second electrode 150 may have a single-layered structure or a multilayered structure.

### [Capping layer]

The light-emitting device 10 may include a first capping layer outside the first electrode 110, and/or the second capping layer outside the second electrode 150. In an embodiment, the light-emitting device 10 may have a structure in which the first capping layer, the first electrode 110, the interlayer 130, and the second electrode 150 are stacked in this stated order, a structure in which the first electrode 110, the interlayer 130, the second electrode 150, and the second capping layer are stacked in this stated order, or a structure in which the first capping layer, the first electrode 110, the interlayer 130, the second electrode 150, and the second capping layer are stacked in this stated order.

Light generated in the emission layer of the interlayer 130 of the light-emitting device 10 may pass through the first electrode 110 which may be a transflective electrode or a transmissive electrode, and through the first capping layer to the outside. Light generated in the emission layer of the interlayer 130 of the light-emitting device 10 may pass through the second electrode 150 which may be a transflective electrode or a transmissive electrode, and through the second capping layer to the outside.

The first capping layer and the second capping layer may increase external emission efficiency according to the principle of constructive interference. Accordingly, the light extraction efficiency of the light-emitting device 10 is increased, such that the luminescence efficiency of the light-emitting device 10 may be increased.

The first capping layer and the second capping layer may each include a material having a refractive index greater than or equal to about (with respect to a wavelength of about 589 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent including O, N, S, Se, Si, F, Cl, Br, I, or any combination thereof. In an embodiment, at least one of the first capping layer and the second capping layer may each independently include an amine group-containing compound.

In an embodiment, at least one of the first capping layer and the second capping layer may each independently include a compound represented by Formula 201, a compound represented by Formula 202, or any combination thereof.

In an embodiment, at least one of the first capping layer and the second capping layer may each independently include one of Compounds HT28 to HT33, one of Compounds CP1 to CP6, β-NPB, or any combination thereof:

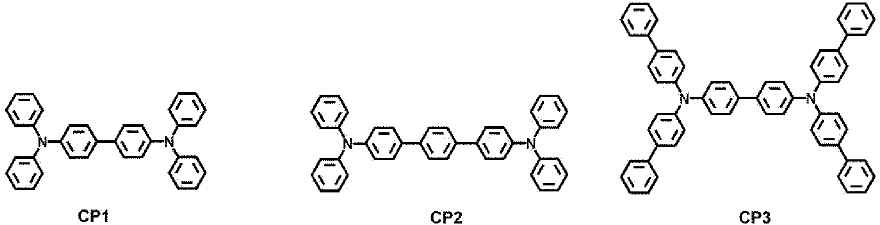

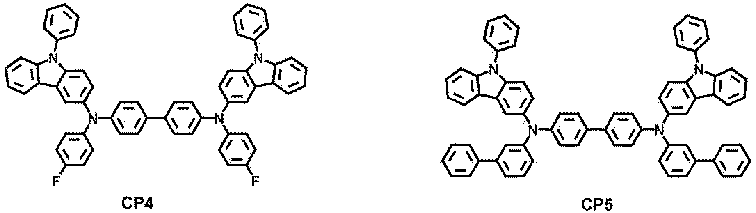

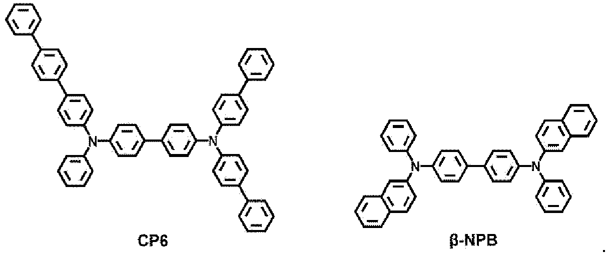

### [Film]

The heterocyclic compound represented by Formula 1 may be included in various films.

Thus, according to an embodiment, a film may include the heterocyclic compound represented by Formula 1. The film may be, for example, an optical member (or a light control means) (for example, a color filter, a color conversion member, a capping layer, a light extraction efficiency enhancement layer, a selective light absorbing layer, a polarizing layer, a quantum dot-containing layer, or like), a light-blocking member (for example, a light reflective layer, a light absorbing layer, or the like), or a protective member (for example, an insulating layer, a dielectric layer, or the like).

### [Electronic apparatus]

The light-emitting device may be included in various electronic apparatuses. For example, an electronic apparatus including the light-emitting device may be a light-emitting apparatus, an authentication apparatus, or the like.

The electronic apparatus (for example, a light-emitting apparatus) may further include, in addition to the light-emitting device, a color filter, a color conversion layer, or a color filter and a color conversion layer. The color filter and/or the color conversion layer may be arranged in at least one traveling direction of light emitted from the light-emitting device. For example, the light emitted from the light-emitting device may be blue light or white light. The light-emitting device may be the same as described above. In an embodiment, the color conversion layer may include quantum dots. The quantum dots may be, for example, the quantum dots as described herein.

The electronic apparatus may include a substrate. The substrate may include subpixels, the color filter may include a color filter regions respectively corresponding to the subpixels, and the color conversion layer may include color conversion regions respectively corresponding to the subpixels.

A pixel-defining film may be arranged between the subpixels to define each subpixel.

The color filter may further include color filter regions and light-shielding patterns arranged between the color filter regions, and the color conversion layer may further include color conversion regions and light-shielding patterns arranged between the color conversion regions.

The color filter regions (or the color conversion regions) may include a first region emitting first color light, a second region emitting second color light, and/or a third region emitting third color light, wherein the first color light, the second color light, and/or the third color light may have different maximum emission wavelengths from one another. In an embodiment, the first color light may be red light, the second color light may be green light, and the third color light may be blue light. In an embodiment, the color filter regions (or the color conversion regions) may include quantum dots. In an embodiment, the first region may include red quantum dots, the second region may include green quantum dots, and the third region may not include quantum dots. The quantum dots may be the same as described herein. The first region, the second region, and/or the third region may each further include a scatterer.

In an embodiment, the light-emitting device may emit first light, the first region may absorb the first light to emit first-first color light, the second region may absorb the first light to emit second-first color light, and the third region may absorb the first light to emit third-first color light. In an embodiment, the first-first color light, the second-first color light, and the third-first color light may have different maximum emission wavelengths. For example, the first light may be blue light, the first-first color light may be red light, the second-first color light may be green light, and the third-first color light may be blue light.

The electronic apparatus may further include a thin-film transistor, in addition to the light-emitting device as described above. The thin-film transistor may include a source electrode, a drain electrode, and an active layer, wherein any one of the source electrode and the drain electrode may be electrically connected to any one of the first electrode and the second electrode of the light-emitting device.

The thin-film transistor may further include a gate electrode, a gate insulating film, and the like.

The active layer may include crystalline silicon, amorphous silicon, an organic semiconductor, an oxide semiconductor, and the like.

The electronic apparatus may further include a sealing portion for sealing the light-emitting device. The sealing portion may be arranged between the color filter and/or the color conversion layer and the light-emitting device. The sealing portion may allow light from the light-emitting device to be extracted to the outside, and may prevent ambient air and moisture from penetrating into the light-emitting device. The sealing portion may be a sealing substrate including a transparent glass substrate or a plastic substrate. The sealing portion may be a thin-film encapsulation layer including at least one layer of an organic layer and/or an inorganic layer. When the sealing portion is a thin-film encapsulation layer, the electronic apparatus may be flexible.

Various functional layers may be further included on the sealing portion, in addition to the color filter and/or the color conversion layer, according to the use of the electronic apparatus. Examples of the functional layers may include a touch screen layer and a polarizing layer. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, etc.).

The authentication apparatus may further include, in addition to the light-emitting device as described above, a biometric information collector.

The electronic apparatus may be applied to various displays, light sources, lights, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

### [Electronic equipment]

The light-emitting device may be included in various electronic equipment.

In embodiments, an electronic equipment that includes the light-emitting device may be a flat panel display, a curved display, a computer monitor, a medical monitor, a television, an advertisement board, an indoor light, an outdoor light, a signal light, a head-up display, a fully transparent display, partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a microdisplay, a three-dimensional (3D) display, a virtual reality display, an augmented reality display, a vehicle, a video wall with multiple displays tiled together, a theater screen, a stadium screen, a phototherapy device, or a signboard.

Since the light-emitting device has excellent effects in terms of luminescence efficiency long lifespan, the electronic equipment including the light-emitting device may have characteristics including high luminance, high resolution, and low power consumption.

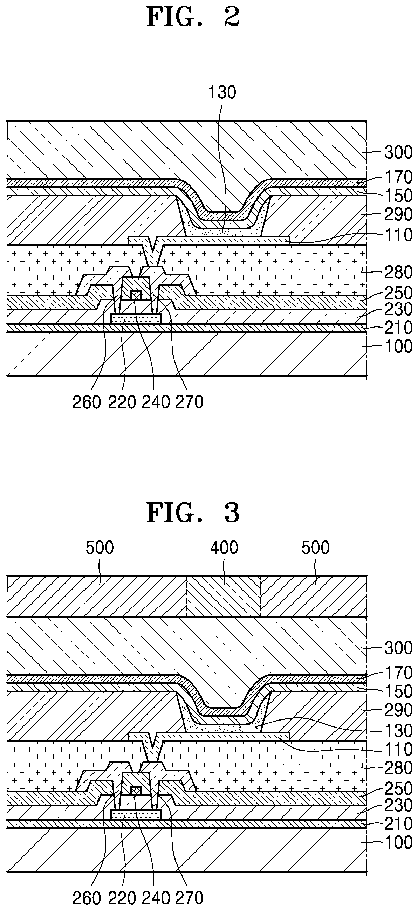

### [Description of FIGS. 2 and 3]

FIG. 2 is a schematic cross-sectional view of an electronic apparatus according to an embodiment.

The electronic apparatus of FIG. 2 includes a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be disposed on the substrate 100. The buffer layer 210 may prevent penetration of impurities through the substrate 100 and may provide a flat surface on the substrate 100.

A TFT may be arranged on the buffer layer 210. The TFT may include an active layer 220, a gate electrode 240, a source electrode 260, and a drain electrode 270.

The active layer 220 may include an inorganic semiconductor, such as silicon or polysilicon, an organic semiconductor, or an oxide semiconductor, and the active layer 220 may include a source region, a drain region, and a channel region.

A gate insulating film 230 for insulating the active layer 220 from the gate electrode 240 may be arranged on the active layer 220, and the gate electrode 240 may be arranged on the gate insulating film 230.

An interlayer insulating film 250 may be arranged on the gate electrode 240. The interlayer insulating film 250 may be arranged between the gate electrode 240 and the source electrode 260 to insulate the gate electrode 240 from the source electrode 260 and between the gate electrode 240 and the drain electrode 270 to insulate the gate electrode 240 from the drain electrode 270.

The source electrode 260 and the drain electrode 270 may be arranged on the interlayer insulating film 250. The interlayer insulating film 250 and the gate insulating film 230 may be formed to expose a source region and a drain region of the active layer 220, and the source electrode 260 and the drain electrode 270 may respectively contact the exposed portions of the source region and the drain region of the active layer 220.

The TFT may be electrically connected to a light-emitting device to drive the light-emitting device, and may be covered and protected by a passivation layer 280. The passivation layer 280 may include an inorganic insulating film, an organic insulating film, or any combination thereof. A light-emitting device may be provided on the passivation layer 280. The light-emitting device includes the first electrode 110, the interlayer 130, and the second electrode 150.

The first electrode 110 may be arranged on the passivation layer 280. The passivation layer 280 may not completely cover the drain electrode 270 and may expose a portion of the drain electrode 270. The first electrode 110 may be connected (for example, electrically connected) to the exposed portion of the drain electrode 270.

A pixel-defining film 290 including an insulating material may be arranged on the first electrode 110. The pixel-defining film 290 may expose a region of the first electrode 110, and the interlayer 130 may be formed on the exposed region of the first electrode 110. The pixel-defining film 290 may be a polyimide-based organic film or a polyacrylic organic film. Although not shown in FIG. 2, at least some layers of the interlayer 130 may extend beyond the upper portion of the pixel-defining film 290 to be provided in the form of a common layer.

The second electrode 150 may be arranged on the interlayer 130, and a capping layer 170 may be further included on the second electrode 150. The capping layer 170 may be formed to cover the second electrode 150.

The encapsulation portion 300 may be located on the capping layer 170. The encapsulation portion 300 may be disposed on a light-emitting device to protect the light-emitting device from moisture and/or oxygen. The encapsulation portion 300 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, or the like), an epoxy-based resin (for example, aliphatic glycidyl ether (AGE), or the like), or any combination thereof; or any combination of the inorganic film and the organic film.

FIG. 3 is a schematic cross-sectional view of an electronic apparatus according to another embodiment.

The electronic apparatus of FIG. 3 may differ from the electronic apparatus of FIG. 2, at least in that a light-shielding pattern 500 and a functional region 400 are further included on the encapsulation portion 300. The functional region 400 may be a color filter region, a color conversion region, or a combination of the color filter region and the color conversion region. In an embodiment, the light-emitting device included in the electronic apparatus of FIG. 3 may be a tandem light-emitting device.

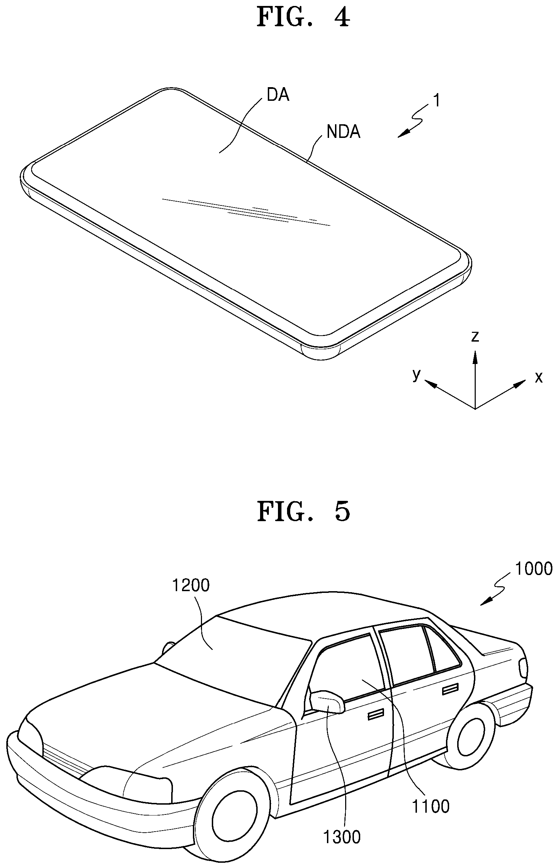

### [Description of FIG. 4]

FIG. 4 is a schematic perspective view of an electronic equipment 1 including a light-emitting device according to an embodiment.

The electronic equipment 1, which may be an apparatus that displays a moving image or a still image, may not only be a portable electronic device, such as a mobile phone, a smartphone, a tablet computer, a mobile communication terminal, an electronic notebook, an electronic book, a portable multimedia player (PMP), a navigation device, or an ultra-mobile personal computer (UMPC), but may also be various products, such as a television, a laptop, a monitor, a billboard, or an Internet of things (IoT) device. The electronic equipment 1 may be any such product as described above or a part thereof.

In an embodiment, the electronic equipment 1 may be a wearable device, such as a smart watch, a watch phone, a glasses-type display, or a head mounted display (HMD), or a part of the wearable device. However, embodiments are not limited thereto.

In an embodiment, examples of the electronic equipment 1 may include a dashboard of a vehicle, a center information display (CID) arranged on a center fascia or dashboard of a vehicle, a room mirror display that replaces a side-view mirror of a vehicle, an entertainment display for a rear seat of a vehicle, a display arranged on the back of a front seat, a head up display (HUD) installed on the front of a vehicle or projected on a front window glass, or a computer generated hologram augmented reality head up display (CGH AR HUD). FIG. 4 illustrates an embodiment in which the electronic equipment 1 is a smartphone, for convenience of explanation.

The electronic equipment 1 may include a display region DA and a non-display region NDA outside the display region DA. A display apparatus may implement an image through a two-dimensional array of pixels that are arranged in the display region DA.

The non-display region NDA may be a region that does not display an image, and may surround (e.g., entirely surround) the display region DA. A driver for providing electrical signals or power to display devices arranged in the display region DA may be arranged in the non-display area NDA. A pad, which is a region to which an electronic element or a printed circuit board may be electrically connected, may be arranged in the non-display area NDA.

In the electronic equipment 1, a length in an x-axis direction and a length in a y-axis direction may be different from each other. In an embodiment, as shown in FIG. 4, a length in the x-axis direction may be less than a length in the y-axis direction. In an embodiment, a length in the x-axis direction may be the same as a length in the y-axis direction. In an embodiment, a length in the x-axis direction may be greater than a length in the y-axis direction.

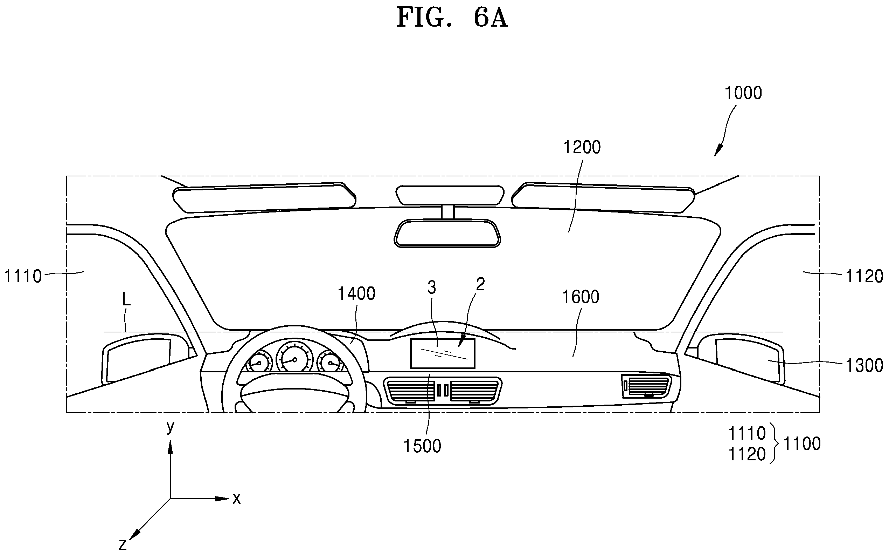

### [Descriptions of FIGS. 5 and 6A to 6C]

FIG. 5 is a schematic perspective view of an exterior of a vehicle 1000 as an electronic equipment including a light-emitting device, according to an embodiment. FIGS. 6A to 6C are each a schematic diagram of an interior of the vehicle 1000 according to embodiments.

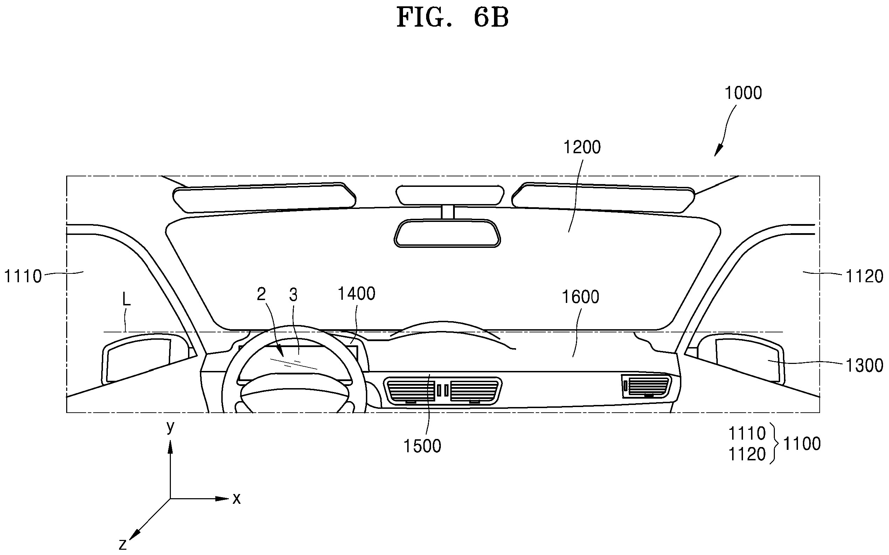

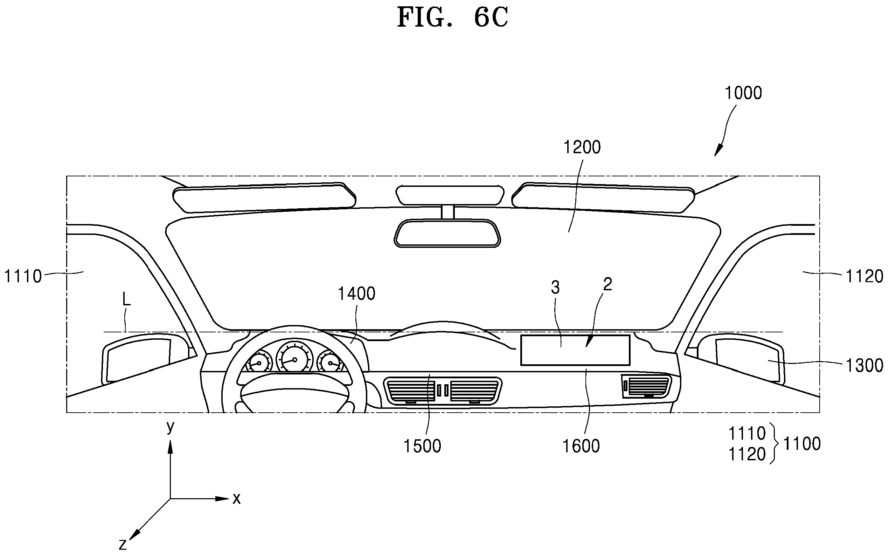

Referring to FIGS. 5, 6A, 6B, and 6C, embodiments of the vehicle 1000 may include various apparatuses for moving a subject to be transported, such as a person, an object, or an animal, from a departure point to a destination. In an embodiment, examples of the vehicle 1000 may include a vehicle traveling on a road or a track, a vessel moving over the sea or river, an airplane flying in the sky using the action of air, and the like.

The vehicle 1000 may travel on a road or a track. The vehicle 1000 may move in a selected or given direction according to the rotation of at least one wheel. In an embodiment, the vehicle 1000 may include a three-wheeled or four-wheeled vehicle, a construction machine, a two-wheeled vehicle, a prime mover device, a bicycle, and a train running on a track.

The vehicle 1000 may include a body having an interior and an exterior, and a chassis that is a portion excluding the body in which mechanical apparatuses necessary for driving are installed. The body of the vehicle 1000 may include a front panel, a bonnet, a roof panel, a rear panel, a trunk, a pillar provided at a boundary between doors, and the like. The chassis of the vehicle 1000 may include a power generating device, a power transmitting device, a driving device, a steering device, a braking device, a suspension device, a transmission device, a fuel device, front and rear wheels, left and right wheels, and the like.

The vehicle 1000 may include a side window glass 1100, a front window glass 1200, a side-view mirror 1300, a cluster 1400, a center fascia 1500, a passenger seat dashboard 1600, and a display apparatus 2.

The side window glass 1100 and the front window glass 1200 may be partitioned by a pillar arranged between the side window glass 1100 and the front window glass 1200.

The side window glass 1100 may be installed on a side of the vehicle 1000. In an embodiment, the side window glass 1100 may be installed on a door of the vehicle 1000. Multiple side window glasses 1100 may be provided and may face each other. In an embodiment, the side window glass 1100 may include a first side window glass 1110 and a second side window glass 1120. In an embodiment, the first side window glass 1110 may be arranged adjacent to the cluster 1400, and the second side window glass 1120 may be arranged adjacent to the passenger seat dashboard 1600.

In an embodiment, the side window glasses 1100 may be spaced apart from each other in an x direction or a -x direction. In an embodiment, the first side window glass 1110 and the second side window glass 1120 may be spaced apart from each other in the x direction or the -x direction. For example, a virtual straight line L connecting the side window glasses 1100 may extend in the x direction or the -x direction. In an embodiment, a virtual straight line L connecting the first side window glass 1110 and the second side window glass 1120 to each other may extend in the x direction or the -x direction.

The front window glass 1200 may be installed in front of the vehicle 1000. The front window glass 1200 may be arranged between the side window glasses 1100 facing each other.

The side-view mirror 1300 may provide a rear view of the vehicle 1000. The side-view mirror 1300 may be installed on the body of the vehicle 1000. In an embodiment, multiple side-view mirrors 1300 may be provided. For example, one of the side-view mirrors 1300 may be arranged outside the first side window glass 1110, and another of the side-view mirrors 1300 may be arranged outside the second side window glass 1120.

The cluster 1400 may be arranged in front of a steering wheel. The cluster 1400 may include a tachometer, a speedometer, a coolant thermometer, a fuel gauge, a turn signal indicator, a high beam indicator, a warning light, a seat belt warning light, an odometer, a tachograph, an automatic shift selector indicator, a door open warning light, an engine oil warning light, and/or a low fuel warning light.

The center fascia 1500 may include a control panel on which buttons for adjusting an audio device, an air conditioning device, and a seat heater are disposed. The center fascia 1500 may be arranged on a side of the cluster 1400.

The passenger seat dashboard 1600 may be spaced apart from the cluster 1400, and the center fascia 1500 may be arranged between the cluster 1400 and the passenger seat dashboard 1600. In an embodiment, the cluster 1400 may be arranged to correspond to a driver seat (not shown), and the passenger seat dashboard 1600 may be arranged to correspond to a passenger seat (not shown). In an embodiment, the cluster 1400 may be adjacent to the first side window glass 1110, and the passenger seat dashboard 1600 may be adjacent to the second side window glass 1120.

In an embodiment, the display apparatus 2 may include a display panel 3, and the display panel 3 may display an image. The display apparatus 2 may be arranged inside the vehicle 1000. In an embodiment, the display apparatus 2 may be arranged between the side window glasses 1100 facing each other. The display apparatus 2 may be arranged on at least one of the cluster 1400, the center fascia 1500, and the passenger seat dashboard 1600.

The display apparatus 2 may include an organic light-emitting display, an inorganic electroluminescent display, a quantum dot display, and the like. Hereinafter, an organic light-emitting display apparatus including the light-emitting device will be described as an example of the display apparatus 2. However, various types of display apparatuses as described above may be used in embodiments.

Referring to FIG. 6A, the display apparatus 2 may be arranged on the center fascia 1500. In an embodiment, the display apparatus 2 may display navigation information. In an embodiment, the display apparatus 2 may display information regarding audio settings, video settings, or vehicle settings.

Referring to FIG. 6B, the display apparatus 2 may be arranged on the cluster 1400. In an embodiment, the cluster 1400 may display driving information and the like through the display apparatus 2. For example, the cluster 1400 may digitally implement driving information and the like. The cluster 1400 may digitally display vehicle information and driving information as images. In an embodiment, a needle and a gauge of a tachometer and various warning lights or icons may be displayed by a digital signal.

Referring to FIG. 6C, the display apparatus 2 may be arranged on the passenger seat dashboard 1600. The display apparatus 2 may be embedded in the passenger seat dashboard 1600 or arranged on the passenger seat dashboard 1600. In an embodiment, the display apparatus 2 arranged on the passenger seat dashboard 1600 may display an image that is related to information displayed on the cluster 1400 and/or information displayed on the center fascia 1500. In an embodiment, the display apparatus 2 arranged on the passenger seat dashboard 1600 may display information that is different from information displayed on the cluster 1400 and/or information displayed on the center fascia 1500.

### [Manufacturing method]

The layers constituting the hole transport region, the emission layer, and the layers constituting the electron transport region may be formed in a selected region by using various methods such as vacuum deposition, spin coating, casting, Langmuir-Blodgett (LB) deposition, ink-jet printing, laser-printing, laser-induced thermal imaging, and the like.

When the layers constituting the hole transport region, the emission layer, and the layers constituting the electron transport region are formed by vacuum deposition, the deposition may be performed at a deposition temperature in a range of about 100 °C to about 500 °C, at a vacuum degree in a range of about 10⁻⁸ torr to about 10⁻³ torr, and at a deposition speed in a range of about 0.01 Å/sec to about 100 Å/sec, depending on a material to be included in a layer to be formed and the structure of a layer to be formed.

### [Definitions of terms]

The term "C₃-C₆₀ carbocyclic group" as used herein may be a cyclic group consisting of carbon atoms as the only ring-forming atoms and having three to sixty carbon atoms, and the term "C₁-C₆₀ heterocyclic group" as used herein may be a cyclic group that has one to sixty carbon atoms and further includes, in addition to a carbon atom, at least one heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. In an embodiment, the C₁-C₆₀ heterocyclic group may have 3 to 61 ring-forming atoms.

The term "cyclic group" as used herein may include may be C₃-C₆₀ carbocyclic group or a C₁-C₆₀ heterocyclic group.

The term "π electron-rich C₃-C₆₀ cyclic group" as used herein may be a cyclic group that has three to sixty carbon atoms and may not include *-N=*' as a ring-forming moiety, and the term "π electron-deficient nitrogen-containing C₁-C₆₀ cyclic group" as used herein may be a heterocyclic group that has one to sixty carbon atoms and may include *-N=*' as a ring-forming moiety.

In embodiments,
a C₃-C₆₀ carbocyclic group may be a T1 group or a group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
a C₁-C₆₀ heterocyclic group may be a T2 group, a group in which two or more T2 groups are condensed with each other, or a group in which at least one T2 group and at least one T1 group are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),
a π electron-rich C₃-C₆₀ cyclic group may be a T1 group, a group in which two or more T1 groups are condensed with each other, a T3 group, a group in which two or more T3 groups are condensed with each other, or a group in which at least one T3 group and at least one T1 group are condensed with each other (for example, a C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, or the like),
a π electron-deficient nitrogen-containing C₁-C₆₀ cyclic group may be a T4 group, a group in which two or more T4 groups are condensed with each other, a group in which at least one T4 group and at least one T1 group are condensed with each other, a group in which at least one T4 group and at least one T3 group are condensed with each other, or a group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and the like), wherein
a T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group,
a T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
a T3 group may be a furan group, a thiophene group, a 1H-pyrrole group, a silole group, or a borole group, and
a T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group", "C₃-C₆₀ carbocyclic group", "C₁-C₆₀ heterocyclic group", "π electron-rich C₃-C₆₀ cyclic group", or "π electron-deficient nitrogen-containing C₁-C₆₀ cyclic group" as used herein may each be a group condensed to any cyclic group, a monovalent group, or a polyvalent group (for example, a divalent group, a trivalent group, a tetravalent group, etc.) according to the structure of a formula for which the corresponding term is used. For example, a "benzene group" may be a benzo group, a phenyl group, a phenylene group, or the like, which may be readily understood by those of ordinary skill in the art according to the structure of a formula including the "benzene group".

Examples of a monovalent C₃-C₆₀ carbocyclic group or a monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Examples of a divalent C₃-C₆₀ carbocyclic group or a divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as used herein may be a linear or branched monovalent aliphatic hydrocarbon group that has one to sixty carbon atoms, and examples thereof may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein may be a divalent group having a same structure as the C₁-C₆₀ alkyl group.

The term "C₂-C₆₀ alkenyl group" as used herein may be a monovalent hydrocarbon group having at least one carbon-carbon double bond in the middle or at a terminus of the C₂-C₆₀ alkyl group, and examples thereof may include an ethenyl group, a propenyl group, and a butenyl group. The term "C₂-C₆₀ alkenylene group" as used herein may be a divalent group having a same structure as the C₂-C₆₀ alkenyl group.

The term "C₂-C₆₀ alkynyl group" as used herein may be a monovalent hydrocarbon group having at least one carbon-carbon triple bond in the middle or at a terminus of the C₂-C₆₀ alkyl group, and examples thereof may include an ethynyl group and a propynyl group. The term "C₂-C₆₀ alkynylene group" as used herein may be a divalent group having a same structure as the C₂-C₆₀ alkynyl group.

The term "C₁-C₆₀ alkoxy group" as used herein may be a monovalent group represented by -O(A₁₀₁) (wherein A₁₀₁ may be a C₁-C₆₀ alkyl group), and examples thereof may include a methoxy group, an ethoxy group, and an isopropyloxy group.

The term "C₃-C₁₀ cycloalkyl group" as used herein may be a monovalent saturated hydrocarbon cyclic group having three to ten carbon atoms, and examples thereof may include a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, and a bicyclo[2.2.2]octyl group. The term "C₃-C₁₀ cycloalkylene group" as used herein may be a divalent group having a same structure as the C₃-C₁₀ cycloalkyl group.

The term "C₁-C₁₀ heterocycloalkyl group" as used herein may be a monovalent cyclic group that has one to ten carbon atoms and further includes, in addition to the carbon atoms, at least one heteroatom as a ring-forming atom, and examples thereof may include a 1,2,3,4-oxatriazolidinyl group, a tetrahydrofuranyl group, and a tetrahydrothiophenyl group. The term "C₁-C₁₀ heterocycloalkylene group" as used herein may be a divalent group having a same structure as the C₁-C₁₀ heterocycloalkyl group.

The term "C₃-C₁₀ cycloalkenyl group" as used herein may be a monovalent cyclic group that has three to ten carbon atoms and at least one carbon-carbon double bond in the cyclic structure thereof and no aromaticity, and examples thereof may include a cyclopentenyl group, a cyclohexenyl group, and a cycloheptenyl group. The term "C₃-C₁₀ cycloalkenylene group" as used herein may be a divalent group having a same structure as the C₃-C₁₀ cycloalkenyl group.

The term "C₁-C₁₀ heterocycloalkenyl group" as used herein may be a monovalent cyclic group that has one to ten carbon atoms, further includes, in addition to the carbon atoms, at least one heteroatom as a ring-forming atom, and has at least one double bond in the cyclic structure thereof. Examples of a C₁-C₁₀ heterocycloalkenyl group may include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein may be a divalent group having a same structure as the C₁-C₁₀ heterocycloalkenyl group.

The term "C₆-C₆₀ aryl group" as used herein may be a monovalent group having a carbocyclic aromatic system of six to sixty carbon atoms, and the term "C₆-C₆₀ arylene group" as used herein may be a divalent group having a carbocyclic aromatic system of six to sixty carbon atoms. Examples of a C₆-C₆₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group. When the C₆-C₆₀ aryl group and the C₆-C₆₀ arylene group each include two or more rings, the respective two or more rings may be condensed with each other.

The term "C₁-C₆₀ heteroaryl group" as used herein may be a monovalent group having a heterocyclic aromatic system that has one to sixty carbon atoms and further includes, in addition to the carbon atoms, at least one heteroatom as a ring-forming atom. The term "C₁-C₆₀ heteroarylene group" as used herein may be a divalent group having a heterocyclic aromatic system that has one to sixty carbon atoms and further includes, in addition to the carbon atoms, at least one heteroatom as a ring-forming atom. Examples of a C₁-C₆₀ heteroaryl group may include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the respective two or more rings may be condensed with each other.

The term "monovalent non-aromatic condensed polycyclic group" as used herein may be a monovalent group having two or more rings condensed with each other, only carbon atoms (for example, eight to sixty carbon atoms) as ring-forming atoms, and no aromaticity in its molecular structure when considered as a whole. Examples of a monovalent non-aromatic condensed polycyclic group may include an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein may be a divalent group having a same structure as the monovalent non-aromatic condensed polycyclic group.

The term "monovalent non-aromatic condensed heteropolycyclic group" as used herein may be a monovalent group that has two or more rings condensed with each other, further includes, in addition to carbon atoms (for example, one to sixty carbon atoms), at least one heteroatom as a ring-forming atom, and has no aromaticity in its molecular structure when considered as a whole. Examples of a monovalent non-aromatic condensed heteropolycyclic group may include a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, and a benzothienodibenzothiophenyl group. The term "divalent non-aromatic condensed heteropolycyclic group" as used herein may be a divalent group having a same structure as the monovalent non-aromatic condensed heteropolycyclic group.

The term "C₆-C₆₀ aryloxy group" as used herein may be a group represented by -O(A₁₀₂) (wherein A₁₀₂ may be a C₆-C₆₀ aryl group), and the term "C₆-C₆₀ arylthio group" as used herein may be a group represented by -S(A₁₀₃) (wherein A₁₀₃ may be a C₆-C₆₀ aryl group).

The term "C₇-C₆₀ arylalkyl group" as used herein may be a group represented by -(A₁₀₄)(A₁₀₅) (wherein A₁₀₄ may be a C₁-C₅₄ alkylene group, and A₁₀₅ may be a C₆-C₅₉ aryl group), and the term "C₂-C₆₀ heteroarylalkyl group" as used herein may be a group represented by -(A₁₀₆)(A₁₀₇) (wherein A₁₀₆ may be a C₁-C₅₉ alkylene group, and A₁₀₇ may be a C₁-C₅₉ heteroaryl group).

In the specification, the group "R₁₀₂" may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂).

In the specification, Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, or any combination thereof.

The term "heteroatom" as used herein may be any atom other than a carbon atom or a hydrogen atom. Examples of a heteroatom may include O, S, N, P, Si, B, Ge, Se, and any combination thereof.

The term "third-row transition metal" may include hafnium (Hf), tantalum (Ta), tungsten (W), rhenium (Re), osmium (Os), iridium (Ir), platinum (Pt), and gold (Au).

In the specification, the terms "Ph" and "PH" each refers to a phenyl group, the term "Me" refers to a methyl group, the term "Et" refers to an ethyl group, the terms "tert-Bu" and "Bu^{t}" each refers to a tert-butyl group, and the term "OMe" refers to a methoxy group.

The term "biphenyl group" as used herein may be "phenyl group that is substituted with a phenyl group". For example, the "biphenyl group" may be a substituted phenyl group having a C₆-C₆₀ aryl group as a substituent.

The term "terphenyl group" as used herein may be a "phenyl group that is substituted with a biphenyl group". For example, the "terphenyl group" may be a substituted phenyl group having, as a substituent, a C₆-C₆₀ aryl group substituted with a C₆-C₆₀ aryl group.

The symbols *, *', and *" as used herein, unless defined otherwise, each refer to a binding site to a neighboring atom in a corresponding formula or moiety.

In the specification, the terms "x-axis", "y-axis", and "z-axis" are not limited to three axes in an orthogonal coordinate system (for example, a Cartesian coordinate system), and may be interpreted in a broader sense than the aforementioned three axes in an orthogonal coordinate system. For example, the x-axis, y-axis, and z-axis may describe axes that are orthogonal to each other, or may describe axes that are in different directions that are not orthogonal to each other.

Hereinafter, compounds according to embodiments and light-emitting devices according to embodiments will be described in detail with reference to the following Synthesis Examples and Examples. The wording "B was used instead of A" used in describing Synthesis Examples means that an identical molar equivalent of B was used in place of A.

### [Synthesis Examples and Examples]

### Synthesis Example 1: Synthesis of Compound 1

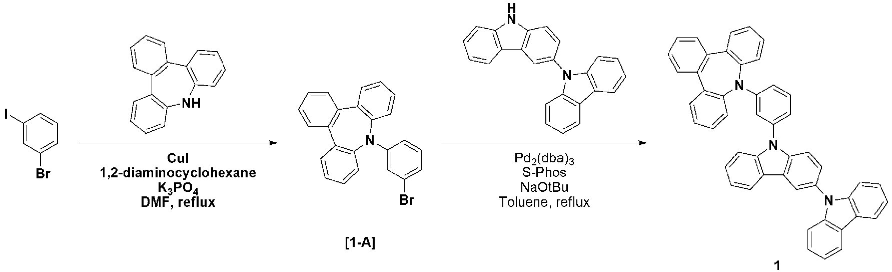

### 1) Synthesis of Intermediate [1-A]

850 mg (3.0 mmol) of 1-bromo-3-iodobenzene, 730 mg (3.0 mmol) of 9H-tribenzo[b,d,f]azepine, 290 mg (1.5 mmol) of copper iodide, 340 mg (3.0 mmol) of 1,2-diaminocyclohexane, and 1.59 g (7.5 mmol) of potassium phosphate tribasic were put into a reaction vessel and suspended in 30 mL of DMF. The reaction temperature was raised to 160 °C, and the reaction mixture was stirred for 12 hours. After completion of the reaction, the reaction mixture was cooled to room temperature and was subjected to an extraction process using ethyl acetate. The extracted organic layer was washed with an aqueous solution of saturated sodium chloride and dried with sodium sulfate. A residue obtained by removing the solvent therefrom was separated by column chromatography to obtain 900 mg (2.25 mmol) of a target compound.

### 2) Synthesis of Compound 1

900 mg (2.25 mmol) of Intermediate [1-A], 750 mg (2.25 mmol) of 9H-3,9'-bicarbazole, 100 mg (0.11 mmol) of tris(dibenzylideneacetone)dipalladium, 70 mg (0.18 mmol) of S-Phos, and 430 mg (4.50 mmol) of sodium tert-butoxide were put into a reaction vessel and suspended in 200 mL of toluene. The reaction temperature was raised to 110 °C, and the reaction mixture was stirred for 12 hours. After completion of the reaction, the reaction mixture was cooled to room temperature and was subjected to an extraction process using ethyl acetate. The extracted organic layer was washed with an aqueous solution of saturated sodium chloride and dried with sodium sulfate. A residue obtained by removing the solvent therefrom was separated by column chromatography to obtain 1.08 g (1.67 mmol) of a target compound.

### Synthesis Example 2: Synthesis of Compound 11

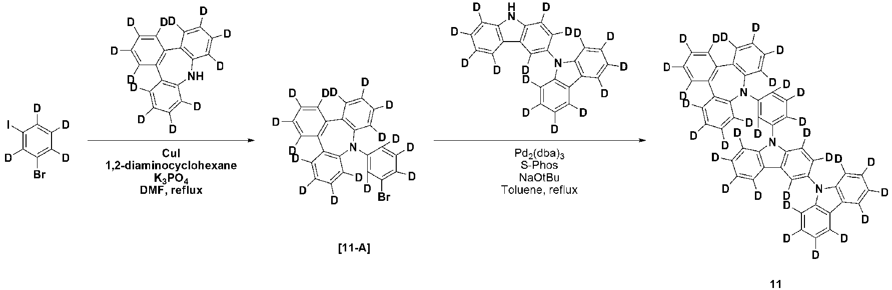

960 mg (1.41 mmol) of Compound 11 was obtained in the same manner as in Synthesis Example 1, except that 1-bromo-3-iodobenzene-2,4,5,6-d4 was used instead of 1-bromo-3-iodobenzene, 9H-tribenzo[b,d,f]azepine-1,2,3,4,5,6,7,8,10,11,12,13-d12 was used instead of 9H-tribenzo[b,d,f]azepine, and 9H-3,9'-bicarbazole-1,1',2,2',3',4,4',5,5',6,6',7,7',8,8'-d15 was used instead of 9H-3,9'-bicarbazole.

### Synthesis Example 3: Synthesis of Compound 31

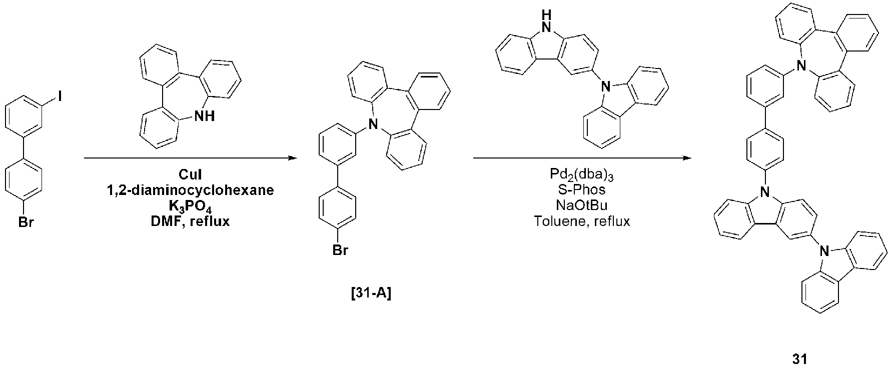

880 mg (1.22 mmol) of Compound 31 was obtained in the same manner as in Synthesis Example 1, except that 4'-bromo-3-iodo-1,1'-biphenyl was used instead of 1-bromo-3-iodobenzene.

### Synthesis Example 4: Synthesis of Compound 33

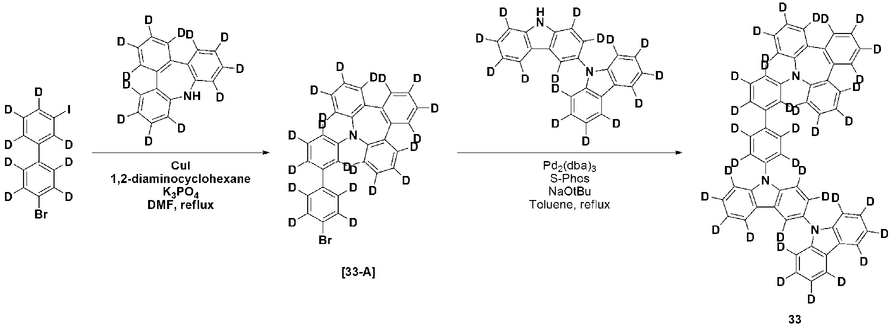

1.01 g (1.33 mmol) of Compound 33 was obtained in the same manner as in Synthesis Example 1, except that 4'-bromo-3-iodo-1,1'-biphenyl-2,2',3',4,5,5',6,6'-d8 was used instead of 1-bromo-3-iodobenzene, 9H-tribenzo[b,d,f]azepine-1,2,3,4,5,6,7,8,10,11,12,13-d12 was used instead of 9H-tribenzo[b,d,f]azepine, and 9H-3,9'-bicarbazole-1,1',2,2',3',4,4',5,5',6,6',7,7',8,8'-d15 was used instead of 9H-3,9'-bicarbazole.

### Synthesis Example 5: Synthesis of Compound 51

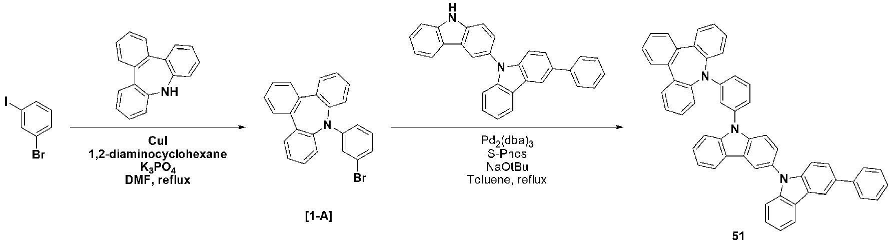

990 mg (1.37 mmol) of Compound 51 was obtained in the same manner as in Synthesis Example 1, except that 3'-phenyl-9H-3,9'-bicarbazole was used instead of 9H-3,9'-bicarbazole.

### Synthesis Example 6: Synthesis of Compound 57

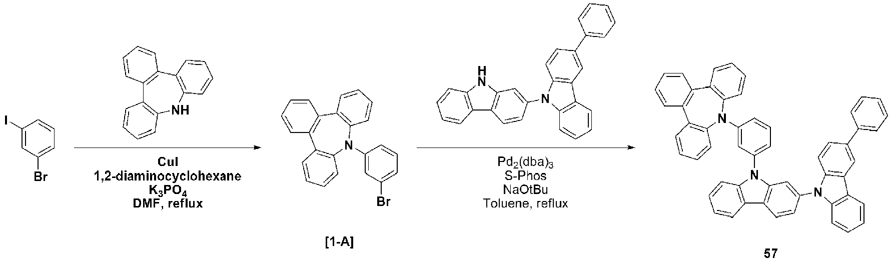

1.03 mg (1.42 mmol) of Compound 57 was obtained in the same manner as in Synthesis Example 1, except that 3'-phenyl-9H-2,9'-bicarbazole was used instead of 9H-3,9'-bicarbazole.

**[Table 1]**

| Compound No. | MS/FAB | |
|---|---|---|
| | found | calc. |
| Compound 1 | 649.2524 | 649.2518 |
| Compound 11 | 680.4460 | 680.4464 |
| Compound 31 | 725.2825 | 725.2831 |

| | | |
|---|---|---|
| Compound 33 | 760.5024 | 760.5028 |
| Compound 51 | 725.2824 | 725.2831 |
| Compound 57 | 725.2825 | 725.2831 |

### [Example 1]

Corning 15 Ω/cm² (1,200 Å) ITO glass substrate was cut to a size of 50 mm x 50 mm x 0.5 mm, sonicated with isopropyl alcohol and pure water each for 5 minutes, and cleaned by irradiation of ultraviolet rays and exposure of ozone thereto for 30 minutes. The resultant glass substrate was loaded onto a vacuum deposition apparatus.

HATCN was formed on the substrate to form a hole injection layer having a thickness of 100 Å, BCFN, which is a first hole-transporting material, was vacuum deposited thereon to a thickness of 600 Å, and SiCzCz, which is a second hole-transporting compound, was vacuum deposited thereon to a thickness of 50 Å, thereby completing the formation of a hole transport layer.

Compound 1 and SiTrzCz2 as hosts and PtON-TBBI as a phosphorescent dopant were co-deposited on the hole transport layer at a weight ratio of 60:27:13 to form an emission layer having a thickness of 350 Å.

mSiTrz was deposited on the emission layer to form a first electron transport layer having a thickness of 50 Å, and mSiTrz and LiQ were co-deposited thereon to form a second electron transport layer at a ratio of 1:1, thereby completing the formation of an electron transport layer having a thickness of 350 Å. LiF, which is a halogenated alkaline metal, was deposited on the electron transport layer to form an electron injection layer having a thickness of 15 Å, and Al was vacuum deposited thereon to form an LiF/Al electrode having a thickness of 80 Å, thereby completing the manufacture of an organic light-emitting device.

The materials used in the organic light-emitting device may be represented by the following formulae.

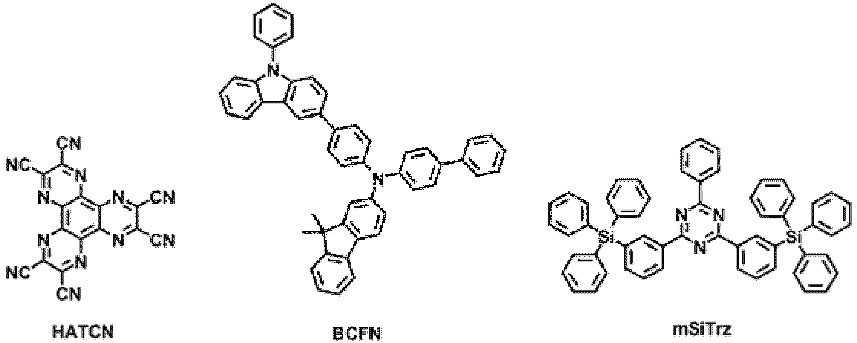

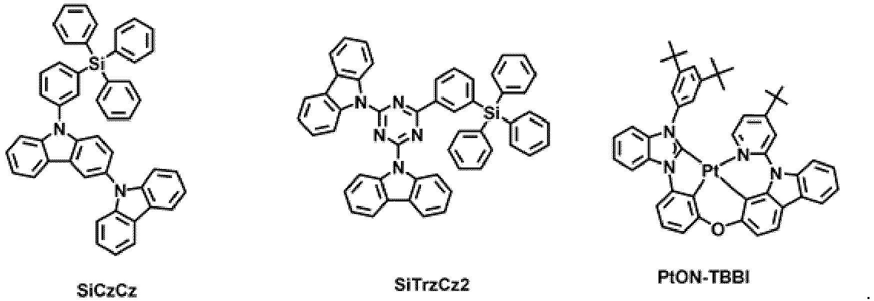

### [Examples 2 to 6 and Comparative Examples 1 to 6]

Organic light-emitting devices were manufactured in the same manner as in Synthesis Example 1, except that compounds shown in Table 1 were each used instead of Compound 1 to form an emission layer.

### Evaluation Example 1

The driving voltage (V), color conversion efficiency ratio, and lifespan ratio of the organic light-emitting devices according to Examples 1 to 6 and Comparative Examples 1 to 6 were each measured at 1,000 cd/m² using Keithley MU 236 and luminance meter PR650, and results thereof are shown in Table 2. The color conversion efficiency ratio and the lifespan ratio were measured relative to the value of Comparative Example 1 as 100.

**[Table 2]**

| Classification | Luminescent material | Driving voltage (V) | Color conversion efficiency ratio | Lifespan ratio | Emission color |
|---|---|---|---|---|---|
| Example 1 | Compound 1 | 4.7 | 119 | 115 | Blue |
| Example 2 | Compound 11 | 4.6 | 118 | 126 | Blue |
| Example 3 | Compound 31 | 4.8 | 126 | 110 | Blue |
| Example 4 | Compound 33 | 4.8 | 121 | 121 | Blue |
| Example 5 | Compound 51 | 4.5 | 111 | 117 | Blue |
| Example 6 | Compound 57 | 4.6 | 108 | 107 | Blue |
| Comparative Example 1 | C1 | 5.2 | 100 | 100 | Blue |
| Comparative Example 2 | C2 | 5.5 | 61 | 47 | Blue |
| Comparative Example 3 | C3 | 5.6 | 71 | 21 | Blue |
| Comparative Example 4 | C4 | 5.8 | 40 | 25 | Blue |
| Comparative Example 5 | C5 | 6.2 | 22 | 15 | Blue |
| Comparative Example 6 | C6 | 5.5 | 82 | 76 | Blue |

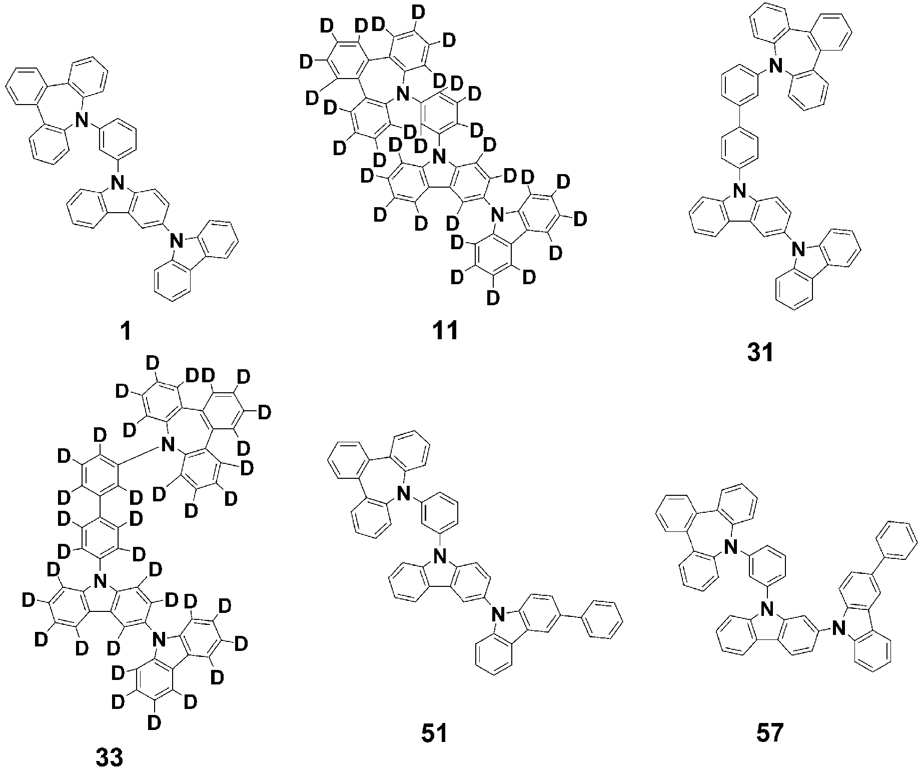

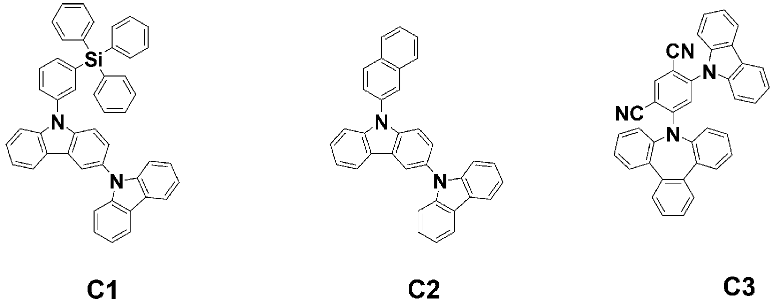

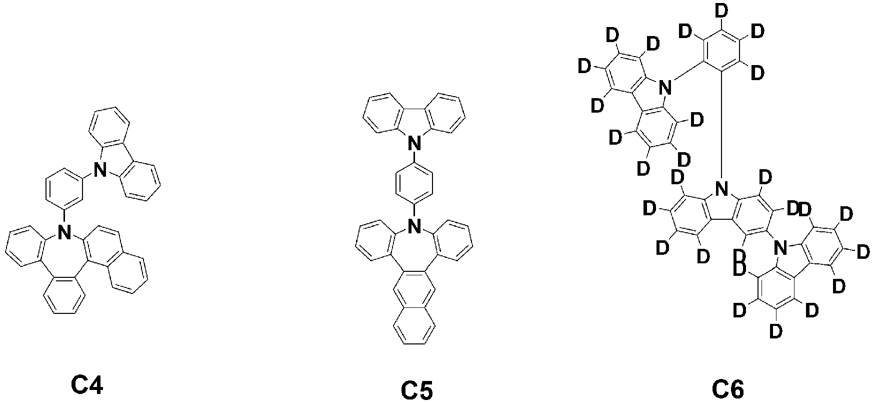

Referring to the results of Table 2, it could be confirmed that the organic light-emitting devices of Examples 1 to 6 had lower driving voltage, excellent color conversion efficiency, and excellent lifespan compared to the organic light-emitting devices of Comparative Examples 1 to 6.

A light-emitting device including the heterocyclic compound may have low driving voltage, high efficiency, and long lifespan. In an embodiment, a high-quality electronic apparatus and a high-quality consumer product may be manufactured by using the light-emitting device.

Embodiments have been disclosed herein, and although terms are employed, they are used and are to be interpreted in a generic and descriptive sense only and not for purposes of limitation. In some instances, as would be apparent by one of ordinary skill in the art, features, characteristics, and/or elements described in connection with an embodiment may be used singly or in combination with features, characteristics, and/or elements described in connection with other embodiments unless otherwise specifically indicated. Accordingly, it will be understood by those of ordinary skill in the art that various changes in form and details may be made without departing from the scope of the disclosure.

## Claims

1. A light-emitting device (10) comprising:
a first electrode (110);
a second electrode (150) facing the first electrode (110);
an interlayer (130) between the first electrode (110) and the second electrode (150) and comprising an emission layer; and
a heterocyclic compound represented by Formula 1: 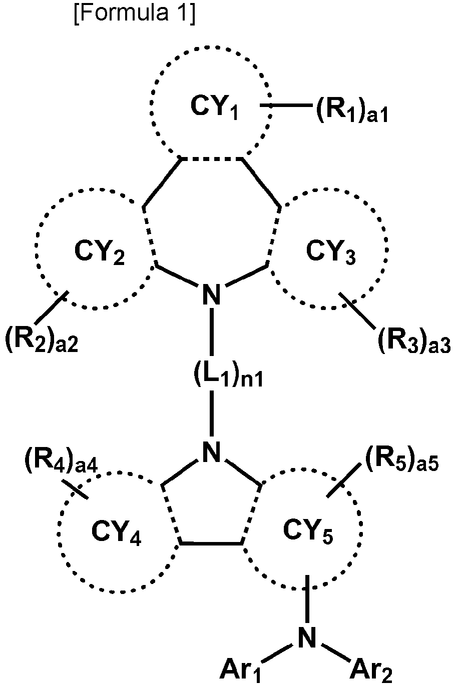 wherein in Formula 1,
rings CY₁ to CY₅ are each independently a C₅-C₆₀ carbocyclic group or a C₂-C₆₀ heterocyclic group,
n1 is an integer from 1 to 5,
L₁ is a C₃-C₆₀ carbocyclic group that is unsubstituted or substituted with at least one R₁₀ₐ or a C₁-C₆₀ heterocyclic group that is unsubstituted or substituted with at least one R₁₀ₐ,
a1 to a5 are each independently an integer from 0 to 10,
Ar₁, Ar₂, and R₁ to R₅ are each independently hydrogen, deuterium, -F, -Cl, -Br, -I, a hydroxyl group, a cyano group, a nitro group, an amidino group, a hydrazino group, a hydrazono group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₁₀ cycloalkyl group that is unsubstituted or substituted with at least one R₁₀ₐ, a C₁-C₁₀ heterocycloalkyl group that is unsubstituted or substituted with at least one R₁₀ₐ, a C₃-C₁₀ cycloalkenyl group that is unsubstituted or substituted with at least one R₁₀ₐ, a C₁-C₁₀ heterocycloalkenyl group that is unsubstituted or substituted with at least one R₁₀ₐ, a C₆-C₆₀ aryl group that is unsubstituted or substituted with at least one R₁₀ₐ, a C₆-C₆₀ aryloxy group that is unsubstituted or substituted with at least one R₁₀ₐ, a C₆-C₆₀ arylthio group that is unsubstituted or substituted with at least one R₁₀ₐ, a C₁-C₆₀ heteroaryl group that is unsubstituted or substituted with at least one R₁₀ₐ, a C₁-C₆₀ heteroaryloxy group that is unsubstituted or substituted with at least one R₁₀ₐ, a C₁-C₆₀ heteroarylthio group that is unsubstituted or substituted with at least one R₁₀ₐ, a monovalent non-aromatic condensed polycyclic group that is unsubstituted or substituted with at least one R₁₀ₐ, a monovalent non-aromatic condensed heteropolycyclic group that is unsubstituted or substituted with at least one R₁₀ₐ, - Si(Q₁)(Q₂)(Q₃), -B(Q₁)(Q₂), -N(Q₁)(Q₂), -P(Q₁)(Q₂), -C(=O)(Q₁), -S(=O)(Q₁), - S(=O)₂(Q₁), -P(=O)(Q₁)(Q₂), or -P(=S)(Q₁)(Q₂),
two or more neighboring groups among Ar₁, Ar₂, and R₁ to R₅ are optionally bonded to each other to form a C₅-C₆₀ carbocyclic group that is unsubstituted or substituted with at least one R₁₀ₐ or a C₁-C₆₀ heterocyclic group that is unsubstituted or substituted with at least one R₁₀ₐ,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, or a C₁-C₆₀ alkoxy group; or
a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, - F, a cyano group, a C₁-C₆₀ alkyl group, a C₁-C₆₀ alkoxy group, a phenyl group, a biphenyl group, or a combination thereof.

2. The light-emitting device (10) of claim 1, wherein:
(i) the interlayer further comprises:
a hole transport region between the first electrode and the emission layer; and
an electron transport region between the emission layer and the second electrode,
the hole transport region comprises a hole injection layer, a hole transport layer, an emission auxiliary layer, an electron-blocking layer, or a combination thereof, and
the electron transport region comprises a buffer layer, a hole-blocking layer, an electron transport layer, an electron injection layer, or a combination thereof; and/or
(ii) the emission layer comprises:
a host; and
a dopant, and
the host comprises the heterocyclic compound.

3. The light-emitting device (10) of claim 1 or claim 2, wherein:
(i) the light-emitting device (10) further comprises:
at least one of a first capping layer outside the first electrode, and a second capping layer outside the second electrode, wherein
the at least one of the first capping layer and the second capping layer comprises the heterocyclic compound; and/or
(ii) the emission layer emits blue light.

4. An electronic apparatus comprising the light-emitting device of any one of claims 1 to 3.

5. The electronic apparatus of claim 4, further comprising:
a thin-film transistor electrically connected to the light-emitting device (10); and
a color filter, a color conversion layer, a touch screen layer, a polarizing layer, or a combination thereof, optionally wherein the color conversion layer comprises quantum dots.

6. An electronic equipment (1) comprising the light-emitting device (10) of any one of claims 1 to 3.

7. The electronic equipment (1) of claim 6, wherein the electronic equipment (1) is a flat panel display, a curved display, a computer monitor, a medical monitor, a television, an advertisement board, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a microdisplay, a three-dimensional (3D) display, a virtual reality display, an augmented reality display, a vehicle, a video wall with multiple displays tiled together, a theater screen, a stadium screen, a phototherapy device, or a signboard.

8. A heterocyclic compound represented by Formula 1: 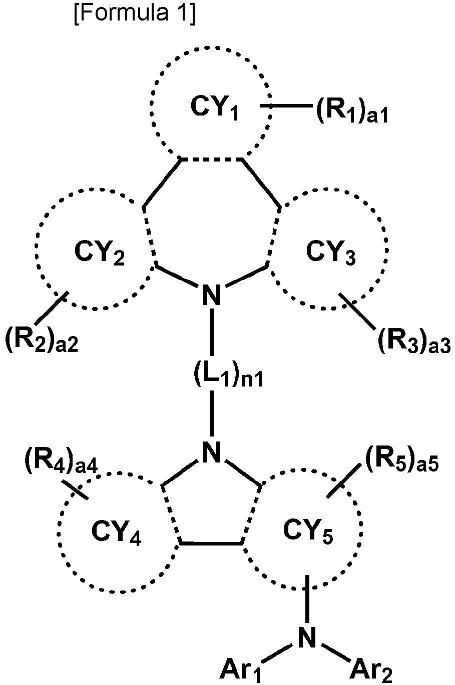 wherein in Formula 1,
ring CY₁ to ring CY₅ are each independently a C₅-C₆₀ carbocyclic group or a C₂-C₆₀ heterocyclic group,
n1 is an integer from 1 to 5,
L₁ is a C₃-C₆₀ carbocyclic group that is unsubstituted or substituted with at least one R₁₀ₐ or a C₁-C₆₀ heterocyclic group that is unsubstituted or substituted with at least one R₁₀ₐ,
a1 to a5 are each independently an integer from 0 to 10,
Ar₁, Ar₂, and R₁ to R₅ are each independently hydrogen, deuterium, -F, -Cl, -Br, -I, a hydroxyl group, a cyano group, a nitro group, an amidino group, a hydrazino group, a hydrazono group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₁₀ cycloalkyl group that is unsubstituted or substituted with at least one R₁₀ₐ, a C₁-C₁₀ heterocycloalkyl group that is unsubstituted or substituted with at least one R₁₀ₐ, a C₃-C₁₀ cycloalkenyl group that is unsubstituted or substituted with at least one R₁₀ₐ, a C₁-C₁₀ heterocycloalkenyl group that is unsubstituted or substituted with at least one R₁₀ₐ, a C₆-C₆₀ aryl group that is unsubstituted or substituted with at least one R₁₀ₐ, a C₆-C₆₀ aryloxy group that is unsubstituted or substituted with at least one R₁₀ₐ, a C₆-C₆₀ arylthio group that is unsubstituted or substituted with at least one R₁₀ₐ, a C₁-C₆₀ heteroaryl group that is unsubstituted or substituted with at least one R₁₀ₐ, a C₁-C₆₀ heteroaryloxy group that is unsubstituted or substituted with at least one R₁₀ₐ, a C₁-C₆₀ heteroarylthio group that is unsubstituted or substituted with at least one R₁₀ₐ, a monovalent non-aromatic condensed polycyclic group that is unsubstituted or substituted with at least one R₁₀ₐ, a monovalent non-aromatic condensed heteropolycyclic group that is unsubstituted or substituted with at least one R₁₀ₐ, - Si(Q₁)(Q₂)(Q₃), -B(Q₁)(Q₂), -N(Q₁)(Q₂), -P(Q₁)(Q₂), -C(=O)(Q₁), -S(=O)(Q₁), - S(=O)₂(Q₁), -P(=O)(Q₁)(Q₂), or -P(=S)(Q₁)(Q₂),
two or more neighboring groups among Ar₁, Ar₂, and R₁ to R₅ are optionally bonded to each other to form a C₅-C₆₀ carbocyclic group that is unsubstituted or substituted with at least one R₁₀ₐ or a C₁-C₆₀ heterocyclic group that is unsubstituted or substituted with at least one R₁₀ₐ,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, or a C₁-C₆₀ alkoxy group; or
a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, - F, a cyano group, a C₁-C₆₀ alkyl group, a C₁-C₆₀ alkoxy group, a phenyl group, a biphenyl group, or a combination thereof.

9. The heterocyclic compound of claim 8, wherein:
(i) ring CY₁ to ring CY₅ are each independently a benzene group, a naphthalene group, an anthracene group, a phenanthrene group, a triphenylene group, a pyrene group, a chrysene group, an acenaphthylene group, a perylene group, a benzopyrene group, a benzochrysene group, a benzotriphenylene group, a fluoranthene group, a coronene group, a cyclopentadiene group, a 1,2,3,4-tetrahydronaphthalene group, an acridine group, a thiophene group, a furan group, an indole group, a benzoborole group, a benzophosphole group, an indene group, a benzosilole group, a benzogermole group, a benzothiophene group, a benzoselenophene group, a benzofuran group, a benzotellurophene group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzotellurophene group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group; or
(ii) ring CY₁ to ring CY₅ are each independently a benzene group, a naphthalene group, or a pyridine group.

10. The heterocyclic compound of claim 8 or claim 9, wherein:
(i) L₁ is a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ, and
R₁₀ₐ is the same as defined in Formula 1; or
(ii) L₁ is a group represented by one of Formulae 3-1 to 3-30: 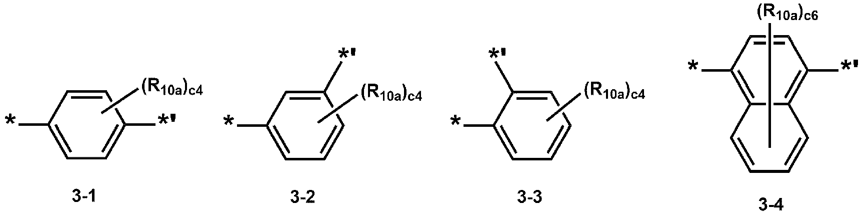 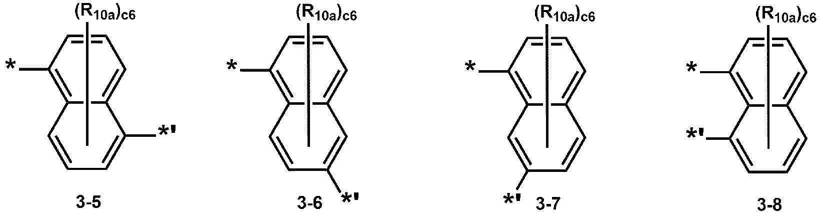 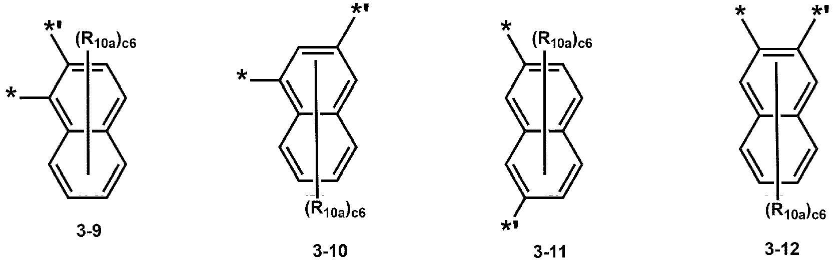 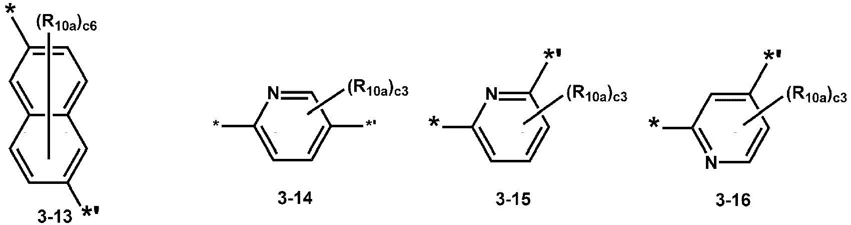 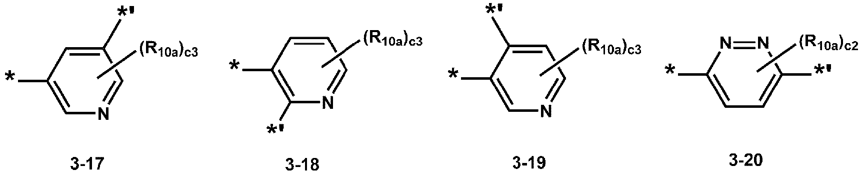 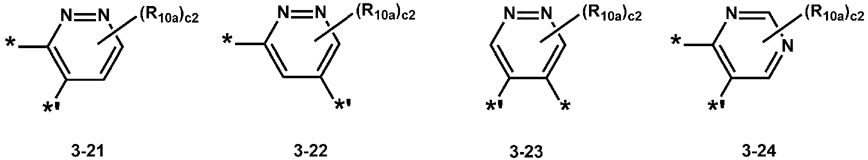 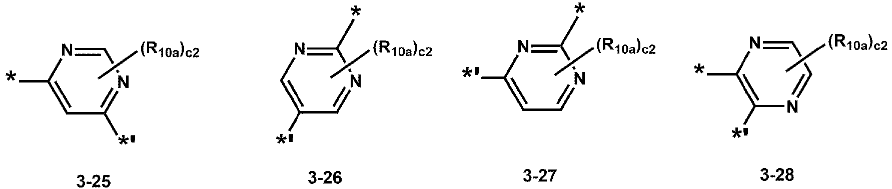 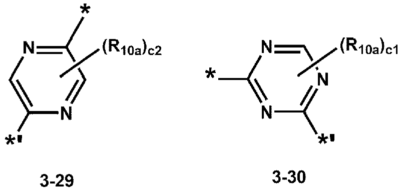 wherein in Formulae 3-1 to 3-30,
R₁₀ₐ is the same as defined in Formula 1,
c1 is 0 or 1,
c2 is an integer from 0 to 2,
c3 is an integer from 0 to 3,
c4 is an integer from 0 to 4,
c6 is an integer from 0 to 6, and
* and *' each indicate a binding site to a neighboring atom.

11. The heterocyclic compound of any one of claims 8 to 10, wherein in Formula 1, a moiety represented by 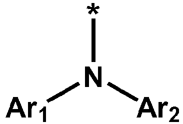 is a moiety represented by Formula 6: 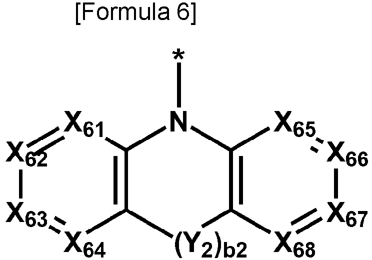 wherein in Formula 6,
Y₂ is *'-N(Z₆ₐ)-*", *'-B(Z₆ₐ)-*", '-P(Z₆ₐ)-", *'-C(Z₆ₐ)(Z_{6b})-*", *'-Si(Z₆ₐ)(Z_{6b})-*", *'-Ge(Z₆ₐ)(Z_{6b})-*", *'-S-*", *'-Se-*", *'-O-*", *'-C(=O)-*", *'-S(=O)-*", *'-S(=O)₂-*", *'-C(=S)-*", *'-N=*", *'=N-*", *'-C(Z₆ₐ)=*", *'=C(Z₆ₐ)-*", *'-Si(Z₆ₐ)=*", *'=Si(Z₆ₐ)-*", *'-Ge(Z₆ₐ)=*", or *'=Ge(Z₆ₐ)-*", and *' and *" each indicate a binding site to a neighboring atom,
b2 is an integer from 0 to 2,
when b2 is 0, *-(Y₂)_{b2}-*' is a single bond,
when b2 is 2, two of Y₂ are identical to or different from each other,
X₆₁ is N or C(Z₆₁),
X₆₂ is N or C(Z₆₂),
X₆₃ is N or C(Z₆₃),
X₆₄ is N or C(Z₆₄),
X₆₅ is N or C(Z₆₅),
X₆₆ is N or C(Z₆₆),
X₆₇ is N or C(Z₆₇),
X₆₈ is N or C(Z₆₈),
Z₆ₐ, Z_{6b}, and Z₆₁ to Z₆₈ are each independently the same as defined in connection with R₁₀ₐ in Formula 1,
two or more neighboring groups among Z₆ₐ, Z_{6b}, and Z₆₁ to Z₆₈ are optionally bonded to each other to form a C₅-C₆₀ carbocyclic group that is unsubstituted or substituted with at least one R₁₀ₐ or a C₁-C₆₀ heterocyclic group that is unsubstituted or substituted with at least one R₁₀ₐ, and
* indicates a binding site to a neighboring atom.

12. The heterocyclic compound of any one of claims 8 to 10, wherein in Formula 1, a moiety represented by 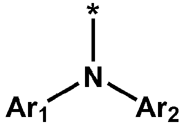 is a moiety represented by one of Formulae 6-1 to 6-3: 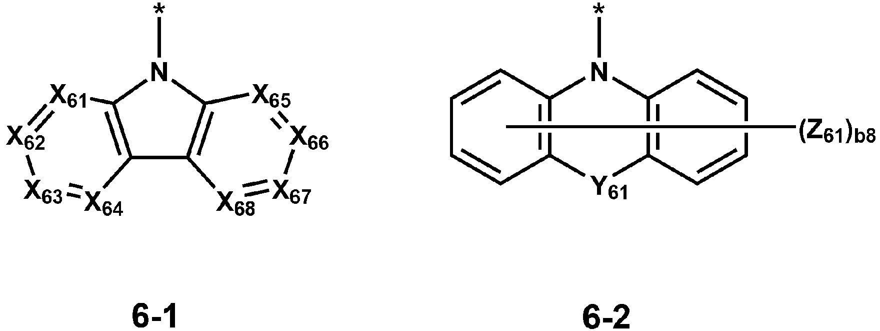 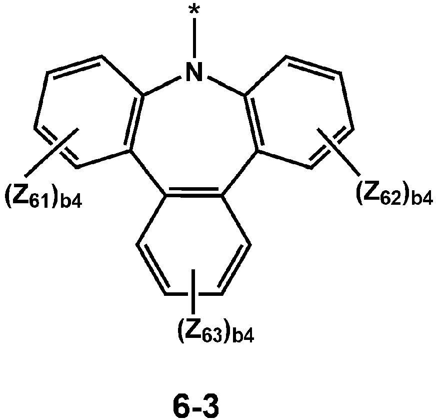 wherein in Formulae 6-1 to 6-3,
X₆₁ is N or C(Z₆₁),
X₆₂ is N or C(Z₆₂),
X₆₃ is N or C(Z₆₃),
X₆₄ is N or C(Z₆₄),
X₆₅ is N or C(Z₆₅),
X₆₆ is N or C(Z₆₆),
X₆₇ is N or C(Z₆₇),
X₆₈ is N or C(Z₆₈),
Y₆₁ is O, S, Se, C(Z₆ₐ)(Z_{6b}), Si(Z₆ₐ)(Z_{6b}), or N(Z₆ₐ),
Z₆ₐ, Z_{6b}, and Z₆₁ to Z₆₈ are each independently the same as defined in connection with R₁₀ₐ in Formula 1,
b4 is an integer from 0 to 4,
b8 is an integer from 0 to 8,
two or more neighboring groups among Z₆ₐ, Z_{6b}, and Z₆₁ to Z₆₈ are optionally bonded to each other to form a C₅-C₆₀ carbocyclic group that is unsubstituted or substituted with at least one R₁₀ₐ or a C₁-C₆₀ heterocyclic group that is unsubstituted or substituted with at least one R₁₀ₐ, and
* indicates a binding site to a neighboring atom.

13. The heterocyclic compound of any one of claims 8 to 12 wherein R₁ to R₅ are each independently:
hydrogen, deuterium, -F, -Cl, -Br, -I, a hydroxyl group, a cyano group, a nitro group, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, or an azadibenzosilolyl group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a tetraphenylenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzothiazolyl group, a benzoisoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzosilolyl group, a dibenzocarbazolyl group, an imidazopyridinyl group, an imidazopyrimidinyl group, - O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), - C(=O)(Q₃₁), -S(=O)₂(Q₃₁), -P(=O)(Q₃₁)(Q₃₂), or a combination thereof; or
-C(Q₁)(Q₂)(Q₃), -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
Q₁ to Q₃ and Q₃₁ to Q₃₃ are each independently: hydrogen; deuterium; -F; -CI; - Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or a combination thereof.

14. The heterocyclic compound of any one of claims 8 to 13, wherein the heterocyclic compound is represented by one of Formulae 1-1 to 1-4: 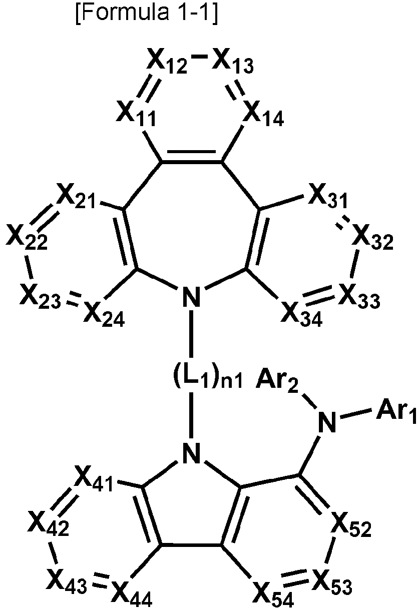 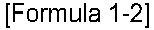 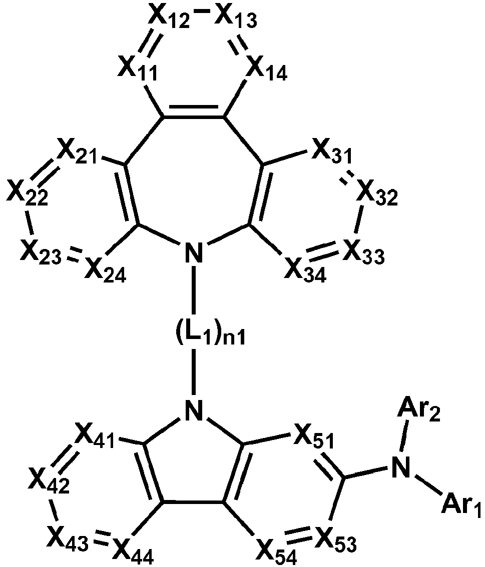 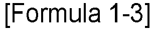 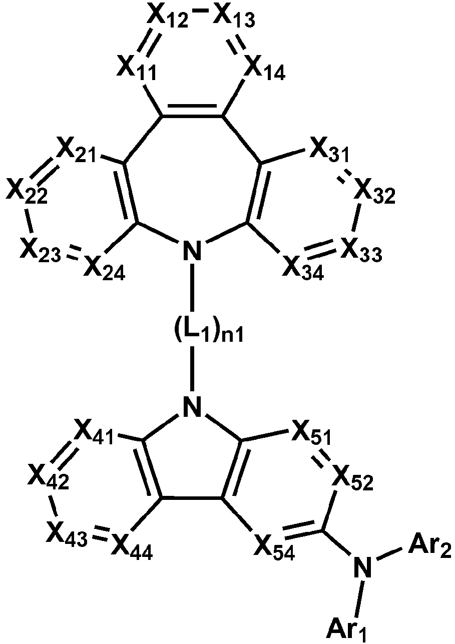 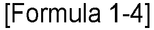 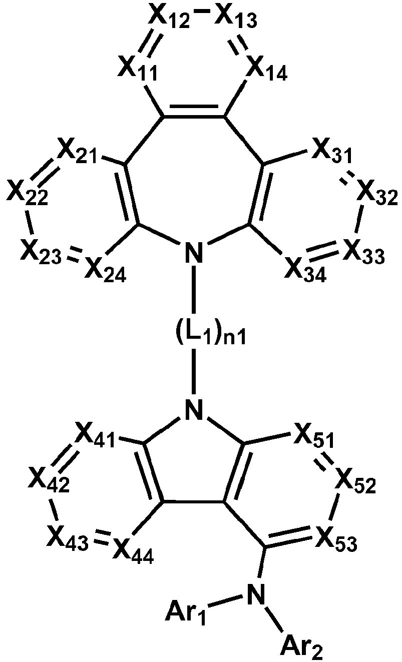 wherein in Formulae 1-1 to 1-4,
n1, L₁, Ar₁, and Ar₂ are each the same as defined in Formula 1,
X₁₁ is N or C(R₁₁),
X₁₂ is N or C(R₁₂),
X₁₃ is N or C(R₁₃),
X₁₄ is N or C(R₁₄),
X₂₁ is N or C(R₂₁),
X₂₂ is N or C(R₂₂),
X₂₃ is N or C(R₂₃),
X₂₄ is N or C(R₂₄),
X₃₁ is N or C(R₃₁),
X₃₂ is N or C(R₃₂),
X₃₃ is N or C(R₃₃),
X₃₄ is N or C(R₃₄),
X₄₁ is N or C(R₄₁),
X₄₂ is N or C(R₄₂),
X₄₃ is N or C(R₄₃),
X₄₄ is N or C(R₄₄),
X₅₁ is N or C(R₅₁),
X₅₂ is N or C(R₅₂),
X₅₃ is N or C(R₅₃),
X₅₄ is N or C(R₅₄),
R₁₁ to R₁₄ are each independently the same as defined in connection with R₁ in claim Formula 1,
R₂₁ to R₂₄ are each independently the same as defined in connection with R₂ in Formula 1,
R₃₁ to R₃₄ are each independently the same as defined in connection with R₃ in Formula 1,
R₄₁ to R₄₄ are each independently the same as defined in connection with R₄ in Formula 1, and
R₅₁ to R₅₄ are each independently the same as defined in connection with R₅ in Formula 1.

15. The heterocyclic compound of claim 8, wherein the heterocyclic compound is one of Compounds 1 to 28 and 30 to 100:
